(19) Europäisches Patentamt
European Patent Office
Office européen des brevets

(11) **EP 2 629 732 B1**

(12) **EUROPÄISCHE PATENTSCHRIFT**

(45) Veröffentlichungstag und Bekanntmachung des Hinweises auf die Patenterteilung:
**07.12.2016 Patentblatt 2016/49**

(21) Anmeldenummer: **11773189.3**

(22) Anmeldetag: **14.10.2011**

(51) Int Cl.:
*A61F 13/496* (2006.01)     *A61F 13/514* (2006.01)
*A61F 13/49* (2006.01)     *A61F 13/539* (2006.01)

(86) Internationale Anmeldenummer:
**PCT/EP2011/005159**

(87) Internationale Veröffentlichungsnummer:
**WO 2012/052137 (26.04.2012 Gazette 2012/17)**

(54) **INKONTINENZARTIKEL IN HÖSCHENFORM**

INCONTINENCE ARTICLE IN THE FORM OF BRIEFS

ARTICLE D'INCONTINENCE SOUS FORME DE CULOTTE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.10.2010 DE 102010048932**

(43) Veröffentlichungstag der Anmeldung:
**28.08.2013 Patentblatt 2013/35**

(73) Patentinhaber: **Paul Hartmann AG 89522 Heidenheim (DE)**

(72) Erfinder:
• **GASSNER, Oliver**
  **73614 Schorndorf (DE)**
• **KESSELMEIER, Rüdiger**
  **89542 Herbrechtingen (DE)**
• **MAX, Sandra**
  **89520 Heidenheim (DE)**
• **BÖSE, Anita**
  **89446 Ziertheim (DE)**
• **GAUSE, Enno**
  **89522 Heidenheim (DE)**

(56) Entgegenhaltungen:
**EP-A2- 0 446 867     WO-A1-00/47152
WO-A1-2010/028751     DE-A1- 19 716 253**

**EP 2 629 732 B1**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft einen Inkontinenzartikel in Höschenform für die Aufnahme von Körperausscheidungen mit einem vorderen Bauchabschnitt und einem hinteren Rückenabschnitt, die zur Bildung eines in Quer- oder Hüftumfangsrichtung durchgehenden Bauch- und Rückenbands mit einer in Hüftumfangsrichtung geschlossenen Hüftöffnung an beidseitigen Seitennahtbereichen herstellerseitig miteinander verbunden sind, und mit einem einen Absorptionskörper aufweisenden Schrittabschnitt, der sich in einer Längsrichtung zwischen Bauchabschnitt und Rückenabschnitt erstreckt und an den Bauchabschnitt und an den Rückenabschnitt in Verbindungsbereichen unlösbar angefügt ist, wobei sowohl der Schrittabschnitt als auch der Bauchabschnitt und der Rückenabschnitt die Beinöffnungen des Inkontinenzartikels begrenzen. Ein derart aus drei Komponenten hergestellter Inkontinenzartikel ist beispielsweise aus WO 2004/052260 A1, WO 03/039423 A1, WO 2005/067842 A1, WO 2005/016200 A1 und EP 1 392 212 B1 bekannt. Bei diesem spezifischen Produktkonzept kann nach dem Anfügen des in Längsrichtung erstreckten Schrittabschnitts an den im Wesentlichen in Quer- oder Hüftumfangsrichtung erstreckten Bauchabschnitt und an den entsprechend erstreckten Rückenabschnitt im eben ausgebreiteten Zustand dieser drei Komponenten eine H-förmige Grundstruktur des Inkontinenzartikels realisiert werden. Der Inkontinenzartikel ist dann modular aus den Komponenten Schrittabschnitt, Bauchabschnitt und Rückenabschnitt gebildet. Diese Komponenten werden vorteilhafterweise zunächst über den Schrittabschnitt miteinander verbunden, und vorzugsweise danach wird in beidseitigen Seitennahtbereichen der Bauchabschnitt mit dem Rückenabschnitt verbunden. Hierbei handelt es sich um eine herstellerseitige Verbindung, durch welche die Höschenform erhalten wird. Diese Verbindung ist typischerweise unlösbar. Die Höschenform kann aber auch insbesondere entlang einer Sollbruchlinie, die insbesondere im Seitennahtbereich verlaufen kann, auftrennbar sein, beispielsweise zum Abnehmen eines gebrauchten Inkontinenzartikels bei einer pflegebedürftigen Person.

Inkontinenzartikel in Höschenform unterscheiden sich prinzipiell von traditionellen öffenbaren und schließbaren Inkontinenzartikeln in üblicher Windelform dadurch, dass in der Regel der Hüftumfang vorgegeben ist und die Anpassung an unterschiedliche Körpergrößen ausgehend von einer Anzahl von Grundgrößen durch eine elastische Dehnbarkeit des Artikels erreicht wird. Hierfür werden in der Regel Elastifizierungsmittel, insbesondere in Form von Bändern oder Fäden, häufig als Lycra-Fäden bezeichnet, in vorgedehntem Zustand (Stretch-Bond-Verfahren) mit Chassismaterialien des Inkontinenzartikels verbunden, das heißt, sie werden in vorgedehntem Zustand an den Chassismaterialien z.B. mittels Kleber fixiert. Infolge ihrer Vorspannung raffen diese Elastifizierungsmittel die Chassismaterialien zusammen und bilden dabei Fältelungen. Der Inkontinenzartikel bzw. die elastifizierten Chassismaterialien des Inkontinenzartikels lassen sich dann wieder elastisch dehnen, wenn der Inkontinenzartikel wie ein Höschen an den Benutzer angelegt wird. Inkontinenzartikel in Höschenform mit derart elastifizierten Chassismaterialien sind mehrfach bekannt geworden und beispielsweise auch in der vorerwähnten WO 2004/052260 A1 behandelt.

Es wurde mit der vorliegenden Erfindung erkannt, dass ein dreikomponentiger Inkontinenzartikel in Höschenform gebildet aus den drei separaten Komponenten Schrittabschnitt, Bauchabschnitt und Rückenabschnitt beim Anlege- und Tragevorgang Kräften ausgesetzt ist.

Zum einen kommt es beim Anlegevorgang des Inkontinenzartikels in Höschenform, insbesondere beim Eingreifen in die seitlichen Bereiche der Chassismaterialien zu einem Verknittern, teilweise zu bereichsweisen Überfaltungen der mit Elastifizierungsmitteln versehenen Chassismaterialien und damit einhergehend zu einem Verdrillen der Chassismaterialien. Dies stellt eine Beeinträchtigung der elastifizierenden Wirkung und damit der Passform dar; auch der visuelle Eindruck wird unvorteilhaft beeinträchtigt.

Zum anderen wirken in der Tragesituation eines Inkontinenzartikels in Höschenform mitunter große Kräfte, und zwar zum einen infolge der Gewichtskraft eines mit beträchtlichen Flüssigkeitsmengen beaufschlagten Absorptionskörpers. Weiterhin werden innerhalb der Chassismaterialien hohe Zugkräfte übertragen, und zwar zum einen durch die an die Chassismaterialien üblicherweise im Stretch-Bond-Verfahren angefügten Elastifizierungsmittel, und zum anderen durch Zugkräfte, die durch Bewegungen des Benutzers übertragen werden.

Es wurde erkannt, dass ein dreikomponentiger Inkontinenzartikel in Höschenform modular gebildet aus der Verbindung des Schrittabschnitts mit dem Bauchabschnitt und mit dem Rückenabschnitt, einer für sämtliche Anforderungen des bestimmungsgemäßen Gebrauchs hinreichende Fügeverbindung zwischen diesen Komponenten bedarf, damit ausgeschlossen werden kann, dass bei Auftreten großer Zugbelastungen die Verbindung zwischen Schrittabschnitt und Bauchabschnitt oder Rückenabschnitt gelöst wird.

So zeigt die WO 2010/028751 A1 einen gattungsgemäßen Inkontinenzartikel, dessen drei Komponenten Schrittabschnitt, Bauchabschnitt und Rückenabschnitt in den Überlappungsbereichen mit Klebermaterial miteinander verbunden sind.

Des Weiteren sollte auch Tragesicherheit und Tragekomfort gegeben sein.

Bei einem gattungsgemäßen Inkontinenzartikel, welcher modular aus den drei Komponenten Schrittabschnitt, Bauchabschnitt und Rückenabschnitt zusammengesetzt ist, und insbesondere keine weiteren diesen modularen dreikomponentigen Aufbau auf der Außenseite des Inkontinenzartikels umhüllende Komponenten umfasst, ist eine besondere Anforderung an die Tragesicherheit gestellt. Darunter ist auch die Fügefestigkeit der Komponenten zueinander zu verstehen, wie schon voranstehend ausgeführt, bedingt durch den Einfluss von Kräften während des Anlegevorgangs bzw. Tragezustands.

Tragesicherheit wird dem Anwender auch durch den Einsatz von flüssigkeitsundurchlässigen Backsheet-Materialien, welche als Wäscheschutzfolien dienen, gegeben.

Der Einsatz von flüssigkeitsundurchlässigen Materialien als Backsheet-Material ist bekannt. Um neben Tragesicherheit auch einen Beitrag zum Tragekomfort zu leisten, ist der Einsatz von flüssigkeitsundurchlässigen, aber atmungsaktiven, wasserdampfdurchlässigen Folien als Backsheet-Materialien üblich. Dennoch besteht auch bei wasserdampfdurchlässigen Folien die Gefahr der Durchnässung. Demnach ist der Einsatz von atmungsaktiven Wäscheschutzfolien beschränkt, insbesondere bei Inkontinenzartikeln mit dem Schrittabschnitt als eine im Wesentlichen eine Außenseite des Inkontinenzartikels bildende Komponente.

Zum Luftaustausch können Öffnungen in den Seitenbereichen einer Wegwerfwindel, also in Bereichen außerhalb des Absorptionskörpers eingebracht werden, wie DE 197 16 253 A1 und EP 0 446 867 A2 zeigen.

Es wurde ebenso erkannt, dass bei einem gattungsgemäßen Inkontinenzartikel, welcher modular aus den drei Komponenten Schrittabschnitt, Bauchabschnitt und Rückenabschnitt gebildet ist, der Tragekomfort für den Nutzer aufgrund des in den Überlappungsbereichen der Komponenten in Z-Richtung ausgebildeten Lagenaufbaus, u.a. auch durch die flächenhafte Erstreckung von flüssigkeitsundurchlässigen Backsheet-Materialien vermindert ist, und zwar durch die dadurch bedingte Rückstauung an Hitze und damit verbunden auch an einer Ansammlung von Schweiß, was neben einem momentanen Unwohlbefinden während des Tragevorgangs auch längerfristig zu Hautreizungen führen kann.

Hiervon ausgehend liegt der vorliegenden Erfindung die Aufgabe zugrunde, den vorstehend geschilderten Problemen zu begegnen, also insbesondere eine stabile Verbindung zwischen Schrittabschnitt und Bauchabschnitt und/oder zwischen Schrittabschnitt und Rückenabschnitt bei einem gattungsgemäßen Inkontinenzartikel in Höschenform auszubilden, ohne dass dies mit einer Beeinträchtigung des Tragekomforts oder weiteren die Funktionalität des Inkontinenzartikels beeinträchtigenden Konsequenzen verbunden ist.

Diese Aufgaben werden nach der Erfindung gelöst durch einen Inkontinenzartikel mit einem dreikomponentigen Aufbau aus Bauchabschnitt, Rückenabschnitt und Schrittabschnitt mit den Merkmalen des Anspruchs 1.

Vorteilhafte Weiterbildungen des Inkontinenzartikels ergeben sich aus den jeweiligen Unteransprüchen.

*Definitionen:*

Unter Fügebereich wird der sich flächenhaft erstreckende Bereich verstanden, in welchem übereinander angeordnete Komponenten bzw. Lagen durch eine mittels Fügemittel sich in Z-Richtung erstreckende Fügeverbindung direkt oder indirekt verbunden sind.

Unter Z-Richtung wird die sich senkrecht zu der von Querrichtung und Längsrichtung beschriebenen Ebene erstreckende Richtung verstanden.

Die Anordnung der Fügemittel innerhalb eines ersten und/oder zweiten Fügebereichs kann dabei vollflächig sein.

Die Anordnung der Fügemittel innerhalb eines ersten und/oder zweiten Fügebereichs kann auch nicht vollflächig vorgesehen sein. Fügemittel, welche innerhalb eines Fügebereiches nicht vollflächig angeordnet sind, können vorzugsweise in Form einer punktuellen, einer streifenförmigen oder linienförmigen Anordnung oder einer sonstigen musterartigen Anordnung innerhalb des Fügebereichs vorgesehen sein. Der Fügebereich umfasst im Falle von nicht vollflächig angeordneten Fügemitteln auch die zwischen den Fügemitteln vorhandenen ungebundenen Bereiche. Die Zusammengehörigkeit von nicht vollflächig angeordneten Fügemitteln zu einem Fügebereich wird durch eine vorzugsweise repetitive Anordnung von vorzugsweise gleichen Fügemitteln durch einen Abstand der Fügemittel voneinander von höchstens 10 mm bestimmt. Bei nicht vollflächig angeordneten Fügemitteln wird die flächenhafte Erstreckung des Fügebereiches in Längs- und Querrichtung unter zu Hilfenahme einer imaginären Verbindungslinie durch die jeweils äußeren, also distal gelegenen, und benachbarten Fügemitteln bzw. deren äußersten Randkanten eingegrenzt.

Der durch den zweiten Fügebereich gebildete Verstärkungsbereich hat die für den zweiten Fügebereich in Längsrichtung und Querrichtung ermittelten Abmessungen.

"Im Bereich des Absorptionskörpers" wird dabei als Positionierung einer flächenhaften Erstreckung verstanden, welche in Aufsicht auf einen plan ausgelegten Inkontinenzartikel durch den Absorptionskörper in Quer- und Längsrichtung überfangen wird.

Zudem beschreibt "Fügebereich im Bereich des Absorptionskörpers" die Anordnung des Fügebereiches in Z-Richtung, nämlich zwischen Absorptionskörper und Bauchabschnitt bzw. Rückenabschnitt. Je nach Anordnung der Komponenten Bauchabschnitt, Rückenabschnitt, Schrittabschnitt mit deren Innen- und Außenseite zueinander, kann der Fügebereich weiter differenziert werden, als "unterhalb des Absorptionskörpers" oder "oberhalb des Absorptionskörpers". "Fügebereich unterhalb des Absorptionskörpers" beschreibt die Anordnung des Fügebereichs in Z-Richtung, nämlich zwischen der Außenseite des Absorptionskörpers und der Innenseite des Bauchabschnitts bzw. Rückenabschnitts. Analog beschreibt "Fügebereich oberhalb des Absorptionskörpers" die Anordnung des Fügebereichs in Z-Richtung, nämlich zwischen der Innenseite des Absorptionskörpers und der Außenseite des Bauchabschnitts bzw. Rückenabschnitts.

Unter "Innenseite" des Schrittabschnitts, Bauchabschnitts oder Rückenabschnitts bzw. des Inkontinenzartikels im Gesamten wird hierbei jeweils die dem Körper des Anwenders zugewandte Oberseite der zu betrachtenden Komponente bzw. des gesamten Inkontinenzartikels verstanden. Entsprechend wird unter "Außenseite" jeweils die dem Körper des Anwenders abgewandte und damit der Kleidung zugewandte Oberseite der zu betrachtenden Komponente bzw. des gesamten Inkontinenzartikels verstanden.

Die Angaben zu Längen und/oder Breiten des Inkontinenzartikels als solchen oder von definierten Bereichen, wie beispielsweise. der Fügebereiche und dessen Teilbereiche, sind stets auf Abmessungen am Inkontinenzartikel in seinem flach ausgelegten und eben ausgebreiteten Zustand bezogen.

Die Öffnungen sind in einer Mehrzahl vorgesehen, so dass die Öffnungen in ihrer flächenhaften Verteilung "luftdurchlässige Bereiche" eingrenzen. Die luftdurchlässigen Bereiche umfassen die Öffnungen und auch die zwischen den Öffnungen vorhandenen, also nicht perforierten Bereiche. Die Zusammenhörigkeit der Öffnungen zu einem luftdurchlässigen Bereich wird durch eine vorzugsweise repetitive Anordnung von vorzugsweise gleichen Öffnungen durch einen Abstand der Öffnungen voneinander von höchstens 10 mm bestimmt. Die flächenhafte Erstreckung des luftdurchlässigen Bereichs in Längs- und Querrichtung wird unter zu Hilfenahme einer imaginären Verbindungslinie anhand den jeweils äußeren, also distal gelegenen und benachbarten Öffnungen, und zwar an deren äußersten Randkanten eingegrenzt.

Unter "Überhang" wird hierbei die Erstreckung des Backsheet-Materials oder des Deckblatt-Materials und des Backsheet-Materials in Querrichtung seitlich außerhalb der Längsränder des Absorptionskörpers verstanden, wobei jeweils die maximale Erstreckung, also die von den Längsrändern des Absorptionskörpers am weitesten distal gelegene äußere Erstreckung des Deckblatt-Materials und/oder des Backsheet-Materials herangezogen wird. Das Backsheet-Material und/oder das Deckblatt-Material kann vorteilhafterweise aus mehreren Komponenten bestehen, so beispielsweise kann das Deckblatt-Material vorteilhafterweise ein Verbund aus einem Topsheet-Material und in Längsrichtung beidseits angrenzenden Barrieremitteln, wie beispielsweise den sogenannten Cuffs, sein. Es wird also so verstanden, dass auch bei Verbunden, also zusammengesetzten Deckblatt-Materialien und/oder Backsheet-Materialien, bei denen die einzelnen Lagen sich nicht kongruent überdecken, bei der Betrachtung des Überhangs die jeweils maximale, also am weitesten distal gelegene äußere Erstreckung des Verbundes bzw. der darin vorkommenden einzelnen Material-Lagen herangezogen wird. Dem Überhang wird jeweils die Breite H zugeordnet.

Der Überhang weist dabei "erste Bereiche" und "zweite Bereiche" auf: Ein jeweils erster Bereich erstreckt sich beidseits der Längsränder des Absorptionskörpers im vorderen und hinteren Überlappungsbereich. Ein jeweiliger zweiter Bereich erstreckt sich beidseits der Längsränder des Absorptionskörpers zwischen den schrittzugewandten Querrändern des Bauchabschnitts und des Rückenabschnitts, also außerhalb der Überlappungsbereiche.

Unter "Seitenbereich des Bauchabschnitts und/oder Rückenabschnitts" wird hierbei die Erstreckung des Chassismaterials des Bauchabschnitts und/oder Rückenabschnitts seitlich außerhalb des jeweiligen Längsrandes des Schrittabschnitts in Querrichtung bis zum jeweiligen Längsrandabschnitt des Bauchabschnitts bzw. Rückenabschnitts verstanden. Dabei wird als Längsrand des Schrittabschnitts die maximale Erstreckung, also die am weitesten distal gelegene äußere Erstreckung des Deckblatt-Materials und/oder des Backsheet-Materials, so wie voranstehend erläutert, herangezogen. Dem Seitenbereich des Bauchabschnitts und/oder des Rückenabschnitts wird jeweils die maximal mögliche Breite N zugeordnet. Auch im Falle, dass der schrittseitige und den Beinöffnungen zugewandte Abschnitt des Bauchabschnitts bzw. des Rückenabschnitts eine von der Quer- oder Hüftumfangsrichtung abweichende in Richtung auf eine Quermittelachse des Schrittabschnitts zulaufende Randkontur hat, so insbesondere, wenn diese Randkontur bogenförmig gestaltet ist, wird stets die maximale Breite N herangezogen. D.h. Breite N ist stets der Abstand in Querrichtung gemessen vom Längsrand des Schrittabschnitts bis zum Längsrand des Bauchabschnitts bzw. Rückenabschnitts, welche dann die Seitennahtbereiche des Inkontinenzartikels bilden.

Mit der Erfindung wurde erkannt, dass eine derartige Konstruktion eines Inkontinenzartikels in Höschenform wesentliche Vorteile mit sich bringt:

Ein großer Überhang des Backsheet-Materials oder des Deckblatt-Materials und Backsheet-Materials in Querrichtung jeweils außerhalb der Längsränder des Absorptionskörpers bringt den Vorteil mit sich, dass aufgrund des großen Überlappungsbereichs von Schrittabschnitt und Bauchabschnitt bzw. von Schrittabschnitt und Rückenabschnitt der Schrittabschnitt herstellerseitig sicher an Rücken- und Bauchabschnitt festlegbar ist.

Durch die Ausbildung eines zweiten Fügebereichs im ersten Bereich des Überhangs, welcher innerhalb des Überlappungsbereichs angeordnet ist, werden in einer gewissen Weise Verstärkungsbereiche gebildet. Das hat den Vorteil, dass die ersten und/oder zweiten Elastifizierungsmittel zumindest in einem Teilbereich zwischen zwei sich in einer Längsrichtung erstreckenden, versteiften Panels angeordnet sind, welche im Wesentlichen quer, zumindest mit einer signifikanten Komponente in Querrichtung zu den ersten und/oder zweiten Elastifizierungsmitteln verlaufen. Während ein Verstärkungsbereich ein erstes versteiftes Panel bildet, stellt die Seitennaht nämlich für gewöhnlich ein zweites versteiftes Panel dar. Dies wirkt sich positiv vergleichmäßigend auf die Verteilung der die Chassismaterialien in diesem Teilbereich raffenden Rückstellkräfte der ersten und/oder zweiten Elastifizierungsmittel aus. Infolgedessen kann dort eine sehr gleichmäßige Raffung erfolgen und der unerwünschten Überfaltungen der Chassismaterialien und damit einhergehend einem unkontrolliertem Verdrillen der Chassismaterialien entgegengewirkt werden. Somit wirkt sich dies positiv auf die Passform des Inkontinenzartikels aus.

Dadurch, dass die Verbindungsbereiche von Schrittabschnitt mit Bauchabschnitt und mit Rückenabschnitt jeweils entsprechend erste und zweite Fügebereiche umfassen, ist außerdem eine den jeweiligen Bereichen des Inkontinenzartikels bedarfsangepasste Ausführung und Gestaltung der Verbindung zwischen Schrittabschnitt und Bauchabschnitt bzw. Rückenabschnitt möglich: Es wurde erkannt, dass durch den ersten Fügebereich, welcher zumindest im Bereich des Absorptionskörpers verläuft, der Absorptionskörper in seiner Positionierung innerhalb des Inkontinenzartikels, insbesondere in Bezug auf die flexiblen Chassismaterialien von Bauch- und Rückenabschnitt, stabilisiert wird. Dies ist vorteilhaft, da beim Anlegevorgang eines Inkontinenzartikels in Höschenform, welcher sich vom Anlegevorgang einer traditionellen Windel des offenen Typs erheblich unterscheidet, Kräfte auf den gesamten Inkontinenzartikel einwirken. Während die Windel des offenen Typs entsprechend plan an den Körper des Anwenders ohne größere Einwirkung von Zugkräften angelegt und dann anschließend verschlossen wird, ist beim Anlegen der herstellerseitig verschlossenen Höschenwindel, bspw. beim Hochziehen zwischen den Beinen, der Absorptionskörper und die damit benachbarten Lagen, durchaus nicht unerheblichen Verformungskräften ausgesetzt. Durch die zumindest abschnittsweise Bindung im Bereich des Absorptionskörpers wird eine nachteilige Verformung und möglicherweise verbleibende wesentliche Verschiebung des Schrittabschnitts zu den Chassismaterialien des Bauch- und Rückenabschnitts unterbunden.

Wenngleich ein Überhang, wie voranstehend ausgeführt, positiv für einen stabilen Zusammenbau des dreikomponentigen Inkontinenzartikels als notwendig erachtet wird, wurde aber andererseits erkannt, dass es im Bereich der Überlappung aufgrund der Vielzahl an Lagen gegenüber dem Bereich außerhalb der Kontur des Schrittabschnitts zu einer Anstauung von Hitze und damit auch Schweiß kommt. Der Lagenaufbau im Überlappungsbereich des Überhangs umfasst dabei das Backsheet-Material, was zwar einerseits dem Anwender die Tragesicherheit durch das Verhindern von Auslaufen von Körperflüssigkeit erhöht. Andererseits kommt im Bereich des Überhangs die flüssigkeitsundurchlässige Eigenschaft der Wäscheschutzfolie, und damit nachteiligerweise verbunden die Hitze- und damit Schweißansammlung viel mehr zu tragen als in den Überlappungsbereichen, in denen sich auch der Absorptionskörper erstreckt: Durch der dem Absorptionskörper inhärenten Eigenschaftszuweisung der Absorption von Körperflüssigkeiten ist in diesem Bereich die Möglichkeit des Aufsaugens von durch den Lagenaufbau und der damit auch abdichtenden Wirkung bedingten Schweißansammlungen gegeben. Hingegen dominiert in dem sich beidseits der Längsränder des Absorptionskörpers erstreckenden Überhangs die abdichtende Eigenschaft der Wäscheschutzfolie quasi in direkter Nachbarschaft zur Haut des Anwenders ohne die derart stark saugenden Kapazitäten des Absorptionskörpers, wobei andererseits eine zu große flächenhafte Erstreckung des Absorptionskörpers aufgrund von Kosten und auch einer optisch nachteiligen Passform durch die Bauschigkeit des Absorptionskörpers auch nur in gewissen Maßen wünschenswert ist. Durch die Bereitstellung von sich durch den Lagenaufbau in Z-Richtung hindurcherstreckenden Öffnungen werden im ersten Bereich des Überhangs luftdurchlässige Zonen geschaffen, was sich somit positiv auf den Tragekomfort des Inkontinenzartikels auswirkt.

Mit den Merkmalen des Patentanspruchs 1 wird insgesamt ein Inkontinenzartikel in Höschenform mit dem genannten dreikomponentigen Aufbau geschaffen, bei dem sich eine sichere Verbindung der Komponenten realisieren lässt und die beschriebenen Nachteile des Standes der Technik überwunden sind, und zwar ohne dass dies mit einer Beeinträchtigung des Tragekomforts oder der Funktionalität des Inkontinenzartikels oder seiner Komponenten verbunden wäre, sondern stattdessen sowohl die Passform des Inkontinenzartikels als auch der Tragekomfort und die Tragesicherheit deutlich verbessert ist.

Die erfindungsgemäßen sich durch den Lagenaufbau in Z-Richtung hindurcherstreckenden Öffnungen sind in den ersten Bereichen des Überhangs im vorderen oder im hinteren Überlappungsbereich vorgesehen.

Insbesondere im vorderen Überlappungsbereich weist der erste Bereich des Überhangs vorteilhaft die sich durch den Lagenaufbau in Z-Richtung hindurch erstreckenden Öffnungen auf: Im Anwende- und Tragezustand des Inkontinenzartikels überfängt der sich im vorderen Überlappungsbereich in Querrichtung erstreckende Überhang die sich im oder um den Schambereich und daran angrenzend befindenden Körperareale. Besonders diese Körperbereiche sind als Schweißzonen bekannt. Zumal es im Tragezustand, so beispielsweise durch die im Sitzen zwischen Oberschenkel und Beckenansatz erhaltenen Hautüberfaltungen zu Wärme/Hitzestauungen und damit zu Schweißansammlungen in diesen Bereichen kommen kann. Öffnungen im ersten Bereich des Überhangs, welche sich aufgrund des modularen dreikomponentigen Aufbaus des Inkontinzenzartikels bei oder nahe den die Beinöffnungen begrenzenden Bereichen des Inkontinenzartikels befinden, tragen somit positiv zum Tragekomfort bei.

In Abhängigkeit des Anwendungsbereiches des Inkontinenzartikels und auch der Mobilität des Anwenders kann es vorteilhaft sein, die ersten Bereiche des Überhangs im vorderen und im hinteren Verbindungsbereich unterschiedlich zu gestalten. So können die Öffnungen im ersten Bereich des Überhangs insbesondere auch nur im hinteren Überlappungsbereich vorgesehen sein. Bei Anwendern von Inkontinenzartikeln in Höschenform mit einer geringeren Mobilität, so also bei bettlägrigen Personen, ist aufgrund des vorwiegenden Liegens auf der Rückenseite der hintere Überlappungsbereich besonders beansprucht. Hierbei treten vor allem im hinteren Überlappungsbereich die voranstehend erläuterten Vorund Nachteile auf, wie eben die durch den Überhang ermöglichte stabile Verbindung der Komponenten zueinander, aber andererseits die durch den Lagenaufbau nachteiligen Hitzestauungen und Schweißansammlungen. Auch hierbei kann der Tragekomfort durch die im hinteren Überlappungsbereich im ersten Bereich des Überhangs vorgesehenen Öffnungen erhöht werden.

In Weiterbildung der Erfindung erweist es sich insbesondere als vorteilhaft, wenn sowohl im vorderen als auch im hinteren Überlappungsbereich die ersten Bereiche des Überhangs sich durch den Lagenaufbau in Z-Richtung hindurcherstreckende Öffnungen aufweisen. Damit kann ein vom späteren Einsatzbereich unabhängiger und damit universal einsetzbarer Inkontinenzartikel, der unterschiedlichen Anforderungsprofilen gerecht wird, bereitgestellt werden.

Der Inkontinenzartikel ist insbesondere dreiteilig modular aus den separaten Komponenten Schrittabschnitt, Bauchabschnitt und Rückenabschnitt gebildet, d.h. der Inkontinenzartikel in Höschenform enthält neben diesen drei Komponenten keine weiteren, diese dreikomponentig modular aufgebaute Höschenform in ihrer Gesamtheit umgebenden, so zu sagen hüllenbildenden Komponenten. Hierbei wird aber das Verständnis zugrunde gelegt, dass dabei nicht ausgeschlossen ist, dass die drei Komponenten als solche, also Schrittabschnitt, Bauchabschnitt und Rückenabschnitt eine Vielfalt an Unterkomponenten, Lagen, Materialien aufweisen können.

Die Komponenten weisen jeweils eine Innenseite und eine Außenseite auf. Dabei können die Fügeverbindungen zwischen den Komponenten unterschiedlich zueinander orientiert sein.

Der Schrittabschnitt kann in den jeweiligen Verbindungsbereichen mit seiner Außenseite an die Innenseite des Bauchabschnitts und/oder an die Innenseite des Rückenabschnitts unlösbar angefügt sein. Bei einer Anbindung des Schrittabschnitts mit seiner Außenseite an die jeweilige Innenseite des Bauchabschnitts bzw. Rückenabschnitts kann der Bauchabschnitt oder Rückenabschnitt als in Querrichtung durchgehende Komponente auf die Längsenden und die daran angrenzenden Bereiche des Schrittabschnitts im Überlappungsbereich eine diese umhüllende und damit fixierende Funktion ausüben, was sich somit positiv für Tragekomfort und Tragesicherheit auswirkt.

In einer alternativen Ausführung kann der Schrittabschnitt in den jeweiligen Verbindungsbereichen mit seiner Innenseite an die Außenseite des Bauchabschnitts und/oder an die Außenseite des Rückenabschnitts unlösbar angefügt sein. Durch die Anbindung des Schrittabschnitts mit seiner Innenseite an die jeweilige Außenseite des Bauchabschnitts bzw. Rückenabschnitts liegt der Bauchabschnitt bzw. Rückenabschnitt als in Querrichtung durchgehende Komponente direkt am Körper des Anwenders an. Dies ist vorteilhaft, da damit die durch den Schrittabschnitt, insbesondere durch den sich in Längsrichtung erstreckenden Absorptionskörper zu einem gewissen Grad eingebrachte Bauschigkeit, und auch die damit durchaus starke und konstruktionsbedingte an den Längsrändern des Schrittabschnitts bzw. des Absorptionskörpers auftretende abrupte, unvermittelte Eigenschaftsänderung und damit Inhomogenität im Bauch- bzw. Rückenbereich vom Nutzer nicht mehr direkt wahrgenommen wird, sondern der Inkontinenzartikel dem Nutzer vielmehr diesbezüglich im Bauch- bzw. Rückenbereich die Anmutung eines normalen Unterwäsche-Slips vermittelt.

In einer weiteren alternativen Ausführung kann der Schrittabschnitt in den beiden Überlappungsbereichen, und damit im vorderen und hinteren Verbindungsbereich, verschieden, also einmal mit seiner Innenseite und einmal mit seiner Außenseite an Bauchabschnitt bzw. Rückenabschnitt unlösbar angefügt sein.

Für den ersten und zweiten Fügebereich können vorteilhaft nicht-adhäsive und/oder adhäsive Fügemittel oder Kombinationen davon eingesetzt werden.

Nicht-adhäsive Fügemittel sind entnommen aus der Gruppe von Schweißstellen, weiter insbesondere Ultraschallschweißstellen, thermische Schweißstellen und/oder Kalanderschweißstellen.

Für adhäsive Fügemittel können trägergebundene Klebemittel, wie Klebebänder, insbesondere zweiseitige Klebebänder und/oder auch nicht trägergebundene Klebemittel eingesetzt werden. Insbesondere wird als Klebemittel Hotmeltkleber eingesetzt. Weiter insbesondere weist das Klebemittel hydrophobe Eigenschaften auf.

Als Kleber können beispielsweise vorzugsweise eingesetzt werden: D 9105 ZP oder LC 3001 ZP (H.B. Fuller Deutschland GmbH, An der Roten Bleiche 2-3, 21335 Lüneburg, Deutschland); H20028 oder H 2481 (Bostik Nederland B.V., Zeggeveld 10, 4705 RP Roosendaal, Niederlanden); Technomelt Q2415 oder Technomelt Q5430 (Henkel KGaA, 40191 Düsseldorf, Deutschland).

Die Anordnung der Fügemittel innerhalb eines ersten und/oder zweiten Fügebereichs kann dabei vollflächig oder nicht vollflächig sein.

Als Fügemittel in einem ersten Fügebereich wird ein Klebemittel, insbesondere ein Hotmeltkleber eingesetzt. Weiter insbesondere weist das Klebemittel bzw. Hotmeltkleber hydrophobe Eigenschaften auf. Dies ist vorteilhaft, da zusätzlich zur verbindenden Funktion gleichzeitig eine Flüssigkeitsbarriere gebildet wird.

Im ersten Fügebereich kann der Schrittabschnitt vorteilhafterweise mittels eines nicht vollflächigen Klebemittelauftrags mit dem Bauchabschnitt und/oder mit dem Rückenabschnitt verbunden sein. Bei einem nicht vollflächigen Klebemittelauftrag kann es sich beispielsweise um ein streifenförmiges Muster, um eine stegförmige kontinuierliche oder nicht kontinuierliche Gitterstruktur oder um inselförmige Bereiche oder aber um eine streifenförmige oder spiralförmig angeordnete Klebemittelstruktur handeln. Es hat sich alternativ auch als besonders vorteilhaft erwiesen, im ersten Fügebereich einen vollflächigen Klebemittelauftrag einzusetzen. Mit dem ersten Fügebereich wird eine optimierte Bindung des Schrittabschnitts mit einem an sich verwindungssteifen Absorptionskörper an die vielmehr flexiblen Chassismaterialien des Bauchabschnitts und/oder Rückenabschnitts geschaffen und eine unerwünschte Verschiebung der Komponenten zueinander unterbunden.

Vorteilhafterweise erstreckt sich der erste Fügebereich im Bereich des Absorptionskörpers mindestens bis zu den Längsrändern des Absorptionskörpers, endet aber vor den Längsrändern des Schrittabschnitts. Weiter vorteilhafterweise erstreckt sind der erste Fügebereich in Querrichtung über die Längsränder des Absorptionskörpers hinaus, derart dass eine Überlappung mit dem jeweiligen zweiten Fügebereich erhalten wird.

Je nach Anordnung von Schrittabschnitt und Bauchabschnitt bzw. Rückabschnitt mit deren Innen- und Außenseite zueinander, liegt der erste Fügebereich unterhalb oder oberhalb des Absorptionskörpers.

Für einen zweiten Fügebereich werden nicht-adhäsive Fügemittel eingesetzt. Die Ausbildung eines zweiten Fügebereichs mit Fügemitteln in Form von Schweißstellen insbesondere Ultraschallschweißstellen, thermische Schweißstellen und/oder Kalanderschweißstellen ist vorteilhaft: Der Einsatz von Klebermaterialien wirft auch bei der maschinellen Fertigung der Inkontinenzartikel die Problematik auf, dass eine äußerst präzise, in schnelllaufenden Windelmaschinen nicht ohne Weiteres machbare Positionierung des Klebermaterials benötigt wird, um ein Austreten der Klebermaterialien über den Randbereich hinaus und damit das Verkleben nicht dafür vorgesehener Bereiche und Materialien innerhalb des Inkontinenzartikels bzw. zwischen mehreren Inkontinenzartikeln zu unterbinden. Der Austritt von Klebemitteln führt außerdem zu Verunreinigung der maschinellen Werkzeuge während der Herstellung des Inkontinenzartikels. Durch den Einsatz von Fügemitteln in Form von Schweißstellen werden die ungewollt produktionstechnisch bedingten Austritte von Klebermaterial vermieden. Zudem wird durch die Fügemittel in Form von Schweißstellen letztlich ein Verstärkungsbereich ohne das Einbringen weiterer zusätzlicher Materialkomponenten erhalten, was Kosten spart.

In Abhängigkeit des Anwendungsbereiches des Inkontinenzartikels und auch der Mobilität des Anwenders kann es vorteilhaft sein, die zweiten Fügebereiche im vorderen und im hinteren Verbindungsbereich unterschiedlich zu gestalten. Vorzugsweise unterscheiden sich die zweiten Fügebereiche im vorderen und hinteren Verbindungsbereich zumindest in einem der Parameter Längenerstreckung, Breite, Überlappungsgrad, Art der Fügemittel und/oder Anordnung der Fügemittel und/oder Kombinationen davon.

In Abhängigkeit des Einsatzbereiches des Inkontinenzartikels und auch der Mobilität des Anwenders kann es vorteilhaft sein, die zweiten Fügebereiche im vorderen und hinteren Verbindungsbereich unterschiedlich in der Längenerstreckung zu gestalten. Weiter vorzugsweise weisen die zweiten Fügebereiche im hinteren Verbindungsbereich eine größere Längenerstreckung auf als im vorderen Verbindungsbereich.

Erfindungswesentlich ist, dass im ersten Bereich des Überhangs sich durch den Lagenaufbau in Z-Richtung hindurcherstreckende Öffnungen vorgesehen sind. Die Öffnungen können dabei auf unterschiedliche Weise eingebracht werden. So können die Öffnungen mittels perforierenden Maßnahmen wie durch einen Schneidevorgang oder auch durch Ausstanzen in den vorliegenden Lagenaufbau eingebracht werden. In einer alternativen Maßnahme können die Öffnungen mittels sich durch den Lagenaufbau in Z-Richtung hindurcherstreckende Schweißstellen eingebracht werden. Es ist auch denkbar die Maßnahmen in Kombination einzusetzen.

Die Öffnungen sind durch Fügemittel in Form von Schweißstellen, insbesondere Ultraschallschweißstellen, thermische Schweißstellen und/oder Kalanderschweißstellen gebildet. Durch das Einbringen der Öffnungen mittels Fügemittel in Form von Schweißstellen kann vorteilhaft in einem einzigen Verfahrensschritt gleichzeitig eine Fügeverbindung des Überhangs mit dem Bauchabschnitt und/oder Rückenabschnitt und damit auch ein Verstärkungsbereich und die tragekomfortfördernde Luftdurchlässigkeit technisch einfach geschaffen werden.

Die sich durch den Lagenaufbau in Z-Richtung hindurcherstreckende Öffnungen sind zumindest innerhalb der zweiten Fügebereiche angeordnet. In Fügebereichen wird definitionsgemäß eine sich in Z-Richtung erstreckende Fügeverbindung zwischen den übereinander angeordneten Komponenten bzw. Lagen in Z-Richtung direkt oder indirekt geschaffen, wobei unter Z-Richtung die Richtung verstanden wird, welche sich senkrecht zu der von Querrichtung und Längsrichtung beschriebenen Ebene erstreckt. Durch die Fügebereiche wird demnach eine dichtere Aneinanderreihung der Lagen/Schichten erreicht und damit auch der Abdichtungseffekt und damit Anstauung von Hitze und Schweiß verstärkt. Umso mehr ist es vorteilhaft, die sich durch den Lagenaufbau hindurcherstreckenden Öffnungen innerhalb des zweiten Fügebereichs anzuordnen.

In vorteilhafter Weise stimmen die zweiten Fügebereiche und die luftdurchlässigen Bereiche in deren flächenhafter Erstreckung überein, sind insbesondere kongruent übereinander gelagert. Hiermit kann die synergistische Wirkung aus Fügeverbindung und tragekomfortsteigender Luftdurchlässigkeit innerhalb den zweiten Fügebereichen vorteilhaft dem Nutzer des Inkontinenzartikels bereitgestellt werden. Insbesondere kann vorgesehen sein, dass ein zweiter Fügebereich und ein luftdurchlässiger Bereich in ihrer flächenhaften Erstreckung kongruent übereinander gelagert sind. Dies wird insbesondere vorteilhaft erreicht, wenn der zweite Fügebereich und der luftdurchlässige Bereich in einem Verfahrensschritt gleichzeitig, so insbesondere durch Einbringen von Schweißstellen, wie insbesondere Ultraschallschweißstellen, thermische Schweißstellen und/oder Kalanderschweißstellen in den Inkontinenzartikel in Höschenform eingebracht werden.

In Weiterbildung der Erfindung erweist es sich als vorteilhaft, wenn ein zweiter Fügebereich und damit ein Verstärkungsbereich und/oder ein luftdurchlässiger Bereich in Längsrichtung betrachtet beginnend vom schrittzugewandten Querrand des Bauchabschnitts und/oder des Rückenabschnitts sich in Richtung auf die Längsenden des Schrittabschnitts, sich insbesondere kontinuierlich bis mindestens zum jeweiligen Längsende des Schrittabschnitts erstreckt. Durch die Bereitstellung der zweiten Fügebereiche und damit der Verstärkungsbereiche ausgehend vom schrittzugewandten Querrand des Bauch- und/oder Rückenabschnitts wird bereits ausgehend von den die Beinöffnungen eingrenzenden und damit bildenden Bereichen, die als distale Bereiche auch vielmehr der Problematik der Überfaltung und damit des unerwünschten Verdrillens der Chassismaterialien ausgesetzt ist, entgegengewirkt. Durch die Bereitstellung der luftdurchlässigen Bereiche ausgehend vom schrittzugewandten Querrand des Bauch- und/oder Rückenabschnitts wird bereits ausgehend von den die Beinöffnungen eingrenzenden und damit bildenden Bereichen, die wie vorangehend erläutert auch schweißansammelnde Zonen sind, tragekomfortfördernde Bereiche geschaffen.

In weiterer vorteilhafter Weise überfängt der zweite Fügebereich einen Teilbereich des Überhangs in Querrichtung mit einer Breite P' und/oder der luftdurchlässige Bereich überfängt einen Teilbereich des Überhangs in Querrichtung mit einer Breite M'. Damit erhalten der zweite Fügebereich und/oder der luftdurchlässige Bereich eine definierte Erstreckung in Querrichtung. Vorzugsweise überfängt der zweite Fügebereich bzw. der luftdurchlässige Bereich einen jeweiligen Teilbereich des Überhangs in Querrichtung in einer durchgehend gleichen Breite P' bzw. M'. Dies ist insbesondere auch vorteilhaft für die produktionstechnische Umsetzung eines jeweiligen Bereichs innerhalb des Inkontinenzartikels.

In einer weiteren vorteilhaften Weise endet der zweite Fügebereich in einem Abstand AP vor dem Längsrand des Absorptionskörpers. Abhängig von der Art des innerhalb des zweiten Fügebereichs eingesetzten Fügemittels kann es vorteilhaft sein, den zweiten Fügebereich ín einem Abstand AP vom Absorptionskörper zu beenden. So ist es beispielsweise vorteilhaft, bei Einsatz von nicht-adhäsiven Fügemitteln , wie Schweißstellen, diese nicht übergreifend in den Bereich des Absorptionskörpers einzusetzen, um somit die durch die Fügemittel eingebrachten, für einen Randbereich des Absorptionskörpers nachteiligen Verdichtungen oder sogar Öffnungen, welche die Absorptionskapazität stören und sogar minimieren können, zu vermeiden. Weiter insbesondere ist der zweite Fügebereich beginnend vom Längsrand des Schrittabschnitts angeordnet. Durch die Anordnung des zweiten Fügebereichs beginnend vom Längsrand des Schrittabschnitts werden vorteilhaft ungebundene Seitenränder des Überhangs vermieden, welche in ihrem ungebundenen Zustand nachteiligerweise zu Überfaltungen führen können, und damit auch Hautreizungen auslösen können.

Der Abstand AP beträgt vorzugsweise mindestens 3 mm, weiter vorzugsweise mindestens 5 mm, weiter vorzugsweise mindestens 7 mm, weiter vorzugsweise mindestens 10 mm, weiter vorzugsweise höchstens 40 mm, weiter vorzugsweise höchstens 30 mm, weiter vorzugsweise höchstens 20 mm.

In einer weiteren vorteilhaften Weise endet der luftdurchlässige Bereich in einem Abstand AM vor dem Längsrand des Absorptionskörpers. Durch eine Beabstandung der sich durch den Lagenaufbau in Z-Richtung hindurch erstreckenden Öffnungen vom Absorptionskörper wird vorteilhaft sichergestellt, dass die durch die Öffnungen eingebrachten Vorteile hinsichtlich Verminderung von Hitzeanstauungen und Schweißansammlungen nicht nachteiligerweise eine verminderte Barrierefunktion und damit reduzierte oder gestörte Auslaufschutzfunktion mit sich bringen. Im Randbereich des Absorptionskörpers besteht die Gefahr des Austritts durch Herauspressen von absorbierten Körperflüssigkeiten. Ebenso wird in Ausführungen, in denen Barrieremittel, so genannte Cuffs, entlang oder im Bereich des Längsrandes des Absorptionskörpers geführt werden, eine mögliche Verletzung dieser Barrieremittel durch darin versehentlich eingebrachte Öffnungen und damit Verminderung deren Barrierefunktion vermieden. Weiter insbesondere ist der luftdurchlässige Bereich beginnend vom Längsrand des Schrittabschnitts angeordnet.

Der Abstand AM beträgt vorzugsweise mindestens 3 mm, weiter vorzugsweise mindestens 5 mm, weiter vorzugsweise mindestens 7 mm, weiter vorzugsweise mindestens 10 mm, weiter vorzugsweise höchstens 40 mm, weiter vorzugsweise höchstens 30 mm, weiter vorzugsweise höchstens 20 mm.

Der Abstand AP, der Abstand AM wird dabei als in Querrichtung vermessen verstanden.

In einer bevorzugten Weise entspricht der Abstand AM, gemessen in Querrichtung, dem Abstand zwischen dem Längsrand des Absorptionskörpers und den insbesondere im zweiten Bereich des Überhangs eingebrachten Beinelastifzierungsmitteln, gemessen in Querrichtung.

Die im vorderen und/oder im hinteren Verbindungsbereich vorgesehenen zweiten Fügebereiche sind vorzugsweise jeweils symmetrisch zueinander angeordnet und weisen vorzugsweise auch jeweils die gleiche Ausgestaltung auf, so dass sie beispielsweise also hinsichtlich Längenerstreckung, Breite, Überlappungsgrad, Art der Fügemittel und/oder Anordnung der Fügemittel und/oder Kombinationen davon übereinstimmen.

In vorteilhafter Weise weist der zweite Fügebereich in dem überfangenen Teilbereich des Überhangs in Querrichtung eine Breite P' von größer 1 mm, vorzugsweise größer 2 mm, weiter vorzugsweise größer 5 mm, aber vorzugsweise von kleiner 60 mm, vorzugsweise kleiner 50 mm, weiter vorzugsweise kleiner 40 mm, weiter vorzugsweise kleiner 30 mm, weiter vorzugsweise kleiner 20 mm auf.

In weiterer vorteilhafter Weise beträgt der Anteil P'/ H des durch den zweiten Fügebereich in Querrichtung überfangenen Teilbereiches des jeweiligen Überhangs mit der Breite P' bezogen auf den jeweiligen Überhang mit einer Breite H im vorderen und/oder hinteren Überlappungsbereich mindestens 0,01, insbesondere mindestens 0,04, weiter insbesondere mindestens 0,07, weiter insbesondere mindestens 0,10, aber vorzugsweise höchstens 0,90, insbesondere höchstens 0,80, weiter insbesondere höchstens 0,70, weiter insbesondere höchstens 0,60, weiter insbesondere höchstens 0,50, weiter insbesondere höchstens 0,40. Durch diesen Anteil des Fügebereichs im jeweiligen Überhang kann eine ausreichende Verstärkung der Bindung der chassisbildenden Hüllmaterialien an den Schrittabschnitt erreicht werden, ohne dass die Flexibilität des Überhangs in erheblichem Maße beeinträchtigt wird.

In vorteilhafter Weise weist der jeweilige Überhang eine Breite H vorzugsweise von wenigstens 10 mm, weiter insbesondere von wenigstens 20 mm, weiter insbesondere von 20 bis 100 mm, weiter insbesondere von 20 bis 80 mm auf.

In einer vorteilhaften Weise beträgt der Anteil P'/N des jeweiligen durch den zweiten Fügebereich in Querrichtung überfangenen Teilbereiches des jeweiligen Überhangs mit der Breite P' bezogen auf den jeweiligen Seitenbereich des Bauchabschnitts und/oder des Rückenabschnitts mit einer Breite N mindestens 0,01, insbesondere mindestens 0,015, weiter insbesondere mindestens 0,020, aber vorzugsweise höchstens 0,35, insbesondere höchstens 0,30, weiter insbesondere höchstens 0,25, weiter insbesondere höchstens 0,20, weiter insbesondere höchstens 0,15, weiter insbesondere höchstens 0,10.

In vorteilhafter Weise erstreckt sich ein jeweiliger Seitenbereich des Bauchabschnitts und/oder des Rückenabschnitts in Querrichtung mit einer Breite N von vorzugsweise wenigstens 100 mm, weiter vorzugsweise von wenigstens 120 mm, und insbesondere 120 mm bis 350 mm, weiter insbesondere 120 bis 320 mm.

Die im vorderen und/oder im hinteren Verbindungsbereich vorgesehenen luftdurchlässigen Bereiche sind vorzugsweise jeweils symmetrisch zueinander angeordnet und weisen vorzugsweise auch jeweils die gleiche Ausgestaltung auf, so dass sie beispielsweise also hinsichtlich Längenerstreckung, Breite, Art der Öffnungen und/oder Anordnung der Öffnungen und/oder Kombinationen davon übereinstimmen.

In vorteilhafter Weise weist der luftdurchlässige Bereich in dem überfangenen Teilbereich des Überhangs in Querrichtung eine Breite M' von größer 1 mm, vorzugsweise größer 2 mm, weiter vorzugsweise größer 5 mm, aber vorzugsweise von kleiner 60 mm, vorzugsweise kleiner 50 mm, weiter vorzugsweise kleiner 40 mm, weiter vorzugsweise kleiner 30 mm, weiter vorzugsweise kleiner 20 mm auf.

In weiterer vorteilhafter Weise beträgt der Anteil M'/H des durch einen luftdurchlässigen Bereich in Querrichtung überfangenen Teilbereiches des jeweiligen Überhangs mit einer Breite M' bezogen auf den jeweiligen Überhang mit einer Breite H im vorderen und/oder hinteren Überlappungsbereich mindestens 0,01, insbesondere mindestens 0,04, weiter insbesondere mindestens 0,07, weiter insbesondere mindestens 0,10, aber vorzugsweise höchstens 0,90, insbesondere höchstens 0,80, weiter insbesondere höchstens 0,70, weiter insbesondere höchstens 0,60, weiter insbesondere höchstens 0,50, weiter insbesondere höchstens 0,40.

In einer vorteilhaften Weise beträgt der Anteil M'/N des jeweiligen durch einen luftdurchlässigen Bereich in Querrichtung überfangenen Teilbereiches des jeweiligen Überhangs mit der Breite M' bezogen auf den jeweiligen Seitenbereich des Bauchabschnitts und/oder des Rückenabschnitts mit einer Breite N mindestens 0,01, insbesondere mindestens 0,015, weiter insbesondere mindestens 0,020, aber vorzugsweise höchstens 0,35, insbesondere höchstens 0,30, weiter insbesondere höchstens 0,25, weiter insbesondere höchstens 0,20, weiter insbesondere höchstens 0,15, weiter insbesondere höchstens 0,10.

In einer weiteren vorteilhaften Weiterbildung sind die Fügemittel im zweiten Fügebereich nicht vollflächig ausgestaltet. Insbesondere sind die Fügemittel innerhalb des zweiten Fügebereiches in Form eines Musters, insbesondere in Form einer punktförmigen und/oder streifenförmigen und/oder linienförmigen Anordnung und/oder einer sonstigen musterartigen Anordnung und/oder in Kombinationen davon, vorgesehen. Hierdurch lässt sich das Ausmaß der Versteifung der zweiten Fügebereiche vorteilhaft einstellen. Wie oben beschrieben wirkt sich die Versteifung vorteilhaft auf die Passform aus; eine zu hohe Versteifung kann jedoch zu als unangenehm empfundenen Hartstellen führen.

In weiterer vorteilhafter Weise nehmen die durch die im zweiten Fügebereich nicht vollflächig angeordneten Fügemittel in Summe eine gebundene Fläche (Fügestellen) mit einem Anteil von mindestens 1,5 %, insbesondere mindestens 2,0%, weiter insbesondere mindestens 2,5% und vorzugsweise von höchstens 60%, insbesondere höchstens 50%, weiter insbesondere höchstens 40%, weiter insbesondere höchstens 30%, weiter insbesondere höchstens 20% bezogen auf die vom zweiten Fügebereich überfangene Gesamtfläche ein.

In besonders vorteilhafter Weise sind die Fügemittel im zweiten Fügebereich in einem Punktmuster angeordnet und die Summe der durch die Fügemittel gebundenen Fläche (Fügestellen) nimmt einen Anteil von mindestens 1,5%, insbesondere mindestens 2,0%, weiter insbesondere mindestens 2,3%, weiter insbesondere mindestens 2,5 %, und vorzugsweise höchstens 20,0%, insbesondere höchstens 15%, weiter insbesondere höchstens 10,0%, weiter insbesondere höchstens 8,0%, weiter insbesondere höchstens 7,0%, weiter insbesondere höchstens 6,0% bezogen auf die vom zweiten Fügebereich überfangene Gesamtfläche ein.

In weiterer vorteilhafter Weise weisen die durch die einzelnen Fügemittel in einem Punktmuster im zweiten Fügebereich gebundenen Flächen einen Durchmesser von mindestens 0,2 mm, insbesondere von mindestens 0,3 mm, weiter insbesondere von mindestens 0,4 mm, weiter insbesondere von mindestens 0,5 mm und vorzugsweise von höchstens 2,5 mm, insbesondere von höchstens 2,0 mm, weiter insbesondere von höchstens 1,5 mm, weiter insbesondere von höchstens 1,2 mm, weiter insbesondere von höchstens 1,0 mm auf.

Insbesondere sind die benachbarten einzelnen in einem Punktmuster vorhandenen Fügemittel jeweils in einem Abstand von 1 - 10 mm, insbesondere von 1 - 8 mm, weiter insbesondere von 1 - 6 mm, weiter insbesondere von 1 - 5 mm, weiter insbesondere von 1,5 - 4,5 mm, weiter insbesondere von 2 - 4 mm, besonders vorzugsweise in einem gleichen Abstand, voneinander angeordnet.

In weiterer vorteilhafter Weise nehmen die durch die im luftdurchlässigen Bereich angeordneten Öffnungen in Summe eine von den Öffnungen überfangene Fläche mit einem Anteil von mindestens 1,5 %, insbesondere mindestens 2,0%, weiter insbesondere mindestens 2,5% und vorzugsweise von höchstens 60%, insbesondere höchstens 50%, weiter insbesondere höchstens 40%, weiter insbesondere höchstens 30%, weiter insbesondere höchstens 20% bezogen auf die vom luftdurchlässigen Bereich überfangene Gesamtfläche ein.

In besonders vorteilhafter Weise sind die Öffnungen in einem Punktmuster angeordnet und die Summe der durch die Öffnungen überfangenen Fläche nimmt einen Anteil von mindestens 1,5%, insbesondere mindestens 2,0%, weiter insbesondere mindestens 2,3%, weiter insbesondere mindestens 2,5 %, und vorzugsweise höchstens 20,0%, insbesondere höchstens 15%, weiter insbesondere höchstens 10,0%, weiter insbesondere höchstens 8,0%, weiter insbesondere höchstens 7,0%, weiter insbesondere höchstens 6,0% bezogen auf die von einem luftdurchlässigen Bereich überfangene Gesamtfläche, insbesondere gezogen auf von einem zweiten Fügebereich überfangene Gesamtfläche ein. Durch diesen Anteil an Öffnungen innerhalb eines luftdurchlässigen Bereichs, insbesondere innerhalb eines zweiten Fügebereichs kann eine ausreichende Luftdurchlässigkeit im Überlappungsbereich erreicht werden, ohne dass die durch die Öffnungen gewissermaßen doch eingebrachten Materialschädigungen die Eigenschaften der eingesetzten Materialien, wie beispielsweise Reiß- und Dehnungsfestigkeiten, in einem erheblichem Maße beeinträchtigen.

Insbesondere vorteilhaft weisen die sich durch den Lagenaufbau in Z-Richtung hindurcherstreckenden Öffnungen einen Durchmesser von mindestens 0,2 mm, insbesondere von mindestens 0,3 mm, weiter insbesondere von mindestens 0,4 mm, weiter insbesondere von mindestens 0,5 mm und vorzugsweise von höchstens 2,5 mm, insbesondere von höchstens 2,0 mm, weiter insbesondere von höchstens 1,5 mm, weiter insbesondere von höchstens 1,2 mm, weiter insbesondere von höchstens 1,0 mm auf. Durch Einbringen von relativ klein dimensionierten Öffnungen wird eine zu starke Perforation der Komponenten bzw. Lagen vermieden. Einerseits würden groß dimensionierte Öffnungen zwar durchaus vorteilhaft für eine gesteigerte Luftdurchlässigkeit sein. Anderseits stellen groß dimensionierte Öffnungen ein Potential für Schädigung der Materiallagen bzw. Komponenten dar, insbesondere bei Einwirkungen von während im Anlege- und Tragezustand im Inkontinenzartikel auftretenden Zugkräften.

Insbesondere sind die benachbarten einzelnen in einem Punktmuster vorhandenen Öffnungen jeweils in einem Abstand von 1 - 10 mm, insbesondere von 1 - 8 mm, weiter insbesondere von 1 - 6 mm, weiter insbesondere von 1 - 5 mm, weiter insbesondere von 1,5 - 4,5 mm, weiter insbesondere von 2 - 4 mm, besonders vorzugsweise in einem gleichen Abstand, voneinander angeordnet.

Insbesondere weist der luftdurchlässige Bereich eine Luftdurchlässigkeit auf, welche um den Faktor 1,5, insbesondere 2, insbesondere 2,5, weiter insbesondere 3, weiter insbesondere höchstens 5, weiter insbesondere höchstens 4,5 größer ist als die Luftdurchlässigkeit im ersten Bereich des Überhangs außerhalb des luftdurchlässigen Bereichs.

Durch das Einbringen von Öffnungen wird neben der Luftdurchlässigkeit auch die Wasserdampfdurchlässigkeit beeinflusst, in dem Sinne, dass bei Luftdurchlässigkeit auch Wasserdampfdurchlässigkeit gegeben ist.

Insbesondere weist der luftdurchlässige Bereich eine Wasserdampflässigkeit auf, welche um den Faktor 1,5, insbesondere 2, insbesondere 2,5, weiter insbesondere 3, weiter insbesondere höchstens 5, weiter insbesondere höchstens 4,5 größer ist als die Wasserdampfdurchlässigkeit im ersten Bereich des Überhangs außerhalb des luftdurchlässigen Bereichs.

Die Bestimmung der Luftdurchlässigkeit erfolgt dabei wie folgt:

Die Messung der Luftdurchlässigkeit ist an die Norm DIN EN ISO 9237: 1995 angelehnt. Die Prüfmethode dient zur Beurteilung der Luftdurchlässigkeit von textilen Flächengebilden. Dabei ist die Luftdurchlässigkeit ein Kennwert zur Beschreibung der Eigenschaften textiler Flächengebilde bei gegebenem Differenzdruck Luft durchströmen zu lassen.

Die Luftdurchlässigkeit wird ausgedrückt als Geschwindigkeit eines Luftstromes, welcher unter festgelegten Bedingungen, nämlich für die Prüffläche, dem Differenzdruck und der Zeit, senkrecht zur Oberfläche durch die Messprobe hindurchgeht.

Prüfgerät:

Als Prüfgeräte ist ein Luftdurchlässigkeitprüfgerät nach DIN EN ISO 9237 einzusetzen. Ein derartiges Luftdurchlässigkeitsprüfgerät umfasst einen kreisförmigen Probenhalter mit einer Öffnung mit einer definierten Prüffläche, wie beispielsweise von 20 cm$^2$, weiter eine Vorrichtung zur verwindungsfreien und sicheren Befestigung der Messprobe, weiter bevorzugt auch zusätzlich eine Schutzringvorrichtung, als Zusatz zur vorgenannten Vorrichtung zur Verhinderung von Entweichen von Luft über die Probenkanten, weiter ein mit dem Prüfkopf verbundenes Druckmessgerät, eine Vorrichtung zur Erzeugung einer konstanten Luftströmung und zur Einstellung der Strömungsgeschwindigkeit, mit der ein Differenzdruck erzeugt werden kann und weiter ein Durchflussmessgerät zur Anzeige der Strömungsgeschwindigkeit Für die Durchführung der Messung kann beispielsweise das Gerät Typ FX 3300 Labortester III der Firma Textest AG, Schwerzenbach, Schweiz eingesetzt werden.

Probenvorbereitung:

Zur Probenvorbereitung ist vor Beginn der Prüfung die Probe mindestens 24 Stunden im Normklima bei 23°C / 50 % relativer Luftfeuchtigkeit zu lagern.

Prüfverfahren:

Beim Prüfverfahren ist darauf zu achten, dass bei vergleichenden Untersuchungen die Prüfungen unter gleichen Bedingungen, mit gleicher Prüffläche und gleichem Differenzdruck durchzuführen sind. Die Messprobe ist auf dem kreisförmigen Probenhalter zur Vermeidung von Falten mit ausreichender Spannung zu befestigen. Falls jedoch Falten entstehen, ist darauf zu achten, dass sich das Flächengebilde, also das Prüfmuster nicht in der Einspannebene verwindet wird. Dabei sind aber generell Webkanten und Flächen mit Knittern oder gefalteten Stellen zu vermeiden. Bei Flächengebilden, bei denen die Luftdurchlässigkeit in den zwei möglichen Prüfrichtungen unterschiedlich sein kann, ist festzuhalten, welche Seite geprüft wurde. Bei einseitig beschichteten textilen Flächengebilden sollte die beschichtete Seite in Richtung der Unterdruckseite eingespannt werden, um Lecks zu vermeiden. Das zum die Luft durch das textile Flächengebilde hindurch zu drücken geeignete Sauggebläse oder eine sonstige derartige Vorrichtung ist in Betrieb zu nehmen und die Strömungsgeschwindigkeit ist bis zum Erreichen des Differenzdruckes stufenlos einzustellen. Nach Erreichen von Strömungsgeschwindigkeiten unter stabilen Bedingungen, zumindest nach Abwarten von mindestens einer Minute ist die Strömungsgeschwindigkeit zu notieren. Die Prüfung ist an unterschiedlichen Stellen der Messprobe mindestens 10 mal unter den gleichen Bedingungen zu wiederholen. Als Differenzdruck wird 100 Pa oder 200 Pa empfohlen. Der für die Prüfung eingesetzte Differenzdruck ist anzugegeben.

Auswertung:

Die Luftdurchlässigkeit R ist in mm/s mit Hilfe der in der Norm angegebenen Gleichung

$$R = \frac{q(v)}{A} \times 167$$

oder in m/s nach der Gleichung zu berechnen

$$R = \frac{q(v)}{A} \times 0{,}167$$

Dabei bedeuten

q (v) : arithmetisches Mittel des Luftstromes in $dm^3$/min (l/min)
A : Prüffläche, in $cm^2$
167: Umrechnungsfaktor von $dm^3$/min oder l/min je $cm^2$, in mm/s
0,167: Umrechnungsfaktor von $dm^3$/min oder l/min je $cm^2$, in m/s

In Abänderung zu der Norm DIN EN ISO 9237: 1995:

Im Falle von Untersuchungen, in denen kein der Prüffläche des kreisförmigen Probenhalters angepasstes Prüfmuster vorhanden ist oder bereitgestellt werden kann, wie beispielsweise bei kleineren und/oder nicht kreisförmigen Prüfmustern, kann ein Prüfmuster mittels Zusammenbau mit einem Trägermaterial eingesetzt werden.

Herstellen des Prüfmusters:

- Bereitstellen eines Prüflings aus dem zu untersuchenden Material mit einer definierten Abmessung, beispielsweise 50x10 mm.
- Vorbereiten eines definierten luft- und wasserdichten Trägermaterials und Ausstanzen eines kreisrunden Stückes, welches dem vorgelegten kreisförmigen Probenhalter mit definierter Prüffläche angepasst ist. Als Trägermaterial kann beispielsweise eine Folie, PE Folie HyFol PP Plus, 16 μm von RKW Wasserburg, Deutschland, eingesetzt werden.
- Ausstanzen eines Teils aus dem Trägermaterial, wobei diese Ausstanzung eine im Vergleich zum aus dem Untersuchungsmaterial ausgestanzten Prüfling etwas kleinere Dimension aufweist, so dass der Prüfling im Bereich des ausgestanzten Loches des Trägermaterials gut befestigt werden kann. Beispielsweise 45x7 mm zum vorgenannten Anwendungsbeispiel des Prüflings.
- Einkleben des Prüflings in das Trägermaterial mittels eines wasser- und luftundurchlässigen Klebebandes, und zwar beidseitig und mit einer kleinen Überlappung, damit keine Luft zwischen dem Trägermaterial und dem Prüfling hindurchströmen kann. Beispielsweise kann dazu tesafilm® kristall klar, 15 mm x 10 m der Firma Tesa SE eingesetzt werden. Die nach dem Befestigen des Prüflings für den Luftstrom durchgängig verbliebene Fläche, also diejenige Fläche des Prüflings, welche weder von der einen noch der anderen Seite von Klebeband bedeckt ist, ist ihren Abmessungen zu notieren.

Messung:

Die Messung wird nach dem in der Methode angegebenen Prüfverfahren durchgeführt, wobei insbesondere parallele zur Korrektur und Normierung notwendige Messungen neben der Messung des eigentlichen Prüfmusters mitgeführt werden müssen, sogenannte Negativ-und Nullkontrollen für das Trägermaterial und für die zur Befestigung eingebrachten Materialien:

a) Messung der Nullkontrolle: Dazu wird das intakte Trägermaterial als solches, also noch ohne ausgestanzten Aufnahmebereich für den Prüfling, gemessen.
b) Messung der Negativkontrolle: Dazu wird das Trägermaterial mit ausgestanztem Bereich, welcher nur mit dem Klebeband überklebt ist, gemessen: Hierbei wird die Durchlässigkeit des Klebebands und des damit erzeugten Saums gemessen und kontrolliert.
c) Messung des Prüfmusters, welches wie voranstehend beschrieben, hergestellt ist.

Auswertung:

- Der gemessene Wert des Prüfmusters wird normiert auf die Nullkontrolle des Prüflings und dann um den Wert der Negativkontrolle vermindert.
- Neben den in der Norm angegebenen Einheiten mm/s oder m/s kann die Luftdurchlässigkeit auch in einer anderen Einheit, wie l/m$^2$/min oder l/m$^2$/s angegeben werden.

Die Bestimmung der Wasserdampfdurchlässigkeit erfolgt dabei wie folgt:

Die Messung der Wasserdampfdurchlässigkeit ist an die Norm ASTM E 96-0 unter Anwendung der Verfahrensvariante Wassermethode angelehnt. Es wird eine Testtemperatur von 37°C eingesetzt.

Die Prüfmethode dient zur Ermittlung der Wasserdampfdurchlässigkeit von Flächengebilden, wie beispielsweise Textilien, atmungsaktiven Folien oder Folienverbundstoffen. Dabei wird die Materialeigenschaft ermittelt, Wasserdampf von der körpernahen zur körperfernen Materialseite abzutransportieren.

Benötigte Prüfgeräte/ Reagenzien:

- Klimaschrank für Konstantklima: 37 ± 1 °C, 50 ± 2 % relative Luftfeuchtigkeit
- Stahlprüftopf mit Ringdeckel, Silikondichtungsring und Feststellschrauben, mit einer definierten oberen Öffnung, wobei der offene Querschnitt einen Durchmesser von 62 mm aufweist, was einer effektiven Prüffläche von 0,00302 m$^2$ entspricht. Der gesamte Prüftopf weist durchgehend einen Innendurchmesser von 62 mm auf und hat eine Höhe 55 mm.

- Rundes Stanzmesser mit Durchmesser 90mm
- Waage
- Messstab mit Millimetereinteilung
- Destilliertes Wasser
- Petroleumsbenzin, Siedepunkt 60 - 80°C

Probenvorbereitung:

Liegt das zu testende Flächenmaterial nicht als gewünschte Rohware, sondern in einem Verbund vor, wie beispielsweise in einem Hygieneprodukt, so wird zum Ablösen Petroleumsbenzin verwendet. Das Material wird mit Petroleumsbenzin bedeckt in einer Wanne mit Abdeckung ungefähr 20 min stehen gelassen. Dann wird das Material aus der Wanne gehoben und die unerwünschten anhaftenden Restmaterialien sollten sich leicht abstreifen lassen. Das Lösemittel lässt man dann abdampfen.

Im Zusammenhang mit der Untersuchung der Wasserdampfdurchlässigkeit des Lagenaufbaus in Z-Richtung in dem erfindungsgemäßen Inkontinenzartikel in Höschenform wird der Verbund /Lagenaufbau als solcher in das Prüfverfahren eingesetzt.

Zur Probenvorbereitung ist vor Beginn der Prüfung die Probe mindestens 24 Stunden im Normklima bei 23°C / 50 % relativer Luftfeuchtigkeit zu lagern. Die Prüfmuster werden durch Ausstanzen der Prüflinge aus dem zu testenden Material bereitgestellt, und zwar in einer angepassten Abmessung, so dass das Prüfmuster die Öffnung des Stahlprüftopfes komplett und faltenfrei abdecken kann. Der Stahlprüftopf und das darin vorzulegende Wasser werden auf 37°C vorgewärmt. Der Stahlprüftopf wird mit dem destilliertem Wasser befüllt, und zwar derart, dass ein Abstand von 30 $\pm$ 2 mm zum oberen Rand des Prüftopfes verbleibt. Anschließend wird das Prüfmuster auf den Prüftopfrand aufgelegt, wobei die körperferne Seite des Prüfmusters nach oben zeigt, der Abdichtungsring und der Ringdeckel werden darauf gelegt und die Feststellschrauben werden zur abdichtenden Fixierung des Abdichtungsringes und des Ringdeckels angezogen.

Prüfverfahren:

Zu Beginn wird der befüllte und verschlossene Prüftopf ausgewogen (Gewicht A). Nach 24 Stunden im Klimaschrank wird der Prüftopf erneut ausgewogen (Gewicht B). Wichtig ist beim Befüllen und Transportieren darauf zu achten, dass der obere Topfrand und das Prüfmuster nicht mit dem Wasser benetzt werden.

Auswertung:

Die Wasserdampfdurchlässigkeit, abgekürzt WVTR, in der Einheit [g/m$^2$/24h] wird nach der folgenden Gleichung ermittelt:

$$\text{WVTR } [g/m^2/24h] = \frac{(A - B)}{\text{Pr üffläche}[m^2]}$$

Die Prüffläche entspricht der Öffnung des Stahlprüftopfes.

In Abänderung zur voranstehend beschriebenen Methode: Im Falle von Untersuchungen, in denen kein der Prüffläche des kreisförmigen Probenhalters angepasstes Prüfmuster vorhanden ist oder bereitgestellt werden kann, wie beispielsweise bei kleineren und/oder nicht kreisförmigen Prüfmustern, kann ein Prüfmuster mittels Zusammenbau mit einem Trägermaterial eingesetzt werden. Dieses Prüfmuster zur Untersuchung der Wasserdampfdurchlässigkeit wird analog dem vorangehend beschriebenen Prüfmuster zur Untersuchung der Luftdurchlässigkeit hergestellt. Solchenfalls sind auch bei der Durchführung der Messungen zur Wasserdampfdurchlässigkeit analog entsprechende Korrektur -und Normierungsmessungen mitzuführen und entsprechend bei der Auswertung zu berücksichtigen.

In Weiterbildung des Erfindungsgedankens erweist es sich als vorteilhaft, dass der zweite Fügebereich dabei sowohl innerhalb des ersten Bereichs des Überhangs angeordnet ist und dabei jeweils einen Teilbereich des Überhangs überfängt, und dann zusätzlich über den Längsrand des Schrittteils in den daran angrenzenden Teilbereich des Bauchabschnitts und/oder des Rückenabschnitts übergeht, also somit als ein den jeweiligen Längsrand des Schrittabschnitts überbrückenden Bereich in den Inkontinenzartikel eingebracht wird. Der zweite Fügebereich ist damit in einem den jeweiligen Längsrand des Schrittbereich überbrückenden Bereichs vorgesehen, wobei also sowohl ein Teilbereich des Überhangs als auch ein daran angrenzender Teilbereich des Bauchabschnitts und/oder des Rückenabschnitts überfangen wird. Dadurch, dass der zweite Fügebereich in einem den Längsrand des Schrittabschnitts überbrückenden Bereich vorgesehen ist, wird der unmittelbare Längsrand des Schrittabschnitts sicher an den Chassismaterialien des jeweiligen Bauch- oder Rückenabschnitts gebunden. Dies ist vorteilhaft, da ein unkontrolliertes Abstehen des Überhangs des Schrittabschnitts und damit eine unkontrollierte Faltenbildung, die zu Hartstellen führen kann, unterbunden werden.

Weiter vorteilhaft überfängt der zweite Fügebereich den an den Längsrand des Schrittabschnitt angrenzenden Teilbereich des Bauchabschnits und/oder Rückenabschnitts in Querrichtung jeweils mit einer Breite P".

Weiter vorteilhaft überfängt der zweite Fügebereich den an den Längsrand des Schrittabschnitt angrenzenden Teilbereich des Bauchabschnits und/oder Rückenabschnitts in Querrichtung jeweils mit einer Breite P" von größer 1 mm, vorzugsweise größer 2 mm, weiter vorzugsweise größer 5 mm, aber vorzugsweise von kleiner 60 mm, vorzugsweise kleiner 50 mm, weiter vorzugsweise kleiner 40 mm, weiter vorzugsweise kleiner 30 mm, weiter vorzugsweise kleiner 20 mm.

Insbesondere vorteilhaft beträgt der Anteil P"/ H des jeweiligen durch den zweiten Fügebereich überfangenen Teilbereiches des Bauchabschnitts und/oder Rückenabschnitts in Querrichtung mit einer Breite P" bezogen auf den jeweiligen Überhang mit einer Breite H mindestens 0,01, insbesondere mindestens 0,04, weiter insbesondere mindestens 0,07, weiter insbesondere mindestens 0,10, aber vorzugsweise höchstens 0,90, insbesondere höchstens 0,80, weiter insbesondere höchstens 0,70, weiter insbesondere höchstens 0,60, weiter insbesondere höchstens 0,50, weiter insbesondere höchstens 0,40.

Besonders vorteilhaft beträgt der Anteil P"/N des jeweiligen durch den zweiten Fügebereich überfangenen Teilbereiches des Bauchabschnitts und/oder Rückenabschnitts in Querrichtung mit einer Breite P" bezogen auf den jeweiligen Seitenbereich des Bauchabschnitts und/oder Rückenabschnitts mit einer Breite N mindestens 0,01, insbesondere mindestens 0,015, weiter insbesondere mindestens 0,020, aber vorzugsweise höchstens 0,35, insbesondere höchstens 0,30, weiter insbesondere höchstens 0,25, weiter insbesondere höchstens 0,20, weiter insbesondere höchstens 0,15, weiter insbesondere höchstens 0,10. Durch diesen Anteil an zweitem Fügebereich und damit Verstärkungsbereich im jeweiligen Seitenbereich des Bauchabschnitts und/oder Rückenabschnitts kann eine ausreichende Verstärkung erreicht werden, ohne dass diese Chassismaterialien und ihre darin festlegten Elastifizierungsmittel in ihrer Flexibilität und auch ihrer Wirkungsweise großflächig beeinträchtigt werden.

Vorteilhafterweise weist der zweite Fügebereich und damit der Verstärkungsbereich eine Gesamtbreite P, also in Summe der Breite P' und Breite P", von 5 - 60 mm, insbesondere von 10 - 50 mm, weiter insbesondere von 10 - 40 mm, weiter insbesondere von 10 - 30 mm auf.

In vorteilhafter Weise ist der zweite Fügebereich mit der Gesamtbreite P derart entlang des Längsrandes des Schrittabschnitts angeordnet, so dass das Verhältnis der Breite P' zur Breite P" vorzugsweise zwischen 1: 4 und 4: 1, weiter vorzugsweise zwischen 1:3 und 3:1, weiter vorzugsweise zwischen 1:2 und 2:1, besonders bevorzugt 1: 1 beträgt.

In einer weiteren vorteilhaften Weise ist der zweite Fügebereich parallel zur Längsrichtung mit einer gleich bleibenden Gesamtbreite P angeordnet. Hierdurch kann der zweite Fügebereich mit geringem technischen Aufwand und damit auch schneller und kostengünstiger in den Inkontinenzartikel eingebracht werden.

Für den an den Längsrand des Schrittteils angrenzenden und einen Teilbereich des Bauchabschnitts und/oder Rückenabschnitts überfangenen zweiten Fügebereich können ebenso die bereits voranstehend dargestellten Fügemittel, also adhäsive und/oder nicht-adhäsive Fügemittel einzeln oder in Kombination eingesetzt werden.

Insbesondere bevorzugt wird der zweite Fügebereich als in einem den jeweiligen Längsrand des Schrittabschnitts überbrückenden Bereich in den Inkontinenzartikel eingebracht, insbesondere derart, dass der den Teilbereich des Überhangs überfangene Teil des zweiten Fügebereichs und der den angrenzenden Teilbereich des Bauchabschnitts und/oder Rückenabschnitts überfangene Teil des zweiten Fügebereichs mit den gleichen Fügemitteln (beispielsweise bezogen auf die Unterscheidung adhäsiv, nicht-adhäsiv), weiter insbesondere in der gleichen Anordnung (beispielsweise bezogen auf vollflächig oder nicht vollflächig) versehen ist.

Insbesondere bevorzugt weist der den angrenzenden Teilbereich des Bauchabschnitts und/oder Rückenabschnitst überfangene Teil des zweiten Fügebereichs nicht-adhäsive Fügemittel, insbesondere in Form von Schweißstellen, insbesondere Ultraschallschweißstellen, thermische Schweißstellen und/oder Kalanderschweißstellen auf.

Weiter insbesondere ist der den angrenzenden Teilbereich des Bauchabschnitts und/oder Rückenabschnitt überfangene Teil des zweiten Fügebereichs aus nicht-adhäsiven Fügemittel, insbesondere in Form von Schweißstellen, insbesondere Ultraschallschweißstellen, thermische Schweißstellen und/oder Kalanderschweißstellen gebildet. Weiter insbesondere sind die nicht-adhesiven Fügemittel in dem den angrenzenden Teilbereich des Bauchabschnitts und/oder Rückenabschnitt überfangenen Teil des zweiten Fügebereichs nicht vollflächig, sondern in Form eines Musters, insbesondere in Form einer punktförmigen und/oder streifenförmigen und/oder linienförmigen Anordnung und/oder einer sonstigen musterartigen Anordnung und/oder in Kombinationen davon, angeordnet.

Weiter insbesondere sind zusätzlich auch in dem den angrenzenden Teilbereich des Bauchabschnitts und/oder Rückenabschnitt überfangenen Teil des zweiten Fügebereichs durch einen in diesem Bereich in Z-Richtung vorliegenden Lagenaufbau sich hindurcherstreckende Öffnungen vorgesehen. Die Öffnungen können dabei mit fachüblich bekannten Methoden eingebracht werden, so beispielsweise mittels Ausschneiden, Ausstanzen oder durch den Lagenaufbau hindurchfördernde Schweißvorgänge.

In einer besonders vorteilhaften Weise sind die Öffnungen, welche innerhalb des vom zweiten Fügebereich überfangenen angrenzenden Teilbereich des Bauchabschnitts und/oder Rückenabschnitt angeordnet sind, durch die in diesen Teil des zweiten Fügebereichs eingebrachten Fügemittel, insbesondere in Form von Schweißstellen, weiter insbesondere Ultraschallschweißstellen, thermische Schweißstellen und/oder Kalanderschweißstellen, gebildet.

Die chassisbildenden Materialien von Bauchabschnitt und/oder Rückenabschnitt umfassen vorzugsweise Vliesstoffmaterialien, wie Spinnvliese (S), Meltblownvliese (M), SM-Vliese, SMS-Vliese, SMMS-Vliese, Kardenvliese oder Through Air bonded Kardenvliese. Besonders bevorzugt umfasst das chassisbildende Material von Bauchabschnitt und/oder Rückenabschnitt Spinnvlies. Die für Bauchabschnitt und/oder Rückenabschnitt eingesetzten Vliesstoffmaterialien haben vorteilhafterweise ein Flächengewicht von 10 - 30 g/m$^2$, weiter vorzugsweise von 15 - 25 g/m$^2$. Besonders bevorzugt umfassen der Bauchabschnitt und der Rückenabschnitt ein Spinnvlies aus Polypropylen, insbesondere mit einem Flächengewicht von 15 - 25 g/m$^2$.

Für die Ausbildung des Schrittabschnitts wird vorzugsweise ein Backsheet-Material bzw. ein Deckblatt-Material mit geringen Flächengewichten, nämlich von 10-40 g/m$^2$ bzw. 5 - 20 g/m$^2$ eingesetzt. Damit wird vorteilhaft die für den Anwender des Inkontinenzartikels in diesen empfindlichen Körperregionen gewünschte Weichheit, Anpassbarkeit und Drapierbarkeit realisiert.

Die chassisbildenden Hüllmaterialien des Schrittabschnitts sind weiter vorteilhaft ausgebildet: Das Backsheet-Material umfasst insbesondere eine Folie, insbesondere eines Flächengewichts von 10 - 40 g/m$^2$. Insbesondere umfasst das Backsheet eine im Gebrauch flüssigkeitsdichte aber gleichwohl atmungsaktive, also wasserdampfdurchlässige, insbesondere mikroporöse Folie. Die Wasserdampfdurchlässigkeit des Backsheets beträgt insbesondere wenigstens 300 g/m$^2$/24h, weiter insbesondere wenigstens 500 g/m$^2$/24h weiter insbesondere wenigstens 1000 g/m$^2$/24h, weiter insbesondere wenigstens 1500 g/m$^2$/24h, weiter insbesondere wenigstens 2000 g/m$^2$/24h, weiter insbesondere wenigstens 2500 g/m$^2$/24h, weiter insbesondere höchstens 6000 g/m$^2$/24h, weiter insbesondere höchstens 5000 g/m$^2$/24h, weiter insbesondere höchstens 4000 g/m$^2$/24h gemessen nach der in Anlehnung an ASTM E 96-00 beschriebenen Methode.

Die Folie kann vorteilhaft auch mit einer Vliesbeschichtung versehen sein, was eine textile Anmutung der körperabgewandten Außenseite des Inkontinenzartikels vermitteln kann. Die Vliesbeschichtung besteht vorzugsweise aus einem Vliesstoff, insbesondere einem Spinnvlies aus Polypropylen, insbesondere mit einem Flächengewicht von 7-25 g/m$^2$, 10-20 g/m$^2$, insbesondere von 12 - 17 g/m$^2$.

Das Deckblatt-Material umfasst vorzugsweise Vliesstoffmaterialien, wie Spinnvliese (S), Meltblownvliese (M), SM-Vliese, SMS-Vliese, SMMS-Vliese, Kardenvliese oder Through Air bonded Kardenvliese.

Das Deckblatt-Material kann dabei vorzugsweise nur aus Topsheet-Material gebildet sein. Weiter vorzugsweise kann das Deckblatt-Material ein Verbund aus Topsheet-Material und Barrieremittel sein. In einer weiteren vorteilhaften Ausbildung ist das Deckblatt-Material ein Verbund aus einem flüssigkeitsdurchlässigen Topsheet-Material mit Längsrändern und angrenzenden Längsrandbereichen und beidseits an den Längsrändern bzw. Längsrandbereichen des Topsheet-Materials in Fügestellen angefügten hydrophoben Barrieremitteln. Durch diesen Verbund werden in einem Inkontinenzartikel die bereichsweise unterschiedlichen Anforderungsprofile, nämlich im mittigen Bereich eine Flüssigkeitsaufnahme und an den Randbereichen eine Retardierung des seitlichen Flüssigkeitsaustritts, bereitgestellt.

Entsprechend der Funktionalität werden nachstehend genannte vorteilhafte Materialien eingesetzt: Das Topsheet-Material umfasst vorzugsweise Vliesstoffmaterialien, wie Spinnvliese, Kardenvliese oder Through Air bonded Kardenvliese. Besonders bevorzugt umfasst das Topsheet-Material Spinnvlies. Die für das Topsheet eingesetzten Vliesstoffmaterialien haben weiter vorteilhafterweise ein Flächengewicht von 5 - 20 g/m$^2$, 8-20 g/m$^2$, weiter vorzugsweise von 10 - 18 g/m$^2$, insbesondere vorzugsweise von 12 - 16 g/m$^2$. Besonders bevorzugt umfasst das Topsheet ein hydrophilisiertes Spinnvlies, insbesondere aus Polypropylen, insbesondere mit einem Flächengewicht von 12 - 16 g/m$^2$.

Das Barrieremittel-Material umfasst vorzugsweise Vliesstoffmaterialien, wie Spinnvliese, Meltblownvliese, Kardenvliese oder Through Air bonded Kardenvliese. Besonders bevorzugt umfasst das Barrieremittel-Material einlagige oder mehrlagige Vliese. Insbesondere bevorzugt umfasst das Barrieremittel-Material Laminate aus einer oder mehreren Spinnvlies(S) und/oder Meltblown(M)-Vlieslagen, insbesondere SMS-Laminate oder SMMS-Laminate, insbesondere auf Basis von Polyolefinen, wie beispielsweise Polyethylen oder Polypropylen. Derartige Materialien sind kostengünstig und aufgrund ihrer inhärent hydrophoben Eigenschaft geeignet, flüssigkeitsretardierend zu wirken.

Die für das Barrieremittel eingesetzten Vliesstoffmaterialien haben weiter vorteilhafterweise ein Flächengewicht von 5 - 20 g/m$^2$, vorzugsweise von 8 - 20 g/m$^2$, weiter vorzugsweise von 10 - 18 g/m$^2$. Besonders bevorzugt umfasst das Barrieremittel ein Laminat aus Spinnvlies und Meltblown-Vlieslagen, insbesondere aus Polypropylen, insbesondere mit einem Flächengewicht von 10 - 18 g/m$^2$.

In einer weiteren Ausführung erstreckt sich das hydrophobe Barrieremittel über die Längsränder des Topsheet-Materials hinaus, und zwar unter Ausbildung eines jeweils beidseits des Absorptionskörpers in Längsrichtung verlaufenden jeweils aufstehenden Barrieremittels, welches typischerweise als Cuffelement oder Bündchenelement bezeichnet wird. Die distalen Enden der Barrieremittel sind vorteilhafterweise mit Elastifizierungsmitteln versehen. Damit werden die Barrieremittel im Gebrauch des Inkontinenzartikels gegen die Hautoberfläche des Benutzers angehoben.

EP 2 629 732 B1

Die Fixierung der Materialbahnen des Deckblatt-Material-Verbundes in den Fügestellen kann vorzugsweise mittels Kleber, insbesondere Hotmeltkleber, thermisches Kalandern (Thermobonding) oder Ultraschallschweißen erfolgen. Die Fixierung kann in Form von kontinuierlichen Fügestellen ausgeführt sein, um eine hohe Verbundkraft zwischen dem Topsheet-Material und dem Barrieremittel zu erzielen. Denkbar ist dabei eine durchgehende Linie. Denkbar und vorteilhaft ist jedoch auch eine Fixierung durch intermittierend angebrachte Fügestellen, also durch eine Abfolge von diskreten Bindepunkten oder Bindelinien oder jedem anderen Bindemuster.

Vorteilhafterweise haben das Backsheet-Material und das Deckblatt-Material dieselbe Erstreckung in Querrichtung. Sie sind kongruent, also deckungsgleich zueinander. Weiter vorteilhaft ist aber auch, wenn das Backsheet-Material und Deckblatt-Material nicht kongruent zueinander sind. Insbesondere vorteilhafterweise hat das Backsheet-Material eine im Vergleich zum Deckblatt-Material schmälere Erstreckung in Querrichtung. Damit wird das den Anwender im Tragekomfort möglicherweise störende Backsheet-Material, wie beispielsweise eine Folie, durch das hautfreundliche Vliesmaterial des Deckblatt-Materials überdeckt.

In Weiterbildung der Erfindung erweist es sich als vorteilhaft, wenn der Anteil der Fläche des Schrittabschnitts an der gesamten Fläche des Inkontinenzartikels 25 - 55 %, insbesondere 30 - 47 %, weiter insbesondere 35 - 47 % und weiter insbesondere 35 - 45 % beträgt.

Der Überlappungsbereich zwischen Schrittabschnitt und Bauchabschnitt ist in Weiterbildung der Erfindung so ausgebildet, dass der Schrittabschnitt 15 - 40 %, insbesondere 15 - 35 % und weiter insbesondere 15 - 25 % der Fläche des Bauchabschnitts überlappt. In vorteilhafter Weise überlappt der Schrittabschnitt den Bauchabschnitt mit einer Fläche von 25.000 - 45.000 mm$^2$.

Der Überlappungsbereich von Schrittabschnitt und Rückenabschnitt ist in Weiterbildung der Erfindung so ausgebildet, dass der Schrittabschnitt 20 - 40 %, insbesondere 20 - 35 % und weiter insbesondere 22 - 32 % der Fläche des Rückenabschnitts überlappt.

In vorteilhafter Weise überlappt der Schrittabschnitt den Rückenabschnitt mit einer Fläche von 35.000 - 65.0000 mm$^2$, insbesondere von 40.000 - 55.000 mm$^2$.

Die Überlappung des Schrittabschnitts mit dem Rückenabschnitt ist in vorteilhafter Weise größer als die Überlappung des Schrittabschnitts mit dem Bauchabschnitt.

Es ist bei der erfindungsgemäßen Ausbildung des Inkontinenzartikels möglich und vorteilhaft, wenn auch der Absorptionskörper 5 - 20%, insbesondere 5 - 15 % der Fläche des Bauchabschnitts und/oder 10 - 20 %, insbesondere 10 - 15 % der Fläche des Rückenabschnitts überlappt.

Die Erstreckung des Bauchabschnitts und des Rückenabschnitts im Seitennahtbereich in Längsrichtung beträgt vorteilhafterweise wenigstens 100 mm, insbesondere wenigstens 150 mm und insbesondere 150 mm bis 220 mm.

Der minimale Abstand des Bauchabschnitts und des Rückenabschnitts in Längsrichtung voneinander beträgt vorteilhafterweise 250 bis 400 mm.

Die maximale Erstreckung des Schrittabschnitts in Querrichtung, also die größte Breite E, beträgt vorteilhafterweise wenigstens 200 mm, insbesondere 200 bis 350 mm, weiter insbesondere 250 bis 320 mm.

Des weiteren erweist es sich als vorteilhaft, wenn der Überhang des Backsheet-Materials und/oder des Deckblatt-Materials in Querrichtung in der Summe, also beidseits der Längsränder des Absorptionskörpers insbesondere 25 - 50 %, weiter insbesondere 30 - 45 % und weiter insbesondere 35 - 45 % bezogen auf die größte Breite E des Schrittabschnitts beträgt. Der verhältnismäßig große Überhang von Backsheet-Material und/oder Deckblatt-Material beidseits der Absorptionskörpers bedeutet also einen breiten Schrittabschnitt mit einem verhältnismäßig schmalen Absorptionskörper. Hierdurch ist es möglich, entlang der Beinöffnungen erstreckte Beinelastifizierungsmittel in dem Schrittabschnitt vorzusehen, die einen verhältnismäßig großen Abstand zum materialreichen und damit biegesteifen Absorptionskörper haben. Dies resultiert wiederum in einer guten Abdichtbarkeit und Anpassbarkeit der beidseitigen Beinöffnungsränder des Schrittabschnitts. Solchenfalls behindert nämlich der materialreiche und gegenüber dünnen Chassismaterialien verwindungssteife Absorptionskörper die Ausbildung eines flüssigkeitsdichten Beinabschlusses nur wenig; es muss daher zur Ausbildung eines flüssigkeitsdichten Beinabschlusses nicht mit extrem hohen Spannungen gearbeitet werden, was sich wiederum positiv auf den Tragekomfort des Inkontinenzartikels auswirkt.

In noch weitergehender Ausbildung der Erfindung erweist es sich als besonders vorteilhaft, wenn die Beinelastifizierungsmittel in Längsrichtung wenigstens 10 mm, insbesondere wenigstens 20 mm vor den zweiten Elastifizierungsmitteln enden. Insbesondere vorteilhaft ist, wenn die Beinelastifizierungsmittel in Längsrichtung vor dem Bauchabschnitt und/oder vor dem Rückenabschnitt enden. Die von ihnen ausgeübte Spannung und Rückstellkraft beeinflusst daher nicht Spannungsverhältnisse der zweiten Elastifizierungsmittel. Insbesondere werden nicht die Spannungsverhältnisse innerhalb des schrittseitigen und den Beinöffnungen zugewandten Bereichs des Bauchabschnitts und des Rückenabschnitts, in dem die zweiten Elastifizierungsmittel sich auffächernden vorgesehen sind, beeinflusst.

Als Beinelastifizierungsmittel werden vorzugsweise faden- oder bandförmige Elastifizierungsmittel, wie Gummi- oder Polyetherpolyurethan- oder Polyesterpolyurethanfäden, vorzugsweise Elastikfäden wie Lycra®-, Creora®- oder Spandex®-Fäden eingesetzt. Die Beinelastifizierungsmittel haben vorzugsweise eine Stärke von 300 - 1500 dtex, insbesondere von 500 - 1200 dtex, weiter insbesondere von 500 - 900 dtex. Die Beinelastifizierungsmittel werden vorzugsweise unter einer Vorspannung von 1,5 - 6,0, insbesondere von 2,5 - 4,5 an den chassisbildenden Hüllmaterialien des Schrittabschnitts fixiert. Die Vorspannung ist definiert als Faktor des Dehnungsgrades gegenüber dem ungedehnten/relaxierten Zustand des Elastifizierungsmittels.

Zur flächenhaften Elastifizierung von Bauchabschnitt und Rückenabschnitt sind die jeweils in einem Abstand voneinander und parallel zueinander in Quer- oder Hüftumfangsrichtung erstreckten ersten Elastifizierungsmittel vorgesehen. Diese haben vorzugsweise gleiche Vorspannung und dienen im Wesentlichen einer flächenhaft durchgehenden gleichmäßigen Elastifizierung des Bauchabschnitts und des Rückenabschnitts in dem Bereich deutlich oberhalb der Beinöffnungen. Es ist indessen möglich, dass die ersten Elastifizierungsmittel in einem oberen Hüftrandbereich eine stärkere Vorspannung aufweisen oder mehrere dieser Elastifizierungsmittel mit geringerem Abstand voneinander vorgesehen sind, um eine etwas stärkere Elastifizierung am Hüftrand zu realisieren.

In noch weitergehender Ausbildung der vorliegenden Erfindung wurde auch erkannt, dass die Spannungsverhältnisse in dem genannten schrittseitigen und den Beinöffnungen zugewandten Bereich des Bauchabschnitts und des Rückenabschnitts im Hinblick auf den Tragekomfort wesentlich sind und so gestaltet werden können, dass der Tragekomfort verbessert wird. Vorteilhafterweise erstrecken sich die zweiten Elastifizierungsmittel ausgehend von den beiden Seitennahtbereichen in Richtung auf eine Längsmittelachse des Inkontinenzartikels und verlaufen dabei bogenförmig sich auffächernd mit zunehmendem Abstand voneinander.

Hierfür ist der schrittseitige und den Beinöffnungen zugewandte Bereich, in welchem die zweiten Elastifizierungsmittel sich in Richtung auf die Längsmittelachse auffächern, vorzugsweise so ausgebildet, dass bei flächenhafter Dehnung dieses Bereichs die dabei auftretende Rückstellkraft in Richtung auf den Schrittabschnitt abnimmt.

Betrachtet man also diesen schrittseitigen und den Beinöffnungen zugewandten Bereich des Bauchabschnitts und des Rückenabschnitts, und zwar in einer Richtung ausgehend von dem jeweiligen Seitennahtbereich in Richtung auf den Schrittabschnitt, also in Richtung auf eine Längsmittelachse des Inkontinenzartikels und gewissermaßen in Richtung der bogenförmigen Auffächerung der zweiten Elastifizierungsmittel, so wird die bei flächenhafter Dehnung auftretende Rückstellkraft in dieser Richtung reduziert. Es handelt sich also hierbei um diejenige Kraft, welche der Bauchabschnitt und der Rückenabschnitt einer flächenhaften Dehnung entgegensetzen. Eine Abnahme dieser Rückstellkraft, die sich dann naturgemäß auf den Benutzer überträgt, ist mit einer erheblichen Verbesserung des Tragekomforts des Inkontinenzartikels verbunden.

Es erweist sich weiter als besonders vorteilhaft, wenn die Abnahme der Rückstellkraft in dem genannten schrittseitigen und den Beinöffnungen zugewandten Bereich des Bauchabschnitts und des Rückenabschnitts so vorgesehen wird, dass in Richtung auf den Schrittabschnitt eine abnehmende Anzahl von Falten pro cm-Querrichtung des Inkontinenzartikels gebildet wird. Solchenfalls können sich der Bauchabschnitt und der Rückenabschnitt entsprechend der Körperform des Benutzers dehnen, ohne dass die hierdurch gebildeten elastischen Kräfte das Chassismaterial mit einer Vielzahl von Falten zu raffen versuchen. Es sei an dieser Stelle nochmals erläutert, dass die Abnahme der Rückstellkraft in Richtung auf den Schrittabschnitt bedeutet, dass diejenige Kraft, die infolge einer flächenhaften Dehnung erzeugt wird, mit zunehmender Annäherung an den Schrittabschnitt geringer wird. Die Rückstellkraft infolge flächenhafter Dehnung ist also in einem Gebiet näher der Seitennaht größer als in einem Gebiet näher dem Schrittabschnitt.

Die genannten Spannungsverhältnisse lassen sich in verschiedenster Weise erreichen, etwa durch Verwendung von in Querrichtung unterschiedlich elastischen Materialien in dem schrittseitigen und den Beinöffnungen zugewandten Bereich, in welchem auch die zweiten Elastifizierungsmittel vorgesehen sind. Es wäre auch denkbar, dass die Vorspannung der zweiten Elastifizierungsmittel mit zunehmender Annäherung an den Schrittabschnitt, also von außen nach innen in Richtung auf eine Längsmittelachse reduziert wird. Außerdem wäre es denkbar, dass die Abnahme der Rückstellkraft bei flächenhafter Dehnung dadurch erreicht wird, dass der Abstand der zweiten Elastifizierungsmittel voneinander zunimmt, wobei hier darauf zu achten ist, dass dies nicht mit einer starken Zunahme der Vorspannung infolge des fächerförmigen Verlaufs der Elastifizierungsmittel ausgeglichen oder gar in Richtung zunehmender Rückstellkraft übertroffen wird.

Zur Bestimmung der Rückstellkräfte können die zu vermessenden Bereiche des Chassis direkt, gleichsam zerstörungsfrei zwischen zwei Klemmbacken von definierter, gleicher Klemmbackenbreite fest eingespannt und die Rückstellkräfte bei einer definierten, den Gebrauchszustand simulierenden Dehnung der zu messenden Bereiche um insbesondere 30% oder 50% oder 80% der Ausgangslänge (des Klemmbackenabstandes bei Fixieren des zu messendes Bereiches in ungespanntem Zustand) ermittelt werden. Die Klemmbacken sollten möglichst viele, zumindest jedoch zwei nebeneinander angeordnete Elastifizierungsmittel des zu messenden Bereiches fixieren und im Wesentlichen senkrecht zum Verlauf der Elastifizierungsmittel orientiert sein, so dass die Dehnung zwischen den Klemmen im Wesentlichen in Richtung des Verlaufs der Elastifizierungsmittel erfolgt.

Es hat sich insbesondere als vorteilhaft erwiesen, wenn ein minimaler Abstand der zweiten Elastifizierungsmittel voneinander (Abstand von unmittelbar nebeneinander liegenden Elastifizierungsmitteln) in den Seitennahtbereichen 3 bis 8 mm, insbesondere 3 bis 7 mm und weiter insbesondere 3 bis 6 mm beträgt.

Des Weiteren hat es sich als vorteilhaft erwiesen, wenn ein maximaler Abstand der zweiten Elastifizierungsmittel voneinander (Abstand von unmittelbar nebeneinander liegenden Elastifizierungsmitteln) an einem Absorptionskörperr-

and oder an einem Längsrand des Schrittabschnitts 7 bis 35 mm, insbesondere 10 bis 32 mm und weiter insbesondere 12 bis 30 mm beträgt.

Des Weiteren hat es sich als vorteilhaft erwiesen, wenn die zweiten Elastifizierungsmittel einen Auffächerungsgrad F

$$F=(A-B)/B * 100\%$$

von 50 bis 900 %, insbesondere von 100 bis 700 % und weiter insbesondere von 150 bis 550 % haben.

Dabei ist der Auffächerungsgrad F als Verhältnis der Abstandszunahme (A-B) zum minimalen Abstand (B) in Prozent definiert. Dabei sind die Größen A und B definiert als der Abstand des in Längsrichtung äußersten zweiten Elastifizierungsmittels von dem in Längsrichtung innersten zweiten Elastifizierungsmittel (also nicht der Abstand von unmittelbar nebeneinander liegenden zweiten Elastifizierungsmitteln), und zwar A als der maximale Abstand, insbesondere am Längsrand des Schrittabschnitts oder am Absorptionskörperrand, und B als der minimale Abstand insbesondere im Seitennahtbereich. Es wurde auch erkannt, dass es sich als vorteilhaft erweist, wenn der Auffächerungsgrad F der zweiten Elastifizierungsmittel im Rückenabschnitt größer ist als im Bauchabschnitt.

Aufgrund der naturbedingten Körperformen im Rückenbereich bzw. Bauchbereich eines Benutzers erweisen sich die hier thematisierten Probleme typischerweise im Rücken- oder Gesäßbereich als gravierender. Insofern erweist es sich als vorteilhaft, wenn der maximale Abstand der zweiten Elastifizierungsmittel voneinander an einem Absorptionskörperrand im Rückenabschnitt größer ist als im Bauchabschnitt.

In einer vorteilhaften Ausführungsform erstrecken sich die jeweiligen zweiten Fügebereiche und damit die Verstärkungsbereiche in Längsrichtung betrachtet beginnend vom schrittzugewandten Querrand des Bauchabschnitts und des Rückenabschnitts in Richtung auf die Längsenden des Schrittabschnitts, und zwar zumindest bis zu der Länge, dass die von den Seitennahtbereichen in Richtung auf die Längsmittelachse des Inkontinenzartikels bogenförmig verlaufenden, und insbesondere mit zunehmenden Abstand auffächernden und an den Längsrand des Schrittabschnitts mündenden zweiten Elastifizierungsmittel damit erfasst werden. Weiter vorteilhafterweise erstrecken sich die jeweiligen zweiten Fügebereiche und damit die Verstärkungsbereiche beginnend vom schrittzugewandten Querrand des Bauchabschnitts und des Rückenabschnitts in Längsrichtung betrachtet zumindest bis zu der Länge, welche beim Anlegen einer gedachten horizontalen Linie fluchtend mit der Höhe des Seitennahtbereichs übereinstimmt, von welchem aus die bogenförmig verlaufenden, und insbesondere sich auffächernden zweiten Elastifizierungsmittel beginnen. Wie eingangs beschrieben, wirkt sich die Versteifung mittels des durch den zweiten Fügebereich bereitgestellten Verstärkungsbereiches vorteilhaft auf die Passform aus.

Es wäre durchaus denkbar, dass die zweiten Elastifizierungsmittel von dem einen Seitennahtbereich zu dem anderen Seitennahtbereich durchgehend verlaufen, was insbesondere die Einbringung in einem kontinuierlichen Herstellungsverfahren im Vergleich zu einem "cut-and-place"-Prozess vereinfacht. Infolge der Überdeckung des Schrittabschnitts mit dem Bauchabschnitt und mit dem Rückenabschnitt kann es je nach Gestaltung auch zu einer Überlappung oder Überdeckung des materialreichen Absorptionskörpers mit dem Bauchabschnitt und/oder dem Rückenabschnitt kommen und somit auch mit demjenigen schrittseitigen und den Beinöffnungen zugewandten Bereich des Bauchabschnitts und des Rückenabschnitts, in dem die zweiten Elastifizierungsmittel verlaufen. Der materialreiche Absorptionskörper behindert dabei üblicherweise eine elastische Dehnbarkeit der Chassismaterialien. Außerdem ist es nicht unbedingt vorteilhaft, wenn der materialreiche Absorptionskörper mit zusätzlichen Spannungskräften beaufschlagt wird. Deshalb kann es sich als vorteilhaft erweisen, wenn die zweiten Elastifizierungsmittel in einem Überlappungsbereich mit dem Absorptionskörper des Schrittabschnitts hinsichtlich ihrer elastischen Eigenschaften deaktiviert sind. Diese Deaktivierung kann beispielsweise durch eine Anzahl von Trennschnitten durch die zweiten Elastifizierungsmittel im Bereich der Überdeckung mit dem Absorptionskörper realisiert werden, wobei auch andere Trennverfahren, wie z. Bsp. mittels Ultraschallschweißen oder Laser denkbar sind.

Es sei erwähnt, dass auch die ersten Elastifizierungmittel im Bereich einer Überdeckung mit dem Absorptionskörper hinsichtlich ihrer elastischen Eigenschaften deaktiviert sein können.

Hinsichtlich der Gesamtabmessungen des Inkontinenzartikels erweist es sich als vorteilhaft, wenn der Abstand (C) des schrittzugewandten innersten zweiten Elastifizierungsmittels des Bauchabschnitts von dem entsprechenden schrittzugewandten innersten zweiten Elastifizierungsmittel des Rückenabschnitts 250 bis 420 mm beträgt.

Der Abstand der innersten, schrittzugewandten zweiten Elastifizierungsmittel von der die Beinöffnungen begrenzenden Randkontur des schrittseitigen und den Beinöffnungen zugewandten Bereichs des Bauchabschnitts und des Rückenabschnitts beträgt vorzugsweise 2 - 40 mm, weiter vorzugsweise 3 - 30 mm, insbesondere vorzugsweise 4 - 15 mm.

Als erste und/oder zweite Elastifizierungsmittel werden vorzugsweise faden- oder bandförmige Elastifizierungsmittel, wie Gummi- oder Polyetherpolyurethan- oder Polyesterpolyurethanfäden, vorzugsweise Elastikfäden wie Lycra®-, Creora (R)- oder Spandex®-Fäden eingesetzt. Die ersten und/oder zweiten Elastifizierungsmittel haben vorzugsweise eine Stärke von 300 - 1500 dtex, insbesondere von 500 - 900 dtex, weiter insbesondere von 500 - 600 dtex. Die ersten und/oder zweiten Elastifizierungsmittel werden vorzugsweise unter einer Vorspannung von 1,5 - 6,0, insbesondere von 2,5 - 5,0 an den chassisbildenden Hüllmaterialien des Bauchabschnitts und Rückenabschnitts fixiert. Die Vorspannung ist dabei definiert als Faktor des Dehnungsgrades gegenüber dem ungedehnten/relaxierten Zustand des Elastifizierungsmittels.

Dessen ungeachtet erweist es sich als vorteilhaft, wenn der Bauchabschnitt und der Rückenabschnitt zumindest außerhalb des Absorptionskörpers über die Längsrichtung im Wesentlichen durchgehend flächenhaft querelastifiziert sind, wobei auch solchenfalls die vorteilhaften Spannungsverhältnisse einzuhalten bzw. zu realisieren sind.

Der Absorptionskörper umfasst Körperflüssigkeiten absorbierende Materialien wie natürliche oder synthetische Fasern, insbesondere Zellulosefasern, vorzugsweise in Form von Zellstofffluff. Vorzugsweise umfasst der Absorptionskern außerdem superabsorbierende Materialien (SAP), insbesondere auf Basis oberflächenvernetzter, teilneutralisierter Polyacrylate.

Der Schrittabschnitt bzw. die Längsränder des Schrittabschnitts, welche die Beinöffnungen begrenzen, sind vorteilhafterweise bogenförmig konturiert ausgebildet.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform des erfindungsgemäßen Inkontinenzartikels. In der Zeichnung zeigt:

Figur 1      eine Draufsicht auf einen erfindungsgemäßen Inkontinenzartikel, wobei ein Bauchabschnitt, ein Rückenabschnitt und ein die beiden verbindender Schrittabschnitt des Inkontinenzartikels noch nicht zur Bildung einer Höschenform verbunden, sondern in flachgelegtem und ausgedehntem Zustand dargestellt sind;

Figur 2      eine Draufsicht auf einen erfindungsgemäßen Inkontinenzartikel, wobei ein Bauchabschnitt, ein Rückenabschnitt und ein die beiden verbindender Schrittabschnitt des Inkontinenzartikels noch nicht zur Bildung einer Höschenform verbunden;

Figur 3      eine Schnittansicht (schematisch) entlang einer Quermittelachse des Schrittabschnitts und daran gebundenen Rückenabschnitts mit Schnittebene III-III in Figur 1;

Figur 4      eine Schnittansicht (schematisch) entlang einer Quermittelachse des Schrittabschnitts und daran gebundenen Rückenabschnitts mit Schnittebene IV-IV in Figur 2;

Figur 5      eine ausschnittsweise Darstellung eines zweiten Fügebereichs und luftdurchlässigen Bereichs des Inkontinenzartikels nach Figur 1;

Figur 6      eine Schnittansicht (schematisch) des zweiten Fügebereichs und luftdurchlässigen Bereichs mit Schnittebene VI-VI in Figur 5;

Figur 7      eine Schnittansicht (schematisch) entlang einer Quermittelachse des Schrittabschnitts mit Schnittebene VII-VII in Figur 1;

Figur 8      eine perspektivische Ansicht (schematisch) des an einen Benutzer angelegten Inkontinenzartikels nach Figur 1 oder 2;

Figur 9      eine ausschnittsweise Darstellung des Inkontinenzartikels nach Figur 1 oder 2;

Figuren 10,11      verdeutlichen beispielhaft die Bestimmung von Rückstellkräften im Bauchabschnitt bzw. Rückenabschnitt des Inkontinenzartikels;

Figur 12      eine Draufsicht auf einen schematisch dargestellten Inkontinenzartikel im flachgelegten und ausgedehnten Zustand, wobei der Schrittabschnitt zunächst nur mit einem Bauchabschnitt verbunden dargestellt ist.

Die Figuren zeigen einen insgesamt mit dem Bezugszeichen 2 bezeichneten Inkontinenzartikel in Höschenform für die Aufnahme fester und flüssiger Körperausscheidungen. Der Inkontinenzartikel 2 ist aus drei weitestgehend unabhängig voneinander fertigbaren Komponenten, nämlich einem vorderen Bauchabschnitt 4, einem hinteren Rückenabschnitt 6 und einem zwischen diesen angeordneten und einen Absorptionskörper 7 aufweisenden Schrittabschnitt 8 gebildet, wobei der Schrittabschnitt 8 mit einem wesentlichen Flächenanteil mit dem Bauchabschnitt 4 einerseits und dem Rückenabschnitt 6 andererseits überlappt und im Überlappungsbereich herstellerseitig unlösbar verbunden ist.

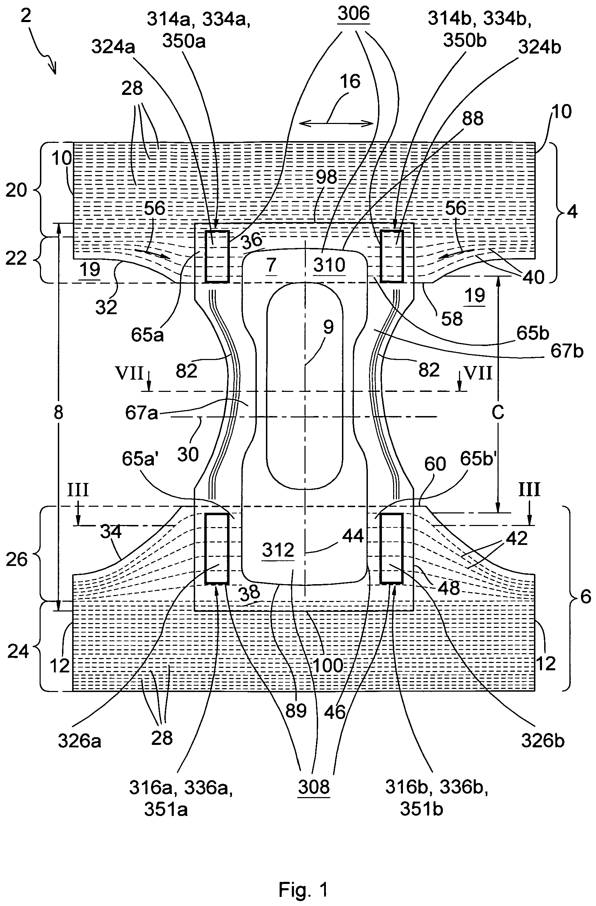

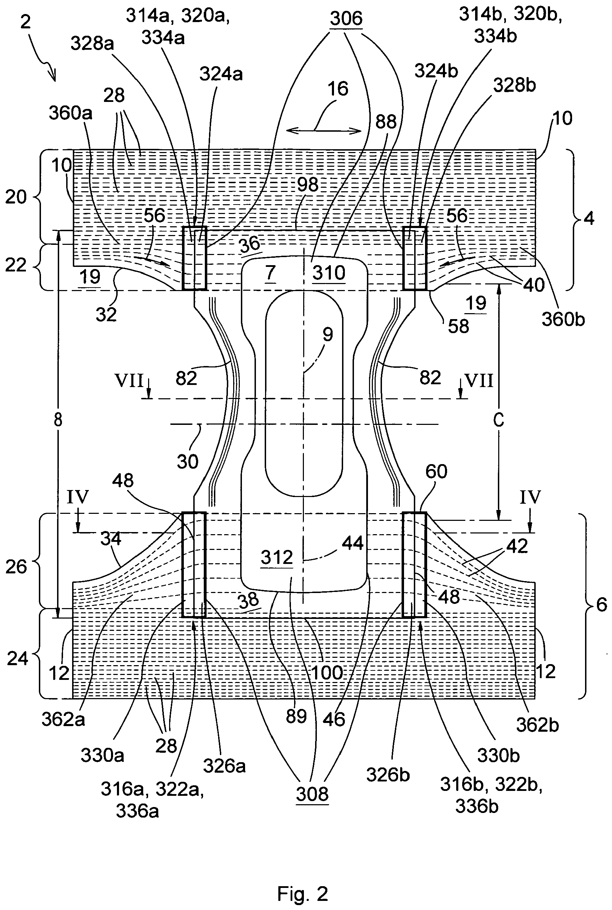

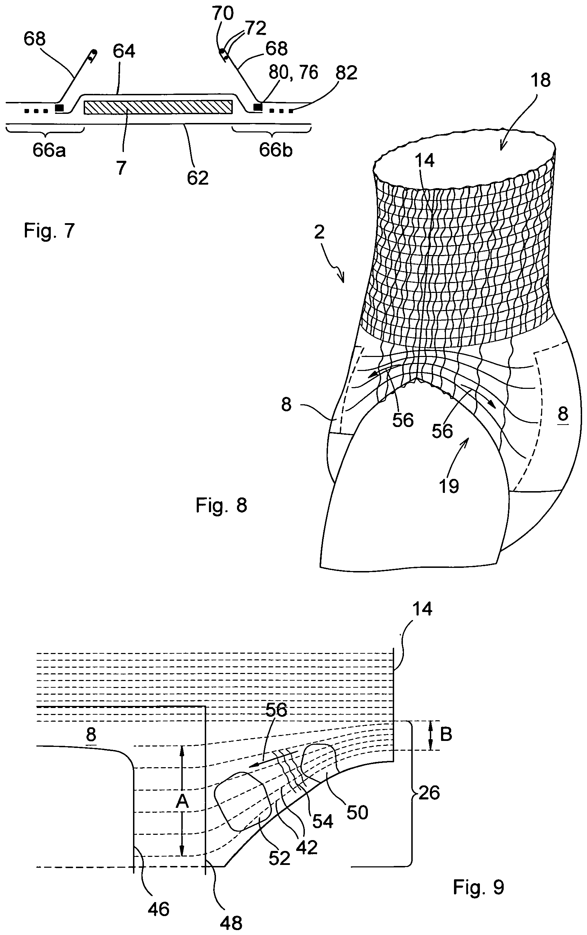

Wie aus den Figuren 1 und 2 ersichtlich, führt dies zu einer H-förmigen Grundstruktur des Inkontinenzartikels mit einer Längsrichtung 9. Die in Figuren 1 und 2 dargestellten miteinander gefügten Bestandteile werden dann zur Bildung der in Figur 8 schematisch dargestellten Höschenform an jeweiligen seitlichen Längsrandabschnitten 10, 12 des Bauchabschnitts 4 und des Rückenabschnitts 6 ebenfalls herstellerseitig miteinander verbunden, wodurch beidseits Seitennahtbereiche 14 (Figur 8) ausgebildet werden. In diesem herstellerseitig gefertigten höschenförmigen Zustand des Inkontinenzartikels erstrecken sich der Bauchabschnitt 4 und der Rückenabschnitt 6 in Quer- oder Hüftumfangsrichtung 16 durchgehend bis zu den Seitennahtbereichen 14 und definieren so eine in Hüftumfangsrichtung geschlossene Hüftöffnung 18 und Beinöffnungen 19, durch welche hindurch der Benutzer den Inkontinenzartikel wie ein Höschen anlegt.

Der Bauchabschnitt 4 lässt sich in einen hüftseitigen Bereich 20 und in einen schrittseitigen und den Beinöffnungen zugewandten Bereich 22 unterteilen. Eine entsprechende Unterteilung ist im Rückenabschnitt 6 vorgesehen, und zwar ebenfalls in einen hüftseitigen Bereich 24 und einen schrittseitigen und den Beinöffnungen zugewandten Bereich 26.

In dem hüftseitigen Bereich 20 des Bauchabschnitts 4 und in dem hüftseitigen Bereich 24 des Rückenabschnitts 6 sind erste Elastifizierungsmittel 28 vorgesehen, bei denen es sich insbesondere um fadenförmige Elastifizierungsmittel, wie Lycra®-Fäden, handeln kann, die in vorgedehntem Zustand, im sogenannten Stretch-Bond-Verfahren, mit den Flachmaterialien (Chassismaterialien) des Bauchabschnitts 4 und des Rückenabschnitts 6 verbunden sind. Diese ersten Elastifizierungsmittel 28 erstrecken sich in Quer- oder Hüftumfangsrichtung 16 von einem Seitennahtbereich 14 zum anderen.

Der schrittseitige und den Beinöffnungen 19 zugewandte Abschnitt 22 des Bauchabschnitts 4 bzw. 26 des Rückenabschnitts 6 haben eine von der Quer- oder Hüftumfangsrichtung 16 abweichende in Richtung auf eine Quermittelachse 30 des Schrittabschnitts 8 zulaufende Randkontur 32 bzw. 34. Diese Randkontur 32, 34 ist auch in der Darstellung nach Figur 1 oder 2 bogenförmig und daher zur Begrenzung der Beinöffnungen 19 geeignet. Durch diesen Verlauf des schrittseitigen und den Beinöffnungen zugewandten Bereichs 22 bzw. 26 wird auch ein verhältnismäßig großer Überlappungsbereich 36, 38 zwischen Schrittabschnitt 8 und Bauchabschnitt 4 bzw. Rückenabschnitt 6 realisiert, der im Hinblick auf eine reißfeste Verbindung von Schrittabschnitt 8 und Bauchabschnitt 4 bzw. Rückenabschnitt 6 wesentlich ist.

Der jeweilige schrittseitige und den Beinöffnungen 19 zugewandte Bereich 22, 26 des Bauchabschnitts 4 bzw. des Rückenabschnitts 6 ist ebenfalls elastifiziert ausgebildet. Dort sind zweite Elastifizierungsmittel 40 bzw. 42 vorgesehen. Die zweiten Elastifizierungsmittel 40, 42 erstrecken sich jeweils ausgehend von den Seitennahtbereichen 14 in Richtung auf eine Längsmittelachse 44 des Inkontinenzartikels. Wie aus den Figuren 1 und 7 zu erkennen ist, fächern die zweiten Elastifizierungsmittel 40, 42 in Richtung auf die Längsmittelachse 44 auf. Dies bedeutet, dass der Abstand zwischen ihnen in Richtung auf die Längsmittelachse 44 zunimmt. Diese Auffächerung der zweiten Elastifizierungsmittel 40 bzw. 42 lässt sich anhand Figur 9 auch quantitativ näher bezeichnen. Beispielsweise haben die in Figur 9 dargestellten zweiten Elastifizierungsmittel 42 des Rückenabschnitts 6 in den Seitennahtbereichen 14 einen minimalen Abstand von 3 bis 8 mm voneinander (Abstand von unmittelbar nebeneinander liegenden Elastifizierungsmitteln) und an einem Absorptionskörperrand 46 oder einem Längsrand 48 des Schrittabschnitts 8 einen maximalen Abstand (Abstand von unmittelbar nebeneinander liegenden Elastifizierungsmitteln) von 7 bis 35 mm voneinander. Es lässt sich anhand der Figur 9 auch ein Auffächerungsgrad F wie folgt definieren:

$$F = (A-B)/B * 100\%.$$

Dieser Auffächerungsgrad kann vorteilhafterweise zwischen 50 und 900 %, insbesondere zwischen 100 und 700 % und weiter insbesondere zwischen 150 bis 550 % liegen. Er ist im Rückenabschnitt 6 vorteilhafterweise größer als im Bauchabschnitt 4. Dabei sind die Größen A und B definiert als der Abstand des in Längsrichtung 9 äußersten zweiten Elastifizierungsmittels 40, 42 von dem in Längsrichtung 9 innersten zweiten Elastifizierungsmittel 40, 42 (also nicht der Abstand von unmittelbar nebeneinander liegenden Elastifizierungsmitteln), und zwar A als der maximale Abstand, insbesondere am Längsrand 48 des Schrittabschnitts 8, und B als der minimale Abstand, insbesondere im Seitennahtbereich 14 (vgl. Figur 9).

Sofern der Auffächerungsgrad bei den zweiten Elastifizierungsmitteln 40, 42 hinreichend groß gewählt wird, so lässt sich auf diese Weise eine abnehmende Rückstellkraft innerhalb des schrittseitigen und den Beinöffnungen 19 zugewandten Bereichs 22 beziehungsweise 26 in Richtung 56 auf den Schrittabschnitt 8 realisieren, sofern dafür Sorge getragen wird, dass in Folge des von der Hüft- oder Querrichtung 16 abgewandten bogenförmigen Verlaufs der zweiten Elastifizierungsmittel 40, 42 sich keine zu starke Erhöhung der Vorspannung in Folge des größeren Wegs dieser zweiten Elastifizierungsmittel 40, 42 ergibt. Betrachtet man dann ein näher am Seitennahtbereich 14 liegendes Gebiet 50 des betreffenden schrittseitigen Bereichs 22 beziehungsweise 26 mit einem näher am Schrittabschnitt 8 liegenden Gebiet 52, so ist die Rückstellkraft, die sich bei flächenhafter Dehnung des Gebiets 52 (Dehnung in Richtung der Elastifizierungsmittel 42) einstellt geringer als diejenige Rückstellkraft, die sich bei Dehnung des Gebiets 50 einstellt. Dies führt in vorteilhafter Weise weiter dazu, dass in Folge der geringeren elastischen Kräfte, welche im beispielhaft dargestellten Fall durch die zweiten Elastifizierungsmittel 40, 42 ausgeübt werden, die Chassismaterialien des Bauchabschnitts 4 und des Rückenabschnitts 6 weniger stark gerafft werden, so dass sich auch eine geringere Anzahl von Falten/Rüschen 54 ergibt, und zwar ausgehend vom jeweiligen Seitennahtbereich 14 in Richtung auf den Schrittabschnitt 8. Dadurch, dass die sich bei flächenhafter Dehnung des Bauchabschnitts in dem schrittseitigen und den Beinöffnungen zugewandten Bereich 22 des Bauchabschnitts 4 beziehungsweise 26 des Rückenabschnitts 6 sich einstellenden Rückstellkräfte in Richtung des Pfeils 56, also generell vom Seitennahtbereich 14 in Richtung auf den Schrittabschnitt 8 abnehmen, wird eine erhebliche Verbesserung des Tragekomforts erzielt, weil gerade in diesen Bereichen - wie festgestellt wurde - sich elastisch dehnbare Materialien als besonders problematisch erweisen, weil diese Materialien der Physiognomie der menschlichen Körperform entsprechend in diesen Bereichen besonders auf Zug und Dehnung beansprucht werden. Durch eine bewusst vorteilhaft vorgesehene Verringerung dieser Rückstellkraft, also abnehmende Rückstellkraft in Richtung des Pfeils 56, das heißt in Richtung zunehmender Annäherung an den Schrittabschnitt 8, wird hier ein zuvor nicht realisierter Freiheitsgrad geschaffen.

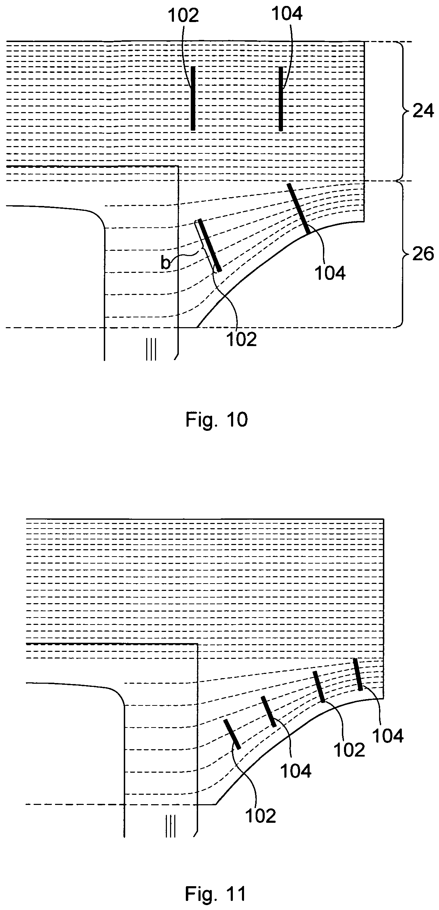

Wie eingangs ausgeführt, können Rückstellkräfte direkt am Chassis des Inkontinenzartikels bestimmt werden. Hierfür wird der betreffende Bereich des Bauchabschnitts 4 oder des Rückenabschnitts zwischen zwei Klemmbacken 102, 104 (siehe Fig. 10, 11) von definierter, gleicher Klemmbackenbreite (b) eingespannt, und es werden dann Rückstellkräfte bei einer definierten, den Gebrauchszustand simulierenden Dehnung der zu messenden Bereiche um insbesondere 30% oder 50% oder 80% der Ausgangslänge (des Klemmbackenabstands im ungespannten Zustand) ermittelt. Die Klemmbacken 102, 104 werden dabei jeweils voneinander wegbewegt. Die Klemmbacken 102, 104 sollten möglichst viele, zumindest jedoch zwei nebeneinander angeordnete Elastifizierungsmittel 40,42 des zu messenden Bereichs fixieren, und sie sollten im Wesentlichen rechtwinklig zum Verlauf der Elastifzierungsmittel orientiert sein, so das die Dehnung zwischen den Klemmbacken 102, 104, also die Auseinanderbewegung der Klemmbacken 102, 104, im Wesentlichen in Richtung des Verlaufs der Elastifizierungsmittel erfolgt. Dies ist in Figuren 10 und 11 verwirklicht.

Bei der dargestellten bevorzugten Ausführungsform des Inkontinenzartikels 2 beträgt ein Abstand C des schrittzugewandten innersten zweiten Elastifizierungsmittels 40 des Bauchabschnitts 4 von dem entsprechenden schrittzugewandten innersten zweiten Elastifizierungsmittel 42 des Rückenabschnitts 6 zwischen 250 bis 420 mm je nach Konfektionsgröße des Inkontinenzartikels. Die zweiten Elastifizierungsmittel 40, 42 erstrecken sich im Wesentlichen bis zum schrittzugewandten Querrand 58, 60 des Bauchabschnitts 4 und des Rückenabschnitts 6. Der Abstand von Bauchabschnitt 4 und Rückenabschnitt 6 voneinander beträgt 250 - 400 mm.

Der Abstand der innersten, schrittzugewandten zweiten Elastifizierungsmittel 40, 42 von der die Beinöffnungen begrenzenden Randkontur 32, 34 des schrittseitigen und den Beinöffnungen zugewandten Bereichs 22, 26 des Bauchabschnitts 4 und des Rückenabschnitts 6 beträgt vorzugsweise 2 - 40 mm, weiter vorzugsweise 3 - 30 mm, insbesondere vorzugsweise 4 - 15 mm.

Die Erstreckung des Bauchabschnitts 4 und des Rückenabschnitts 6 im Seitennahtbereich 14 in Längsrichtung 9 beträgt vorteilhafterweise zwischen 100 und 220 mm. Die Erstreckung des Schrittabschnitts 8 in Querrichtung 16 beträgt vorteilhafterweise 200 bis 350 mm.

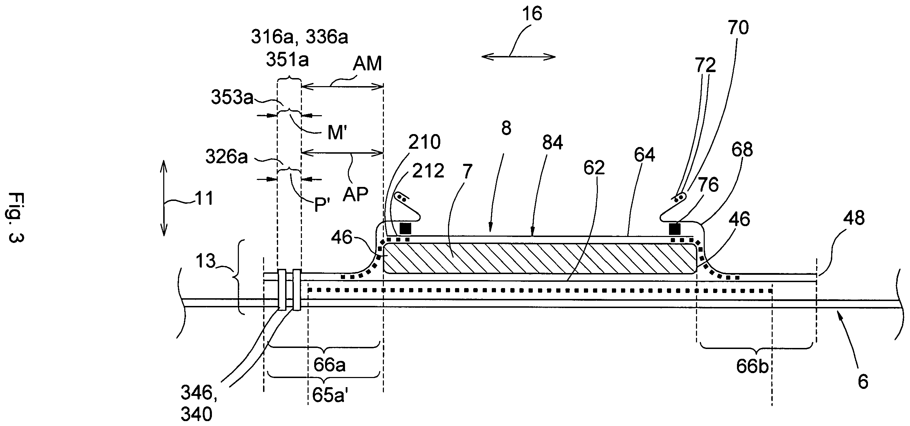

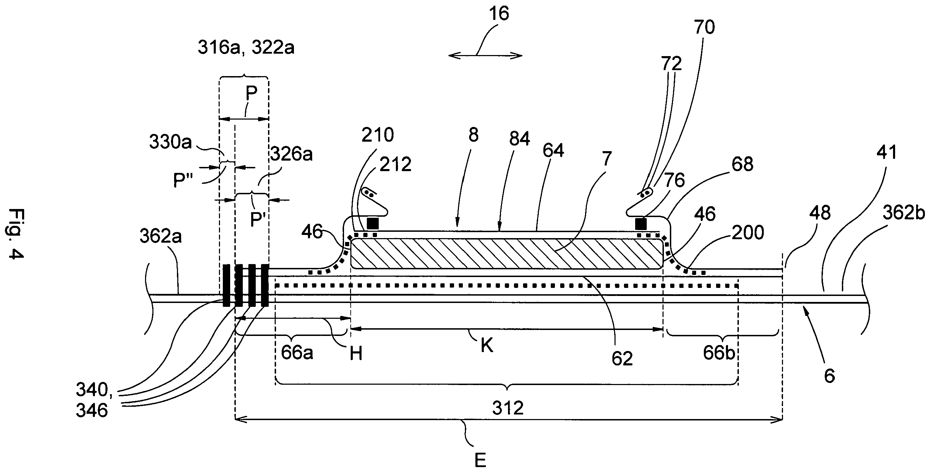

Wie in Figuren 3 und 4 ersichtlich, welche schematisch jeweils eine Schnittansicht entlang der Schnittebene III - III aus der Figur 1 bzw. entlang der Schnittebene IV - IV aus der Figur 2 darstellt, umfasst der Schrittabschnitt 8 ein flüssigkeitsundurchlässiges Backsheet-Material 62, das insbesondere von einem atmungsaktiven, jedoch flüssigkeitsdichten Folienmaterial gebildet sein kann, und ein, vorzugsweise auf Vliesbasis hergestelltes Deckblatt-Material 84, welches ein Verbund aus einem auf Vliesbasis hergestelltem Topsheet-Material 64 und aus beidseits angeordneten Barrieremitteln 68 ist. Zwischen dem Backsheet-Material und dem Topsheet-Material ist, wie aus der Figur 3 bzw. 4 ersichtlich, der Absorptionskörper 7 angeordnet. Der Absorptionskörper 7 weist Längsränder 46 auf. Im beispielhaft dargestellten Fall bildet das Backsheet-Material 62 in Querrichtung 16 beidseits der Längsränder 46 jeweils einen Überhang 66a, 66b. Das Topsheet 64 überragt den Absorptionskörper 7 in Querrichtung nur verhältnismäßig geringfügig; jedoch ist beidseits des Absorptionskörpers 7 in Längsrichtung 9 verlaufend jeweils ein aufstehendes Barrieremittel 68 vorgesehen, welches typischerweise als aufstehendes Cuffelement oder Bündchenelement bezeichnet wird und vorzugsweise aus einem hydrophoben, insbesondere flüssigkeitsundurchlässigen Vliesstoffmaterial gebildet ist, welches sich in Querrichtung 16 vorzugsweise bis zu den seitlichen Längsrändern 48 des Schrittabschnitts 8 erstreckt. Das Barrieremittel 68 ist dabei in Fügestellen 76 an den Längsrändern 210 bzw. den Längsrandbereichen 212 des Topsheet-Materials 64 angefügt. Die distalen Enden 70 der Barrieremittel 68 sind mit weiteren Elastifizierungsmitteln 72 versehen, welche die Barrieremittel 68 im Gebrauch des Inkontinenzartikels gegen die Hautoberfläche des Benutzers anheben, so wie dies in Figur 7, eine schematische Schnittansicht entlang der Schnittebene VII - VII aus der Figur 1 bzw. Figur 2, dargestellt ist. Die in Längsrichtung 9 angeordeten Fügestellen 76 bilden die durchgehend erstreckte Cuffsockellinie 80.

Der erwähnte Überhang 66a, 66b des Backsheet-Materials 62 und/oder des Deckblatt-Materials 84 beidseits der Längsränder 46 des Absorptionskörpers 7, also in der Summe, beträgt wenigstens 25 % bezogen auf die größte Breite E des Schrittabschnitts 8 . Durch einen großen Überhang des Backsheet-Materials oder des Deckblatt-Materials und des Backsheet-Materials in Querrichtung jeweils außerhalb der Längsränder des Absorptionskörpers kann aufgrund des damit einhergehenden großen Überlappungsbereichs von Schrittabschnitt und Bauchabschnitt bzw. von Schrittabschnitt und Rückenabschnitt der Schrittabschnitt vorteilhaft herstellerseitig sicher an Rücken- und Bauchabschnitt festgelegt werden.

Ein großer Überhang ist zusätzlich vorteilhaft, da auf diese Weise nämlich in Querrichtung 16 Raum für die Anordnung entlang der Beinöffnungen 19 erstreckter Beinelastifizierungsmittel 82 ist. Es erweist sich nämlich als vorteilhaft, wenn die Beinelastifizierungsmittel 82 mit einem gewissen Abstand vom materialreichen und damit eher biegesteifen Absorptionskörper 7 verlaufen, um einerseits keine zusätzlichen Dehnungs- oder Verwindungskräfte auf den Absorptionskörper auszuüben, was dessen Absorptionsverhalten nachteilig beeinflussen könnte, und um andererseits einen vom Absorptionskörper weitgehend unbeeinflussten flüssigkeitsdichten Beinabschluss zu realisieren. Es erweist sich im dargestellten Fall als besonders vorteilhaft, dass diese Beinelastifizierungsmittel 82 in Längsrichtung 9 mit einem deutlichen Abstand von insbesondere wenigstens 10 mm, vorzugsweise wenigstens 20 mm vor den zweiten Elastifizierungsmitteln 40 und 42 des Bauchabschnitts 4 beziehungsweise des Rückenabschnitts 6 enden. Vorzugsweise enden diese Beinelastifizierungsmittel 82 in Längsrichtung 9 vor dem Bauchabschnitt 4 und dem Rückenabschnitt 6. Dies ist deshalb vorteilhaft und wesentlich, weil die Beinelastifizierungsmittel 82 solchenfalls das Spannungsverhalten des Bauchabschnitts 4 und des Rückenabschnitts 6 wenig oder gar nicht beeinflussen. Es wurde nämlich erkannt, dass es sich im Hinblick auf das vorteilhaft zu erreichende Ziel der Verbesserung des Tragekomforts gerade in dem schrittseitigen und den Beinöffnungen 19 zugewandten Bereich 22 und 26 des Bauchabschnitts 4 und des Rückenabschnitts 6 als negativ erweist, wenn dort die üblicherweise mit großer Vorspannung und entsprechend großer Rückstellkraft ausgebildeten Beinelastifizierungsmittel 82 zusätzlich verlaufen.

Wie aus Figur 3 bzw. 4 in Verbindung mit Figur 1 bzw. 2 ersichtlich ist, ist bei dem Schrittabschnitt 8 ein in Querrichtung 16 verhältnismäßig großer Überhang 66a, 66b beidseits der Längsränder 46 des Absorptionskörpers 7 realisiert, und zwar insbesondere auch an dem Bauchabschnitt 4 beziehungsweise dem Rückenabschnitt 6 zugewandten Bereichen des Schrittabschnitts 8. Hierdurch wird - worauf bereits hingewiesen wurde - ein verhältnismäßig großer Überlappungsbereich 36, 38 des Schrittabschnitts 8 mit dem Bauchabschnitt 4 und mit dem Rückenabschnitt 6 realisiert. Nach einer bevorzugten Ausführungsvariante umfasst der Überlappungsbereich 36 von Schrittabschnitt 8 mit dem Bauchabschnitt 4 wenigstens 12% der Fläche des Bauchabschnitts 4, und der Überlappungsbereich 38 von Schrittabschnitt 8 mit dem Rückenabschnitt 6 umfasst wenigstens 20% der Fläche des Rückenabschnitts 6. Dies erweist sich als vorteilhaft, da solchenfalls eine sichere Fixierung des Schrittabschnitts 8 an dem Bauchabschnitt 4 beziehungsweise an dem Rückenabschnitt 6 erreicht werden kann.

Der Überhang 66a, 66b weist dabei "erste Bereiche" 65a, 65a', 65b, 65b' und "zweite Bereiche" 67a, 67b auf (siehe Figur 1), wobei ein jeweils erster Bereich sich beidseits der Längsränder 46 des Absorptionskörpers 7 im vorderen und hinteren Überlappungsbereich 36, 38 erstreckt. Ein jeweiliger zweiter Bereich 67a, 67b erstreckt sich beidseits der Längsränder des Absorptionskörpers zwischen den schrittzugewandten Querrändern 58, 60 des Bauchabschnittes 4 und des Rückenabschnitts 6, also außerhalb der Überlappungsbereiche.

Wie in Figur 1 und 2 ersichtlich ist, weist der Inkontinenzartikel 2 die erfindungsgemäßen ersten Fügebereiche 310, 312 und zweiten Fügebereiche 314a, 314b, 316a, 316b auf, welche im vorderen und im hinteren Überlappungsbereich 36, 38 angeordnet sind und damit einen vorderen und einen hinteren Verbindungsbereich 306, 308 bereitstellen, in denen der Schrittabschnitt 8 an den Bauchabschnitt 4 bzw. an den Rückenabschnitt 6 unlösbar angefügt ist.

Figur 1 zeigt weiter schematisch die zweiten Fügebereiche 314a, 314b, 316a, 316b, welche zumindest innerhalb der ersten Bereiche 65a, 65a', 65b, 65b' des Überhangs 66a, 66b des Schrittabschnitts 8 angeordnet sind, so dass die zweiten Fügebereiche 314a, 314b, 316a, 316b Verstärkungsbereiche 334a, 334b, 336a, 336b bilden. In Zusammenschau mit Figur 3, welche eine schematische Schnittansicht entlang der Ebene III - III aus Figur 1 darstellt (dabei wurde aber nur linkerhand der zweite Fügebereich schematisch eingezeichnet), sind auch die luftdurchlässigen Bereich 350a, 350b, 351 a, 351 b erläutert, welche im gezeigten Ausführungsbeispiel insbesondere vorteilhaft in ihrer flächenhaften Erstreckung mit den jeweiligen Verstärkungsbereichen 334a, 334b, 336a, 336b übereinstimmen.

In den Überlappungsbereichen 36, 38 liegt ein Lagenaufbau 13 in Z-Richtung 11 vor, wobei die Z-Richtung zu der durch die Längsrichtung 9 und Querrichtung 16 beschriebenen Ebene senkrecht steht. So wird ein Lagenaufbau 13 beispielsweise durch Überlappung von Schrittabschnitt 8 mit dem Rückenabschnitt 6 gebildet (siehe Figur 3).

Aus Figur 3 wird ersichtlich, dass der im hinteren Überlappungsbereich angeordnete erste Bereich 65a' des Überhangs 66a sich durch den Lagenaufbau 13 in Z-Richtung 11 hindurcherstreckende Öffnungen 346 aufweist.. Der zweite Fügebereich 316a überfängt dabei einen Teilbereich 326a des Überhangs 66a in Querrichtung 16 mit einer Breite P'. Der durch die Öffnungen 346 gebildete luftdurchlässige Bereich 351a überfängt einen Teilbereich 353a des Überhangs 66a in Querrichtung 16 mit einer Breite M'. Die Öffnungen 346 sind durch Fügemittel 340 in Form von Schweißstellen gebildet. Der dargestellte zweite Fügebereich 316a bildet einen Verstärkungsbereich 336a und stellt mittels der innerhalb des zweiten Fügebereichs angeordneten Öffnungen 346 zudem den luftdurchlässigen Bereich 35a dar. Beide Bereiche stimmen in ihrer flächenhaften Erstreckung überein und sind kongruent übereinander gelagert. Der zweite Fügebereich 316a endet in einem Abstand AP vor dem Längsrand 46 des Absorptionskörpers 7. Ebenso endet der luftdurchlässige Bereich 351 a vor dem Längsrand 46 des Absorptionskörpers 7 in einem Abstand AM.

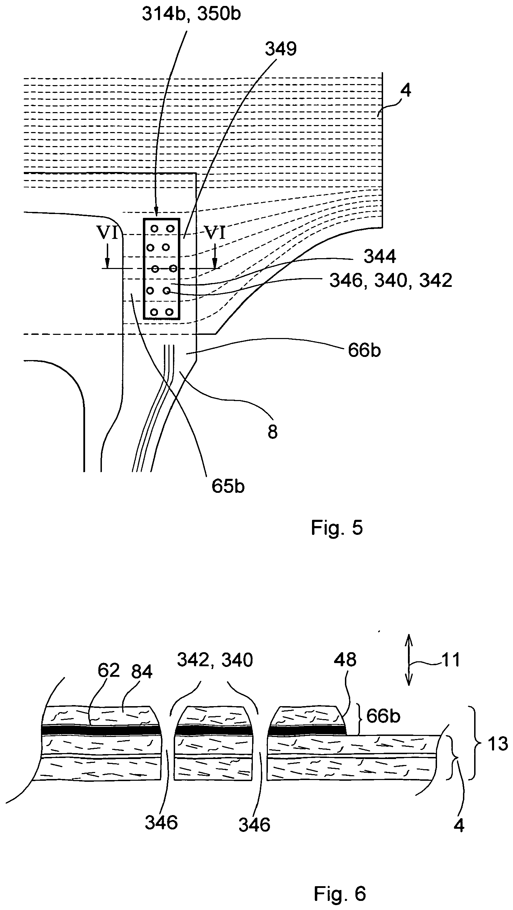

Wie weiter in Figur 5 dargestellt (hier ein Ausschnitt des Inkontinenzartikels mit der Bindung des Schnittabschnitts 8 an den Bauchabschnitt 4), sind die Öffnungen 346 innerhalb des ersten Bereichs 65b des Überhangs 66b punktförmig angeordnet. Die Öffnungen sind mittels Fügemittel 340 in Form von Ultraschallschweißstellen eingebracht. Die Fügemittel bilden jeweils eine Fügestelle (342), welche von der nächsten Fügestelle durch einen ungebundenen Bereich 344 beabstandet ist. Entsprechend sind die Öffnungen 346 zueinander durch einen nicht perforierten Bereich 344 beabstandet. Zur Bestimmung der flächenhaften Erstreckung eines zweiten Fügebereiches (hier 314b) und des luftdurchlässigen Bereichs 350b werden die jeweils äußersten Fügemittel 340 bzw. Fügestellen 342 bzw. Öffnungen 346 an deren äußersten Randkanten mittels einer imaginären Linie 349 verbunden.

Die im Punktmuster angeordneten Öffnungen 346 als auch die Fügemittel 340 bilden in der vom luftdurchlässigen Bereich 350b bzw. in der vom zweiten Fügebereich 314b überfangenen Gesamtfläche Öffnungen bzw. Fügestellenflächen, welche in Summe vorzugsweise mindestens 1,5%, insbesondere mindestens 2,0%, weiter insbesondere mindestens 2,3%, weiter insbesondere mindestens 2,5 % und vorzugsweise höchstens 20,0%, insbesondere höchstens 15,0%, weiter insbesondere höchstens 10,0%, weiter insbesondere höchstens 8,0%, weiter insbesondere höchstens 7,0%, weiter insbesondere höchstens 6,0% bezogen auf die vom luftdurchlässigen Bereich bzw. vom zweiten Fügebereich überfangene Gesamtfläche einnehmen. Die einzelnen Öffnungen bzw. Fügemittel weisen einen Durchmesser vorzugsweise von mindestens 0,2 mm, insbesondere von mindestens 0,3 mm, weiter insbesondere von mindestens 0,4 mm und vorzugsweise von höchstens 2,5 mm, insbesondere höchstens 2,0 mm, weiter insbesondere höchstens 1,5 mm, weiter insbesondere höchstens 1,2 mm, insbesondere höchstens 1,0 mm auf. Die benachbarten einzelnen in dem Punktmuster vorhandenen Öffnungen 346 bzw. Fügemittel 340 haben einen Abstand voneinander von vorzugsweise 1 - 10 mm, insbesondere von 1 - 8 mm, weiter insbesondere von 1 - 6 mm, weiter insbesondere von 1 - 5 mm, weiter insbesondere von 1,5 - 4,5 mm, weiter insbesondere von 2 - 4 mm.

Figur 6 stellt eine Schnittansicht des luftdurchlässigen Bereichs 350b bzw. des zweiten Fügebereichs 314 a entlang der Schnittebene VI-VI aus der Figur 5 dar. Der Überhang 66b des Schrittabschnitts 8 bestehend aus einem Deckblatt-Material 84 aus Vlies und einem Backsheet 62 aus Folie ist mittels durch die Fügemittel 340 erzeugten Fügestellen 342 an den darunter liegenden Bauchabschnitt 4 gebunden. Zudem sind durch die Fügemittel 340 durch den Lagenaufbau 13 in Z-Richtung 11 sich hindurch erstreckende Öffnungen 346 eingebracht.

Zusammen mit der Figur 4, welche eine schematische Schnittansicht entlang der Ebene IV - IV aus Figur 2 darstellt (dabei wurde aber nur linkerhand der zweite Fügebereich schematisch eingezeichnet), wird eine weitere bevorzugte Ausführungsform näher dargestellt, nämlich dass der zweite Fügebereich dabei sowohl innerhalb des ersten Bereichs des Überhangs angeordnet ist und dabei jeweils einen Teilbereich des Überhangs überfängt, und dann zusätzlich über den Längsrand des Schrittteils in den daran angrenzenden Teilbereich des Bauchabschnitts und/oder des Rückenabschnitts übergeht, also somit als ein den jeweiligen Längsrand des Schrittabschnitts überbrückenden Bereich in den Inkontinenzartikel eingebracht wird.

Es wird ersichtlich, dass der zweite Fügebereich 314a, 314b, 316a, 316b dabei in einem den jeweiligen Längsrand 48 des Schrittabschnitts 8 überbrückenden Bereich 320a, 320b, 322a, 322b angeordnet ist. Damit wird durch den zweiten Fügebereich 314a, 314b, 316a, 316b mit den darin angeordneten Fügemitteln 340 jeweils sowohl ein Teilbereich 324a, 324b, 326a, 326b des Überhangs 66a, 66b als auch ein an den Längsrand 48 angrenzender Teilbereich 328a, 328b, 330a, 330b des Bauchabschnitts 4 oder Rückenabschnitts 6 überfangen. Diese zweiten Fügebereiche 314a, 314b, 316a, 316b bilden jeweils einen Verstärkungsbereich 334a, 334b, 336a, 336b aus. Die zweiten Fügebereiche 314a, 314b, 316a, 316b und damit die Verstärkungsbereiche 334a, 334b, 336a, 336b erstrecken sich in Längsrichtung 9 betrachtet beginnend vom schrittzugewandten Querrand 58, 60 des Bauchabschnitts 4 und des Rückenabschnitts 6 kontinuierlich in Richtung auf die Längsenden des Schrittabschnitts, vorzugsweise bis mindestens zu einem jeweiligen Längsende 98, 100 des Schrittabschnitts 8.

Die zweiten Fügebereiche 314a, 314b, 316a, 316b weisen eine Gesamtbreite P von vorzugsweise 5 - 60 mm, insbesondere von 10 - 50 mm, insbesondere von 10 - 40 mm, weiter insbesondere 10 - 30 mm auf. In dem vom zweiten Fügebereich 314a, 314b, 316a, 316b überfangenden Teilbereich 324a, 324b, 326a, 326b des jeweiligen Überhangs 66a, 66b weist der zweite Fügebereich eine Breite P' von vorzugsweise größer 1 mm, weiter vorzugsweise größer 2 mm, weiter vorzugsweise größer 5 mm und aber vorzugsweise kleiner 60 mm, weiter vorzugsweise kleiner 50 mm, weiter vorzugsweise kleiner 40 mm, weiter vorzugsweise kleiner 30 mm, weiter vorzugsweise kleiner 20 mm, insbesondere vorzugsweise von 10 mm auf.

Der zweite Fügebereich 314a, 314b, 316a, 316b überfängt den jeweiligen Teilbereich 324a, 324b, 326a, 326b des Überhangs 66a, 66b in Querrichtung derart, dass der Anteil Anteil P'/ H des durch den zweiten Fügebereich 314a, 314b, 316a, 316b überfangenen Teilbereiches 324a, 324b, 326a, 326b des jeweiligen Überhangs 66a, 66b mit der Breite P' bezogen auf den jeweiligen Überhang 66a, 66b mit der Breite H vorzugsweise mindestens 0,01, insbesondere mindestens 0,04, weiter insbesondere vorzugsweise mindestens 0,07, weiter insbesondere mindestens 0,10, und aber vorzugsweise höchstens 0,90, weiter insbesondere höchstens 0,80, weiter insbesondere höchstens 0,70, weiter insbesondere höchstens 0,60, weiter insbesondere höchstens 0,50, weiter insbesondere höchstens 0,40 beträgt.

In dem vom zweiten Fügebereich 314a, 314b, 316a, 316b überfangenden Teilbereich 328a, 328b, 330a, 330b des Bauchabschnitts 4 oder Rückenabschnitts 6 weist der zweite Fügebereich eine Breite P" vorzugsweise größer 1 mm, weiter vorzugsweise größer 2 mm, weiter vorzugsweise größer 5 mm und aber vorzugsweise kleiner 60 mm, weiter vorzugsweise kleiner 50 mm, weiter vorzugsweise kleiner 40 mm, weiter vorzugsweise kleiner 30 mm, weiter vorzugsweise kleiner 20 mm, insbesondere vorzugsweise von 10 mm auf.

Der zweite Fügebereich 314a, 314b, 316a, 316b überfängt den jeweiligen Teilbereich 328a, 328b, 330a, 330b des Bauchabschnitts 4 und des Rückenabschnitts 6 in Querrichtung 16 derart, dass der Anteil P"/ H des jeweiligen durch den zweiten Fügebereich 314a, 314b, 316a, 316b überfangenen Teilbereiches 328a, 328b, 330a, 330b des Bauchabschnitts und Rückenabschnitts mit der Breite P" bezogen auf den jeweiligen Überhang 66a, 66b mit der Breite H vorzugsweise mindestens 0,01, insbesondere mindestens 0,04, weiter insbesondere mindestens 0,07, weiter insbesondere mindestens 0,10 und aber vorzugsweise höchstens 0,90, insbesondere höchstens 0,80, weiter insbesondere höchstens 0,70, weiter insbesondere höchstens 0,60, weiter insbesondere höchstens 0,50 und weiter insbesondere höchstens 0,40 beträgt.

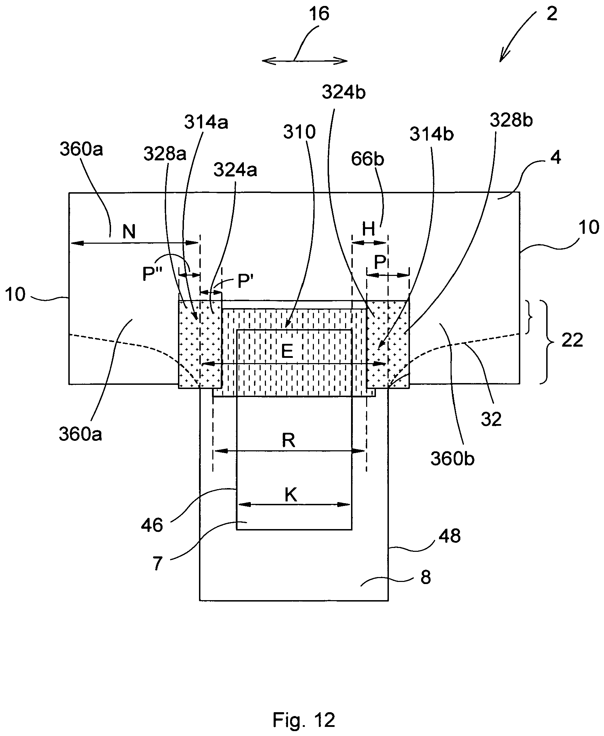

Wie voranstehend erläutert, überfängt der zweite Fügebereich 314a, 314b, 316a, 316b einen angrenzenden Teilbereich 328a, 328b, 330a, 330b des Bauchabschnitts und des Rückenabschnitts. Zwischen dem Längsrand 48 des Schrittabschnitts 8 und dem Längsrandabschnitt 10, 12 des Bauchabschnitts 4 und Rückenabschnitts 6 sind die jeweiligen Seitenbereiche 360a, 360b, 362a, 362b. Der jeweilige Seitenbereich weist eine Breite N auf. Wie in Figur 12 weiter schematisch dargestellt, wird durch den zweiten Fügebereich 314a, 314b (bzw. 316a, 316b) mit der anteiligen Breite P" der jeweilige Seitenbereich 360a, 360b (bzw. 362a, 362b) des Bauchabschnitts bzw. des Rückenabschnitts mit der Breite N überfangen. In Figur 12 ist der Bauchabschnitt bzw. Rückenabschnitt als rechteckförmiges Panel dargestellt. Der dem Schritt zugewandte Bereich 22 kann aber, so wie in Figur 1 dargestellt und auch wie in Figur 12 gestrichelt eingezeichnet, eine bogenförmige Randkontur 32 einnehmen.

Der Anteil P"/N des jeweiligen durch den zweiten Fügebereich 314a, 314b, 316a, 316b überfangenen Teilbereiches 328a, 328b, 330a, 330b des Bauchabschnitts und/oder Rückenabschnitts mit der Breite P" bezogen den jeweiligen Seitenbereich 360a, 360b, 362a, 362b des Bauchabschnitts und/oder Rückenabschnitts mit der Breite N beträgt vorzugsweise mindestens 0,01, insbesondere mindestens 0,015, weiter insbesondere mindestens 0,020, und vorzugsweise höchstens 0,35, insbesondere höchstens 0,30, weiter insbesondere höchstens 0,25, weiter insbesondere höchstens 0,20, weiter insbesondere höchstens 0,15, weiter insbesondere höchstens 0,10.

In einer vorteilhaften Weise beträgt der Anteil P'/N des jeweiligen durch den zweiten Fügebereich 314a, 314b, 316a, 316b überfangenen Teilbereiches 324a, 324b, 326a, 326b des jeweiligen Überhangs 66a, 66b mit der Breite P' bezogen den jeweiligen Seitenbereich 360a, 360b, 362a, 362b des Bauchabschnitts und/oder Rückenabschnitts mit der Breite N vorzugsweise mindestens 0,01, insbesondere mindestens 0,015, weiter insbesondere mindestens 0,020 und vorzugsweise höchstens 0,35, insbesondere höchstens 0,30, weiter insbesondere höchstens 0,25, weiter insbesondere höchstens 0,20, weiter insbesondere höchstens 0,15, weiter insbesondere höchstens 0,10.

Die in der Figur 4 zusammen mit Figur 2 schematisch dargestellte Ausführung des Inkontinenzartikels weist zweite Fügebereiche auf, welche dabei sowohl innerhalb des ersten Bereichs des Überhangs angeordnet ist und dabei jeweils einen Teilbereich des Überhangs überfangen, und dann zusätzlich über den Längsrand des Schrittteils in den daran angrenzenden Teilbereich des Bauchabschnitts und/oder des Rückenabschnitts übergehen. Durch die in den zweiten Fügebereich eingebrachten Fügemittel 340 sind gleichzeitig die Öffnungen 346 gebildet, mittels Fügemitteln in Form von Schweißstellen, bevorzugt Ultraschallschweißstellen, thermischen Schweißstellen, Kalanderschweißstellen.

Wie in Figur 4 dargestellt, verläuft der erste Fügebereich 312 unterhalb des Absorptionskörpers 7 mit der Breite K, erstreckt sich also auch über die Kontur, also den jeweiligen Längsrand 46 des Absorptionskörpers 7 hinaus, jedoch nicht außerhalb der Kontur des Schrittabschnitts 8, sondern endet innerhalb der Längsränder 48 des Schrittabschnitts 8. Der Schrittabschnitt 8 ist dabei vorzugsweise mittels eines vollflächigen Kleberauftrags mit dem Rückenabschnitt 6 verbunden.

Das Deckblatt-Material ist, wie im vorliegenden Beispiel, ein Verbund aus einem Topsheet 64 und beidseits an den Längsrändern 210 bzw. den Längsrandbereichen 212 des Topsheets 64 angefügten Barrieremitteln 68. Das Deckblatt-Material ist dabei mittels eines Klebers 200, insbesondere eines Hotmeltklebers, an den Absorptionskörper und den Seitenrand 46 des Absorptionskörpers 7 überlappend auch noch an einen angrenzenden Teilbereich des Backsheet-Materials 62 gebunden.

Auch zwischen Backsheet-Material und Absorptionskörper und zwischen Deckblatt-Material und Absorptionskörper ist ein separates Fügemittel vorgesehen (in der Figur 4 nicht dargestellt). Diese separaten Fügemittel sind in Form eines Klebers nicht vollflächig, sondern in Form eines unterbrochenen Musters aufgetragen. Diese in Form eines Klebers vorgesehenen separaten Fügemittel sind also beispielsweise rasterförmig, streifenförmig oder als Spiralmuster aufgetragen.

Figur 12 zeigt schematisch einen noch nicht fertig gestellten Inkontinenzartikel 2 mit einem an den Schrittabschnitt 8 angefügten Bauchabschnitt 4 und eine vorteilhafte Ausführung der Anordnung des ersten Fügebereichs 310 und der jeweils zweiten Fügebereiche 314a, 314b. Wie in Figur 12 dargestellt, erstreckt sich der erste Fügebereich 310 in Querrichtung 16 mit der Breite R vorteilhafterweise über die Längsränder 46 des Absorptionskörpers 7 mit einer Breite K hinaus, aber ohne sich über die Längsränder 48 des Schrittabschnitts 8 hinauszustrecken. Der jeweils zweite Fügebereich 314a, 314b mit Breite P überbrückt jeweils den Längsrand 48 des Schrittabschnitts 8. Der Fügebereich 314a, 314b überfängt dabei jeweils einen angrenzenden Teilbereich 324a, 324b des Überhangs 66a, 66b mit der Breite H, uns zwar mit der Breite P'. Der zweite Fügebereich 314a, 314b überfängt auch jeweils einen angrenzenden Teilbereich 328a, 328b des Bauchabschnitts 4, und zwar mit der Breite P". Der erste Fügebereich 310 erstreckt sich dabei derart in Querrichtung, dass eine Überlappung mit dem jeweiligen zweiten Fügebereich 324a, 324b erhalten wird.

Ein Inkontinenzartikel in Höschenform gebildet aus den drei Komponenten Schrittabschnitt, Bauchabschnitt und Rückenabschnitt, wobei der Schrittabschnitt in Überlappungsbereichen unter Ausbildung eines Lagenaufbaus in Z-Richtung an den Bauchabschnitt und Rückenabschnitt unlösbar angefügt ist, weist erfindungsgemäß im ersten Bereich des Überhangs sich durch diesen Lagenaufbau in Z-Richtung hindurcherstreckende Öffnungen auf. Die Öffnungen bilden einen luftdurchlässigen Bereich aus, wie vorangehend an den schematischen Darstellungen der Figuren erläutert wird. In der in Figur 5 skizzenhaft dargestellten Ausführungsform ist innerhalb des ersten Bereichs 65b der die Öffnungen 346 aufweisende luftdurchlässige Bereich 350b angeordnet, umgeben von einem Bereich ohne Öffnungen. Die Öffnungen sind in einem Punktmuster angeordnet, wobei die Öffnungen mit einem Durchmesser von 0,5 mm in Summe einen Anteil von 2,8% bezogen auf die vom luftdurchlässigen Bereich überfangene Gesamtfläche einnehmen. Der luftdurchlässige Bereich weist dabei eine Luftdurchlässigkeit auf, welche um den Faktor 1,5, insbesondere 2, insbesondere 2,5, weiter insbesondere 3, weiter insbesondere höchstens 5, weiter insbesondere höchstens 4,5 größer ist als die Luftdurchlässigkeit im ersten Bereich des Überhangs außerhalb des luftdurchlässigen Bereichs (350b). Der luftdurchlässige Bereich weist in diesem Ausführungsbeispiel eine Breite M' von 20 mm auf, und erstreckt sich in Längsrichtung betrachtet im Wesentlichen beginnend vom schrittzugewandten Querrand des Bauchabschnitts 4 in Richtung auf das Längsende des Schrittabschnitts. Aus dem luftdurchlässigen Bereich wird ein Prüfling mit entsprechend angepassten Abmessungen ausgestanzt, im konkreten Anwendungsbeispiel 10 x 50 mm. Der aus diesem Lagenaufbau ausgestanzte Prüfling umfasst dabei die von Bauchabschnitt und Schrittabschnitt eingebrachten Lagen. Der Prüfling weist im Ausführungsbeispiel zwei Lagen Vlies aus dem Bauchabschnitt, eine Lage Deckblatt-Material in Form eines SMS-Laminats und das Backsheet-Material als ein Vlies-Folien-Laminat mit einer mikroporösen Folie auf. Die Untersuchung der Luftdurchlässigkeit erfolgt nach der eingangs beschriebenen Messmethode. Das zur Messung der Luftdurchlässigkeit für die Prüffläche von 20cm$^2$ notwendige Prüfmuster 1 wird hergestellt, indem der aus dem luftdurchlässigen Bereich 350b ausgestanzte Prüfling mittels eines Klebebands, wie beispielsweise tesafilm® kristall klar, 15 mm x 10 m der Firma Tesa, in den ausgestanzten Bereich (7 x 45 mm) eines Trägermaterial, wie beispielsweise der luft- und wasserdichten Trägerfolie PE Folie HyFol PP Plus, 16 $\mu$m (RKW Wasserburg, Deutschland) mit Überlappung beidseitig festgeklebt wird. Bei der Messung sind neben dem hergestellten Prüfmuster 1 auch noch Messungen zum Trägermaterial als solchem und zum Trägermaterial mit der mit Klebeband überklebten Ausstanzung als Nullkontrolle und Negativkontrolle durchzuführen und bei der Auswertung zu berücksichtigen. Die Prüfmuster und Kontrollmuster werden bei 23 °C und 50% relativer Luftfeuchtigkeit 24 Stunden konditioniert und dann bei einem Differenzdruck von 200 Pa nach der eingangs beschriebenen Methode gemessen. Zur vergleichenden Untersuchung der Luftdurchlässigkeit wird aus dem den luftdurchlässigen Bereichs 350b umgebenen Bereich in analoger Weise ein entsprechendes Prüfmuster 2 hergestellt und ebenso der Messung unterzogen. Der luftdurchlässige Bereich (Prüfmuster 1) weist unter Berücksichtigung der Kontrollproben eine Luftdurchlässigkeit von durchschnittlich 121 l/m$^2$/s auf, der diesen luftdurchlässigen Bereich umgebende Bereich (Prüfmuster 2) weist unter Berücksichtigung der Kontrollproben eine Luftdurchlässigkeit von durchschnittlich 48 l/m$^2$/s auf (Tabelle 1). Der luftdurchlässige Bereich weist vergleichsweise eine um den Faktor 2,5 größere Luftdurchlässigkeit auf. Durch die in den ersten Bereich des Überhangs eingebrachten erfindungswesentlichen Öffnungen wird ein Inkontinenzartikel mit verbessertem Tragekomfort bereitgestellt.

Zur vergleichenden Untersuchung der Wasserdampfdurchlässigkeit durch den Lagenaufbau des luftdurchlässigen Bereichs mit der Wasserdampfdurchlässigkeit des ebenfalls im ersten Bereich des Überhangs, aber außerhalb des luftdurchlässigen Bereich angeordneten Lagenaufbaus, werden in analoger Weise entsprechend Prüfmuster 1 und Prüfmuster 2 und Kontrollmuster hergestellt, so wie vorangehend für die Messung der Luftdurchlässigkeit beschrieben ist.

Bei der Prüfung der Wasserdampfdurchlässigkeit werden die Prüfmuster derart in die Apparatur eingebracht, dass die dem Inkontinenzartikel in Höschenform im Anwendungszustand zugeordnete Innenseite, also die körpernahe Seite, in Richtung Prüftopfinnenseite zeigt. Wie den Ergebnissen aus Tabelle 2 zu entnehmen ist, weist der luftdurchlässige Bereich (Prüfmuster 1) im Vergleich zum diesen luftdurchlässigen Bereich umgebenden Bereich (Prüfmuster 2) eine um den Faktor 2,3 größere Wasserdampfdurchlässigkeit auf.

Tabelle1: Luftdurchlässigkeit

| | Probe | Parameter | Einheit | n | **x** | s | min | max |
|---|---|---|---|---|---|---|---|---|
| | | | | | | | | |
| 1 | Intakte Trägerfolie | Prüffläche | cm$^2$ | 1 | **20** | | 20 | 20 |
| | | Luftdurchlässigkeit | l/m$^2$/s | 1 | **0** | | 0 | 0 |
| | | | | | | | | |
| 2 | Trägerfolie mit einer mit Klebeband überklebten Ausstanzung 7 x 45 mm | Prüffläche * | cm$^2$ | 2 | **3,09** | 0,01 | 3,08 | 3,10 |
| | | Luftdurchlässigkeit: abgelesener Wert ** | l/m$^2$/s | 2 | **0,45** | 0,09 | 0,39 | 0,52 |
| | | Luftdurchlässigkeit bezogen auf die eigentliche Fläche *** | l/m$^2$/s | 2 | **2,92** | 0,57 | 2,52 | 3,32 |
| | | | | | | | | |
| 3 | Prüfmuster 2 | Prüffläche * | cm$^2$ | 6 | **1,23** | 0,11 | 1,11 | 1,37 |
| | | Luftdurchlässigkeit: abgelesener Wert ** | l/m$^2$/s | 6 | **3,12** | 1,00 | 1,50 | 4,00 |
| | | Luftdurchlässigkeit bezogen auf die eigentliche Fläche *** | l/m$^2$/s | 6 | **50,39** | 14,66 | 25,00 | 65,81 |
| | | | | | | | | |
| 4 | Prüfmuster 1 | Prüffläche * | cm$^2$ | 6 | **1,39** | 0,21 | 1,17 | 1,64 |
| | | Luftdurchlässigkeit: abgelesener Wert ** | l/m$^2$/s | 6 | **8,53** | 1,56 | 7,00 | 10,60 |
| | | Luftdurchlässigkeit bezogen auf die eigentliche Fläche *** | l/m$^2$/s | 6 | **124,5** | 25,75 | 91,46 | 157,0 |

* Prüffläche entspricht der nach Einkleben des Prüflings in das Trägermaterial effektiv zugänglichen Fläche des Prüflings ** Der abgelesene gemessene Wert bezieht sich auf die von der Messapparatur vorgegebene definierte Abmessung der Prüffläche, hier 20 cm$^2$.

*** Der auf die effektiv eingesetzte Prüffläche normierte Wert.

N Anzahl der Messungen X Mittelwert S Standardabweichung Min/max: Extremwerte

Tabelle 2: Wasserdampfdurchlässigkeit

| | Probe | Parameter | Einheit | n | **x** | s | min | max |
|---|---|---|---|---|---|---|---|---|
| | | | | | | | | |
| 1 | Nullkontrolle: Intakte Trägerfolie | Gewicht A | g | 1 | **1160,24** | | 1160,24 | 1160,24 |
| | | Gewicht B nach 24h | g | 1 | **1160,19** | | 1160,19 | 1160,19 |
| | | Fläche Ø62mm | m$^2$ | 1 | **0,0030** | | 0,0030 | 0,0030 |
| | | WVTR | g/m$^2$/24h | 1 | **16,56** | | 16,56 | 16,56 |

(fortgesetzt)

| | Probe | Parameter | Einheit | n | **x** | s | min | max |
|---|---|---|---|---|---|---|---|---|
| 2 | Trägerfolie mit einer mit Klebeband überklebten Ausstanzung 7 x 45 mm | Gewicht A | g | 1 | **1165,86** | | 1165,86 | 1165,86 |
| | | Gewicht B nach 24h | g | 1 | **1165,82** | | 1165,82 | 1165,82 |
| | | Probenfläche | $m^2$ | 1 | **0,00030** | | 0,00030 | 0,00030 |
| | | WVTR | $g/m^2/24h$ | 1 | **132,89** | | 132,89 | 132,89 |
| 3 | Prüfmuster 2 | Gewicht A | g | 6 | **1163,56** | 5,60 | 1155,59 | 1170,87 |
| | | Gewicht B nach 24h | g | 6 | **1163,28** | 5,62 | 1155,32 | 1170,63 |
| | | Probenfläche # | $m^2$ | 6 | **0,00016** | 0,00002 | 0,00014 | 0,00020 |
| | | WVTR | $g/m^2/24h$ | 6 | **1669,47** | 169,03 | 1346,15 | 1829,27 |
| 4 | Prüfmuster 1 | Gewicht A | g | 6 | **1161,71** | 7,44 | 1150,49 | 1172,08 |
| | | Gewicht B nach 24h | g | 6 | **1161,11** | 7,43 | 1149,94 | 1171,51 |
| | | Probenfläche # | $m^2$ | 6 | **0,00015** | 0,00002 | 0,00013 | 0,00018 |
| | | WVTR | $g/m^2/24h$ | 6 | **3945,35** | 360,18 | 3475,61 | 4358,97 |

\# Probenfläche entspricht der nach Einkleben des Prüflings in das Trägermaterial effektiv zugänglichen Fläche des Prüflings
N Anzahl der Messungen
X Mittelwert
S Standardabweichung
Min/max: Extremwerte

**Patentansprüche**

**1.** Inkontinenzartikel (2) in Höschenform für die Aufnahme von Körperausscheidungen mit einem vorderen Bauchabschnitt (4) und einem hinteren Rückenabschnitt (6), die zur Bildung eines in Quer- oder Hüftumfangsrichtung (16) durchgehenden Bauch- und Rückenbands mit einer in Hüftumfangsrichtung geschlossenen Hüftöffnung (18) an beidseitigen Seitennahtbereichen (14) herstellerseitig miteinander verbunden sind, und mit einem einen Absorptionskörper (7) aufweisenden Schrittabschnitt (8) mit Längsrändern (48) und Längsenden (98,100), der sich in einer Längsrichtung (9) zwischen dem Bauchabschnitt (4) mit einem schrittzugewandten Querrand (58) und dem Rückenabschnitt (6) mit einem schrittzugewandten Querrand (60) erstreckt, wobei der Schrittabschnitt (8) mit dem Bauchabschnitt (4) in einem vorderen Überlappungsbereich (36) und der Schrittabschnitt (8) mit dem Rückenabschnitt (6) in einem hinteren Überlappungsbereich (38) unter Ausbildung eines jeweiligen Lagenaufbaus (13) in Z-Richtung (11) überlappt, wobei der Schrittabschnitt (8) an den Bauchabschnitt (4) in einem vorderen Verbindungsbereich (306) und der Schrittabschnitt (8) an den Rückenabschnitt (6) in einem hinteren Verbindungsbereich (308) unlösbar angefügt ist, wobei sowohl der Schrittabschnitt (8) als auch der Bauchabschnitt (4) und der Rückenabschnitt (6) Beinöffnungen (19) des Inkontinenzartikels begrenzen, wobei in dem Bauchabschnitt (4) und dem Rückenabschnitt (6) erste Elastifizierungsmittel (28) vorgesehen sind, die sich in einem Abstand voneinander und parallel zueinander in Quer- oder Hüftumfangsrichtung (16) erstrecken und so den Bauchabschnitt (4) und den Rückenabschnitt (6) flächenhaft elastifizieren, wobei in einem schrittseitigen und den Beinöffnungen (19) zugewandten Bereich (22, 26) des Bauchabschnitts (4) und des Rückenabschnitts (6) zweite Elastifizierungsmittel (40, 42) vorgesehen sind, wobei der Schrittabschnitt (8) ein flüssigkeitsundurchlässiges Backsheet-Material (62) umfasst, und wobei der Absorptionskörper (7) zwischen Backsheet-Material (62) und einem Deckblatt-Material (84) angeordnet ist und der Absorptionskörper (7) Längsränder (46) aufweist, wobei das Backsheet-Material (62) oder das Deckblatt-Material (84) und das Backsheet-Material (62) in Querrichtung (16) einen sich jeweils außerhalb der Längsränder (46) des Absorptionskörpers (7) erstreckenden Überhang (66a, 66b) bilden, wobei der Überhang (66a, 66b) in Querrichtung (16) in der Summe, also beidseits der Längsränder (46) des Absorptionskörpers (7) wenigstens 25 % bezogen auf eine größte Breite (E) des Schrittabschnitts (8) beträgt, wobei der Überhang (66a, 66b) beidseits der Längsränder (46) des Absorptionskörpers (7) jeweils erste Bereiche (65a, 65a', 65b, 65b') und einen zweiten Bereich (67a, 67b) aufweist, wobei die ersten Bereiche (65a, 65a', 65b, 65b') sich jeweils innerhalb der Überlappungsbereiche (36, 38) erstrecken und der zweite Bereich (67a, 67b) sich in Längsrichtung (9) zwischen den schrittzugewandten Querrändern (58, 60) des Bauchabschnitts (4) und Rückenabschnitts (6) erstreckt, wobei die Verbindungsbereiche (306, 308) des Bauchabschnitts (4) und des Rückenabschnitts (6) jeweils einen ersten Fügebereich (310, 312) und zweite Fügebereiche (314a, 314b, 316a, 316b) umfassen, wobei der erste Fügebereich (310, 312) sich zumindest abschnittsweise im Bereich des Absorptionskörpers (7) erstreckt, und wobei die zweiten Fügebereiche (314a, 314b, 316a, 316b) zumindest innerhalb der ersten Bereiche (65a, 65a', 65b, 65b') des Überhangs (66a, 66b) des Schrittabschnitts (8) angeordnet sind, so dass die zweiten Fügebereiche (314a, 314b, 316a, 316b) Verstärkungsbereiche (334a, 334b, 336a, 336b) bilden, **dadurch gekennzeichnet, dass** als Fügemittel in einem ersten Fügebereich ein Klebemittel eingesetzt wird und für einen zweiten Fügebereich nicht-adhäsive Fügemittel eingesetzt werden, dass im vorderen oder im hinteren Überlappungsbereich (36, 38) die ersten Bereiche (65a, 65a', 65b, 65b') des Überhangs (66a, 66b) sich durch den Lagenaufbau (13) in Z-Richtung hindurcherstreckende Öffnungen (346) aufweisen, wobei die Öffnungen (346) luftdurchlässige Bereiche (350a, 350b, 351a, 351b) bilden, und die Öffnungen (346) zumindest innerhalb der zweiten Fügebereiche (314a, 314b, 316a, 316b) angeordnet sind, wobei die Öffnungen (346) durch Fügemittel (340) in Form von Schweißstellen, insbesondere Ultraschallschweißstellen, thermische Schweißstellen und/oder Kalanderschweißstellen gebildet sind.

2. Inkontinenzartikel nach Anspruch 1, **dadurch gekennzeichnet, dass** die ersten Bereiche (65a, 65a', 65b, 65b') des Überhangs (66a, 66b) im vorderen und im hinteren Überlappungsbereich (36, 38) sich durch den Lagenaufbau (13) in Z-Richtung hindurcherstreckende Öffnungen (346) aufweisen.

3. Inkontinenzartikel nach Anspruch 1 oder 2, **dadurch gekennzeichnet, dass** der zweite Fügebereich (314a, 314b, 316a, 316b) einen Teilbereich (324a, 324, 326a, 326b) des Überhangs (66a, 66ab) in Querrichtung (16) mit einer Breite P' und/oder die luftdurchlässigen Bereiche (350a, 350b, 351 a, 351 b) einen Teilbereich (352a, 352b, 353a, 353b) des Überhangs (66a, 66ab) in Querrichtung (16) mit einer Breite M' überfängt.

4. Inkontinenzartikel nach einem oder mehreren der voranstehenden Ansprüche, **dadurch gekennzeichnet, dass** der zweite Fügebereich (314a, 314b, 316a, 316b) in einem Abstand (AP) vor dem Längsrand (46) des Absorptionskörpers (7) endet, und insbesondere beginnend vom Längsrand (48) des Schrittabschnitts (8) angeordnet ist.

5. Inkontinenzartikel nach einem oder mehreren der voranstehenden Ansprüche, **dadurch gekennzeichnet, dass** der luftdurchlässigen Bereich (350a, 350b, 351 a, 351b) in einem Abstand (AM) vor dem Längsrand (46) des Absorptionskörpers (7) endet, und insbesondere beginnend vom Längsrand (48) des Schrittabschnitts (8) angeordnet ist.

6. Inkontinenzartikel nach einem oder mehreren der voranstehenden Ansprüche, **dadurch gekennzeichnet, dass** der jeweilige zweite Fügebereich (314a, 314b, 316a, 316b) und der jeweilige luftdurchlässige Bereiche (350a, 350b, 351 a, 351 b) in deren flächenhafter Erstreckung übereinstimmen, insbesondere kongruent übereinander gelagert sind.

7. Inkontinenzartikel nach einem oder mehreren der voranstehenden Ansprüche, **dadurch gekennzeichnet, dass** ein jeweiliger zweiter Fügebereich (314a, 314b, 316a, 316b) und damit Verstärkungsbereich (334a, 334b, 336a, 336b) und/oder ein jeweiliger luftdurchlässiger Bereich (350a, 350b, 351 a, 351 b) in Längsrichtung (9) betrachtet sich beginnend von dem schrittzugewandten Querrand (58, 60) des Bauchabschnitts (4) und/oder des Rückenabschnitts (6) in Richtung auf die Längsenden (98, 100) des Schrittabschnitts (8) erstreckt, sich insbesondere kontinuierlich bis mindestens zum jeweiligen Längsende (98, 100) des Schrittabschnitts (8) erstreckt.

8. Inkontinenzartikel nach einem oder mehreren der vorstehenden Ansprüche, **dadurch gekennzeichnet, dass** ein Anteil P'/ H des durch einen zweiten Fügebereich (314a, 314b, 316a, 316b) in Querrichtung (16) überfangenen Teilbereiches (324a, 324b, 326a, 326b) des jeweiligen Überhangs (66a, 66b) mit einer Breite (P') bezogen auf den jeweiligen Überhang (66a, 66b) in Querrichtung (16) mit einer Breite (H) im vorderen und/oder hinteren Überlappungsbereich (36, 38) mindestens 0,01, insbesondere mindestens 0,04, weiter insbesondere mindestens 0,07, weiter insbesondere mindestens 0,10, aber vorzugsweise höchstens 0,90, insbesondere höchstens 0,80, weiter insbesondere höchstens 0,70, weiter insbesondere höchstens 0,60, weiter insbesondere höchstens 0,50, weiter insbesondere höchstens 0,40 beträgt.

9. Inkontinenzartikel nach einem oder mehreren der vorstehenden Ansprüche, **dadurch gekennzeichnet, dass** ein Anteil M'/ H des durch einen luftdurchlässigen Bereich (350a, 350b, 351 a, 351 b) in Querrichtung (16) überfangenen Teilbereiches (352a, 352b, 353a, 353b) des jeweiligen Überhangs (66a, 66b) mit einer Breite (M') bezogen auf den jeweiligen Überhang (66a, 66b) in Querrichtung (16) mit einer Breite (H) im vorderen und/oder hinteren Überlappungsbereich (36, 38) mindestens 0,01, insbesondere mindestens 0,04, weiter insbesondere mindestens 0,07, weiter insbesondere mindestens 0,10, aber vorzugsweise höchstens 0,90, insbesondere höchstens 0,80, weiter insbesondere höchstens 0,70, weiter insbesondere höchstens 0,60, weiter insbesondere höchstens 0,50, weiter insbesondere höchstens 0,40 beträgt.

10. Inkontinenzartikel nach einem oder mehreren der vorstehenden Ansprüche, **dadurch gekennzeichnet, dass** ein Anteil P'/N des durch einen zweiten Fügebereich (314a, 314b, 316a, 316b) überfangenen Teilbereiches (324a, 324b, 326a, 326b) in Querrichtung (16) des jeweiligen Überhangs (66a, 66b) mit einer Breite (P') bezogen den jeweiligen Seitenbereich (360a, 360b, 362a, 362b) des Bauchabschnitts (4) und/oder des Rückenabschnitts (6) in Querrichtung (16) mit einer Breite (N) mindestens 0,01, insbesondere mindestens 0,015, weiter insbesondere mindestens 0,020, aber vorzugsweise höchstens 0,35, insbesondere höchstens 0,30, weiter insbesondere höchstens 0,25, weiter insbesondere höchstens 0,20, weiter insbesondere höchstens 0,15, weiter insbesondere höchstens 0,10 beträgt.

11. Inkontinenzartikel nach einem oder mehreren der vorstehenden Ansprüche, **dadurch gekennzeichnet, dass** ein Anteil M'/N des durch einen luftdurchlässigen Bereich (350a, 350b, 351a, 351b) überfangenen Teilbereiches (352a, 352b, 353a, 353b) in Querrichtung (16) des jeweiligen Überhangs (66a, 66b) mit einer Breite (M') bezogen den jeweiligen Seitenbereich (360a, 360b, 362a, 362b) des Bauchabschnitts (4) und/oder des Rückenabschnitts (6) in Querrichtung(16) mit einer Breite (N) mindestens 0,01, insbesondere mindestens 0,015, weiter insbesondere mindestens 0,020, aber vorzugsweise höchstens 0,35, insbesondere höchstens 0,30, weiter insbesondere höchstens 0,25, weiter insbesondere höchstens 0,20, weiter insbesondere höchstens 0,15, weiter insbesondere höchstens 0,10 beträgt.

12. Inkontinenzartikel nach einem oder mehreren der vorstehenden Ansprüche, **dadurch gekennzeichnet, dass** die Öffnungen (346) in einem Punktmuster angeordnet sind und wobei die Summe der durch die Öffnungen (346) überfangene Fläche einen Anteil von mindestens 1,5 %, insbesondere von mindestens 2,0 %, weiter insbesondere mindestens 2,3 %, weiter insbesondere mindestens 2,5 %, und vorzugsweise höchstens 20,0%, insbesondere höchstens 15,0%, weiter insbesondere höchstens 10,0%, weiter insbesondere höchstens 8,0%, weiter insbesondere höchstens 7,0 %, weiter insbesondere höchstens 6,0% bezogen auf die von einem luftdurchlässigen Bereich (350a, 350b, 351a, 351 b), insbesondere von einem zweiten Fügebereich (314a, 314b, 316a, 316b) überfangene Gesamtfläche einnimmt.

13. Inkontinenzartikel nach einem oder mehreren der vorstehenden Ansprüche, **dadurch gekennzeichnet, dass** der luftdurchlässige Bereich (350a, 350b, 351 a, 351 b) eine Luftdurchlässigkeit aufweist, welche um den Faktor 1,5, insbesondere 2, insbesondere 2,5, weiter insbesondere 3, weiter insbesondere höchstens 5, weiter insbesondere höchstens 4,5 größer ist als die Luftdurchlässigkeit im ersten Bereich (65a, 65a', 65b, 65b') des Überhangs (66a, 66b) außerhalb des luftdurchlässigen Bereichs (350a, 350b, 351a, 351 b).

14. Inkontinenzartikel nach einem oder mehreren der vorstehenden Ansprüche, **dadurch gekennzeichnet, dass** der Überhang (66a, 66b) des Backsheet-Materials (62) und/oder des Deckblatt-Materials (84) in Querrichtung (16) in der Summe, also beidseits der Längsränder (46) des Absorptionskörpers (7) 25 - 50 %, weiter insbesondere 30 - 45 % und weiter insbesondere 35 - 45 % bezogen auf eine größte Breite (E) des Schrittabschnitts (8) beträgt.

## Claims

1. Incontinence article (2) in the form of briefs for receiving body excretions, with a front stomach portion (4) and a rear back portion (6) which, to form a stomach and back band which is continuous in the transverse or waist-encircling direction (16) and has a waist opening (18) that is closed in the waist-encircling direction, are connected to one another at the manufacturer's at side seam regions (14) on both sides, and with a crotch portion (8), which has an absorbent body (7) and has longitudinal peripheries (48) and longitudinal ends (98, 100) and extends in a longitudinal direction (9) between the stomach portion (4) with a crotch-facing transverse periphery (58) and the back portion (6) with a crotch-facing transverse periphery (60), wherein the crotch portion (8) overlaps with the stomach portion (4) in a front overlapping region (36) and the crotch portion (8) overlaps with the back portion (6) in a rear overlapping region (38) while forming a respective buildup of plies (13) in the Z direction (11), wherein the crotch portion (8) is inseparably joined to the stomach portion (4) in a front connecting region (306) and the crotch portion (8) is inseparably joined to the back portion (6) in a rear connecting region (308), wherein not only the crotch portion (8) but also the stomach portion (4) and the back portion (6) bound leg openings (19) of the incontinence article, wherein first elasticating means (28) are provided in the stomach portion (4) and the back portion (6), extend at a distance from one another and parallel to one another in the transverse or waist-encircling direction (16) and thus elasticate the stomach portion (4) and the back portion (6) over their surface area, wherein second elasticating means (40, 42) are provided in a crotch-side region (22, 26) of the stomach portion (4) and of the back portion (6) that is facing the leg openings (19), wherein the crotch portion (8) comprises a liquid-impermeable backsheet material (62), and wherein the absorbent body (7) is arranged between the backsheet material (62) and a topsheet material (84) and the absorbent body (7) has longitudinal peripheries (46), wherein the backsheet material (62) or the topsheet material (84) and the backsheet material (62) form in the transverse direction (16) an overhang (66a, 66b) extending respectively outside the longitudinal peripheries (46) of the absorbent body (7), wherein the sum of the overhang (66a, 66b) in the transverse direction (16), that is to say on both sides of the longitudinal peripheries (46) of the absorbent body (7), is at least 25% with respect to the greatest width (E) of the crotch portion (8), wherein the overhang (66a, 66b) on both sides of the longitudinal peripheries (46) of the absorbent body (7) respectively has first regions (65a, 65a', 65b, 65b') and a second region (67a, 67b), wherein the first regions (65a, 65a', 65b, 65b') respectively extend within the overlapping regions (36, 38) and the second region (67a, 67b) extends in the longitudinal direction (9) between the crotch-facing transverse peripheries (58, 60) of the stomach portion (4) and back portion (6), wherein the connecting regions (306, 308) of the stomach portion (4) and of the back portion (6) respectively comprise a first joining region (310, 312) and second joining regions (314a, 314b, 316a, 316b), wherein the first joining region (310, 312) extends at least in certain portions in the region of the absorbent body (7), and wherein the second joining regions (314a, 314b, 316a, 316b) are arranged at least within the first regions (65a, 65a', 65b, 65b') of the overhang (66a, 66b) of the crotch portion (8), so that the second joining regions (314a, 314b, 316a, 316b) form reinforcing regions (334a, 334b, 336a, 336b), **characterized in that** an adhesive is used as joining means in a first joining region and non-adhesive joining means are used for a second joining region, **in that** the first regions (65a, 65a', 65b, 65b') of the overhang (66a, 66b) have openings (346) extending through the buildup of plies (13) in the Z direction in the front or rear overlapping region (36, 38), the openings (346) forming air-permeable regions (350a, 350b, 351a, 351b), and the openings (346) being arranged at least within the second joining regions (314a, 314b, 316a, 316b), the openings (346) being formed by joining means (340) in the form of welding locations, in particular ultrasonic welding locations, thermal welding locations and/or calender welding locations.

2. Incontinence article according to Claim 1, **characterized in that** the first regions (65a, 65a', 65b, 65b') of the overhang (66a, 66b) in the front and rear overlapping region (36, 38) have openings (346) extending through the buildup of plies (13) in the Z direction.

3. The incontinence article according to Claim 1 or 2, **characterized in that** the second joining region (314a, 314b, 316a, 316b) extends over a subregion (324a, 324, 326a, 326b) of the overhang (66a, 66b) in the transverse direction (16) with a width P' and/or the air-permeable regions (350a, 350b, 351a, 351b) extend over a subregion (352a, 352b, 353a, 353b) of the overhang (66a, 66b) in the transverse direction (16) with a width M'.

4. Incontinence article according to one or more of the preceding claims, **characterized in that** the second joining region (314a, 314b, 316a, 316b) ends at a distance (AP) before the longitudinal periphery (46) of the absorbent body (7), and particularly is arranged from the longitudinal periphery (48) of the crotch portion (8).

5. Incontinence article according to one or more of the preceding claims, **characterized in that** the air-permeable region (350a, 350b, 351a, 351b) ends at a distance (AM) before the longitudinal periphery (46) of the absorbent body (7), and particularly is arranged from the longitudinal periphery (48) of the crotch portion (8).

6. Incontinence article according to one or more of the preceding claims, **characterized in that** the respective second joining region (314a, 314b, 316a, 316b) and the respective air-permeable regions (350a, 350b, 351a, 351b) coincide in their areal extent, in particular are disposed congruently one on top of the other.

7. Incontinence article according to one or more of the preceding claims, **characterized in that** a respective second joining region (314a, 314b, 316a, 316b), and consequently reinforcing region (334a, 334b, 336a, 336b) and/or a respective air-permeable region (350a, 350b, 351a, 351b) extends, when considered in the longitudinal direction (9), from the crotch-facing transverse periphery (58, 60) of the stomach portion (4) and/or of the back portion (6) in the direction of the longitudinal ends (98, 100) of the crotch portion (8), in particular extends continuously up to at least the respective longitudinal end (98, 100) of the crotch portion (8).

8. Incontinence article according to one or more of the preceding claims, **characterized in that** a proportion P'/H of the subregion (324a, 324b, 326a, 326b) of the respective overhang (66a, 66b) that is extended over in the transverse direction (16) by a second joining region (314a, 314b, 316a, 316b), with a width (P') with respect to the respective overhang (66a, 66b) in the transverse direction (16) with a width (H) in the front and/or rear overlapping region (36, 38), is at least 0.01, particularly at least 0.04, more particularly at least 0.07, more particularly at least 0.10, but preferably at most 0.90, particularly at most 0.80, more particularly at most 0.70, more particularly at most 0.60, more particularly at most 0.50, more particularly at most 0.40.

9. Incontinence article according to one or more of the preceding claims, **characterized in that** a proportion M'/H of the subregion (352a, 352b, 353a, 353b) of the respective overhang (66a, 66b) that is extended over in the transverse direction (16) by an air-permeable region (350a, 350b, 351a, 351b), with a width M' with respect to the respective overhang (66a, 66b) in the transverse direction (16) with a width H in the front and/or rear overlapping region (36, 38), is at least 0.01, particularly at least 0.04, more particularly at least 0.07, more particularly at least 0.10, but preferably at most 0.90, particularly at most 0.80, more particularly at most 0.70, more particularly at most 0.60, more particularly at most 0.50, more particularly at most 0.40.

10. Incontinence article according to one or more of the preceding claims, **characterized in that** a proportion P'/N of the subregion (324a, 324b, 326a, 326b) of the respective overhang (66a, 66b) that is extended over in the transverse direction (16) by a second joining region (314a, 314b, 316a, 316b), with a width (P') with respect to the respective side region (360a, 360b, 362a, 362b) of the stomach portion (4) and/or of the back portion (6) in the transverse direction (16) with a width (N), is at least 0.01, particularly at least 0.015, more particularly at least 0.020, but preferably at most 0.35, particularly at most 0.30, more particularly at most 0.25, more particularly at most 0.20, more particularly at most 0.15, more particularly at most 0.10.

11. Incontinence article according to one or more of the preceding claims, **characterized in that** a proportion M'/N of the subregion (352a, 352b, 353a, 353b) of the respective overhang (66a, 66b) that is extended over in the transverse direction (16) by an air-permeable region (350a, 350b, 351a, 351b), with a width (M') with respect to the respective side region (360a, 360b, 362a, 362b) of the stomach portion (4) and/or of the back portion (6) in the transverse direction (16) with a width (N), is at least 0.01, particularly at least 0.015, more particularly at least 0.020, but preferably at most 0.35, particularly at most 0.30, more particularly at most 0.25, more particularly at most 0.20, more particularly at most 0.15, more particularly at most 0.10.

12. Incontinence article according to one or more of the preceding claims, **characterized in that** the openings (346) are arranged in a point pattern and wherein the sum of the surface area extended over by the openings (346) assumes a proportion of at least 1.5%, particularly at least 2.0%, more particularly at least 2.3%, more particularly at least 2.5% and preferably at most 20.0%, particularly at most 15.0%, more particularly at most 10.0%, more particularly at most 8.0%, more particularly at most 7.0%, more particularly at most 6.0%, with respect to the overall surface area extended over by an air-permeable region (350a, 350b, 351a, 351b), particularly by a second joining region (314a, 314b, 316a, 316b).

13. Incontinence article according to one or more of the preceding claims, **characterized in that** the air-permeable region (350a, 350b, 351a, 351b) has an air permeability which is greater than the air permeability in the first region (65a, 65a', 65b, 65b') of the overhang (66a, 66b) outside the air-permeable region (350a, 350b, 351a, 351b) by a factor of 1.5, particularly 2, particularly 2.5, more particularly 3, more particularly at most 5, more particularly at most 4.5.

14. Incontinence article according to one or more of the preceding claims, **characterized in that** the overhang (66a, 66b) of the backsheet material (62) and/or of the topsheet material (84) in the transverse direction (16) is in total, that is to say on both sides of the longitudinal peripheries (46) of the absorbent body (7), 25 - 50%, more particularly 30 - 45% and more particularly 35 - 45%, with respect to the greatest width (E) of the crotch portion (8).

**Revendications**

1. Article (2) pour incontinents, destiné à reprendre des sécrétions corporelles, en forme de culotte présentant une partie ventrale avant (4) et une partie dorsale arrière (6) qui sont reliées l'une à l'autre lors de leur fabrication pour former une bande ventrale et dorsale continue dans la direction (16) transversale ou du tour de hanches, une ouverture de hanche (18) fermée dans la direction du tour de hanches sur des parties (14) de cordon latéral situées des deux côtés, et

   une partie (8) d'entrejambes qui présente un corps d'absorption (7) et des bords longitudinaux (48) ainsi que des extrémités longitudinales (98, 100), qui s'étend dans une direction longitudinale (9) entre la partie ventrale (4) et la partie dorsale (6) et qui présente un bord transversal (58) de partie ventrale tourné vers l'entrejambes et un bord transversal (60) de partie dorsale tourné vers l'entrejambes,

   la partie d'entrejambes (8) recouvrant la partie ventrale (4) dans une zone (36) de superposition avant et la partie d'entrejambes (8) recouvrant la partie dorsale (6) dans une zone (38) de superposition arrière, chaque fois en formant une superposition (13) de couches dans la direction Z (11),

   la partie d'entrejambes (8) étant jointe de manière non libérable à la partie ventrale (4) dans une zone (306) de liaison avant et la partie d'entrejambes (8) étant jointe de manière non libérable à la partie dorsale (6) dans une zone (308) de liaison arrière,

   tant la partie d'entrejambes (8) que la partie ventrale (4) et la partie dorsale (6) délimitant des ouvertures (19) prévues pour les jambes dans l'article pour incontinents,

   des premiers moyens d'élastification (28) qui s'étendent à distance mutuelle et parallèlement l'un à l'autre dans la direction transversale ou de tour de hanches (16) et élastifiant ainsi la partie ventrale (4) et la partie dorsale (6) de telle sorte qu'elles puissent épouser une surface étant prévus dans la partie ventrale (4) et la partie dorsale (6), des deuxièmes moyens d'élastification (40, 42) étant prévus dans des sections (22, 26) de la partie ventrale (4) et de la partie dorsale (6) tournées vers l'entrejambes et les ouvertures (19) prévues pour les jambes,

   la partie d'entrejambes (8) comprenant un matériau (62) de feuille arrière imperméable aux liquides,

   le corps d'absorption (7) étant disposé entre le matériau (62) de feuille arrière et un matériau (84) de feuille de recouvrement et le corps d'absorption (7) présentant des bords longitudinaux (46),

   le matériau (62) de feuille arrière ou le matériau (84) de feuille de recouvrement et le matériau (62) de feuille arrière formant dans la direction transversale (16) un débord (66a, 66b) qui s'étend à l'extérieur des bords longitudinaux (46) du corps d'absorption (7),

   le débord (66a, 66b) représentant dans la direction transversale (16) en somme, et donc des deux côtés des bords longitudinaux (46) du corps d'absorption (7), au moins 25 % de la plus grande largeur (E) de la partie d'entrejambes (8),

   le débord (66a, 66b) présentant des deux côtés des bords longitudinaux (46) du corps d'absorption (7) des premières sections (65a, 65a', 65b, 65b') et une deuxième section (67a, 67b),

   les premières sections (65a, 65a', 65b, 65b') s'étendant à l'intérieur des sections de recouvrement (36, 38) et la deuxième section (67a, 67b) s'étendant dans le sens de la longueur (9) entre les bords transversaux (58, 60), tournés vers l'entrejambes, de la partie ventrale (4) et de la partie dorsale (6),

   les sections de liaison (306, 308) de la partie ventrale (4) et de la partie dorsale (6) comprenant chacune une première section de jonction (310, 312) et des deuxièmes sections de jonction (314a, 314b, 316a, 316b),

   la première section de jonction (310, 312) s'étendant au moins en partie au niveau du corps d'absorption (7),

   les deuxièmes sections de jonction (314a, 314b, 316a, 316b) étant disposées au moins à l'intérieur des premières sections (65a, 65a', 65b, 65b') du débord (66a, 66b) de la partie d'entrejambes (8) de telle sorte que les deuxièmes sections de jonction (314a, 314b, 316a, 316b) forment des sections de renfort (334a, 334b, 336a, 336b),

   **caractérisé en ce que**

   un adhésif servant de moyen de jonction est utilisé dans la première section de jonction et des moyens non adhésifs de jonction sont utilisés pour une deuxième section de jonction,

   **en ce que** dans la section de superposition avant ou dans la section de superposition arrière (36, 38), les premières sections (65a, 65a', 65b, 65b') du débord (66a, 66b) présentent des ouvertures (346) qui traversent la superposition (13) de couches dans la direction Z,

   **en ce que** les ouvertures (346) forment des sections (350a, 350b, 351a, 351b) perméables à l'air,

   **en ce que** les ouvertures (346) sont disposées au moins à l'intérieur des deuxièmes sections de jonction (314a, 314b, 316a, 316b) et

   **en ce que** les ouvertures (346) sont formées par des moyens de jonction (340) qui présentent la forme d'emplacements soudés, en particulier d'emplacements soudés par ultrasons, d'emplacements soudés thermiquement et/ou d'emplacements soudés à la calandreuse.

**2.** Article pour incontinents selon la revendication 1, **caractérisé en ce que** les premières sections (65a, 65a', 65b, 65b') du débord (66a, 66b) présentent dans la section avant et la section arrière de superposition (36, 38) des ouvertures (346) qui traversent la superposition (13) de couches dans la direction Z.

**3.** Article pour incontinents selon les revendications 1 ou 2, **caractérisé en ce que** la deuxième section de jonction (314a, 314b, 316a, 316b) recouvre sur une largeur M' une section partielle (324a, 324, 326a, 326b) du débord (66a, 66ab) dans la direction transversale (16) sur une largeur P' et/ou **en ce que** les sections (350a, 350b, 351a, 351b) perméables à l'air débordent sur une largeur M' une section partielle (352a, 352b, 353a, 353b) du débord (66a, 66ab) dans la direction transversale (16).

**4.** Article pour incontinents selon l'une ou plusieurs des revendications précédentes, **caractérisé en ce que** la deuxième section de jonction (314a, 314b, 316a, 316b) se termine à une distance (AP) en avant du bord longitudinal (46) du corps d'absorption (7) et est en particulier située de manière à commencer à partir du bord longitudinal (48) de la section d'entrejambes (8).

**5.** Article pour incontinents selon l'une ou plusieurs des revendications précédentes, **caractérisé en ce que** la section (350a, 350b, 351a, 351b) perméable à l'air se termine à une distance (AM) en avant du bord longitudinal (46) du corps d'absorption (7) et est en particulier disposée de manière à commencer sur le bord longitudinal (48) de la partie d'entrejambes (8).

**6.** Article pour incontinents selon l'une ou plusieurs des revendications précédentes, **caractérisé en ce que** chaque deuxième section de jonction (314a, 314b, 316a, 316b) et chaque section (350a, 350b, 351a, 351b) perméable à l'air se correspondent dans leur extension épousant les surfaces et sont en particulier superposées l'une à l'autre de manière congruente.

**7.** Article pour incontinents selon l'une ou plusieurs des revendications précédentes, **caractérisé en ce que** chaque deuxième section de jonction (314a, 314b, 316a, 316b) et donc chaque section de renfort (334a, 334b, 336a, 336b) et/ou chaque section (350a, 350b, 351a, 351b) perméable à l'air s'étend dans le sens de la longueur (9) en commençant du bord transversal (58, 60) tourné vers l'entrejambes, de la partie ventrale (4) et/ou de la partie dorsale (6), en direction de l'extrémité longitudinale (98, 100) de la partie d'entrejambes (8), en particulier de manière continue jusqu'au moins l'extrémité longitudinale (98, 100) correspondante de la partie d'entrejambes (8).

**8.** Article pour incontinents selon l'une ou plusieurs des revendications précédentes, **caractérisé en ce que** dans la section de superposition avant et/ou dans la section de superposition arrière (36, 38), le rapport P'/H entre la section partielle (324a, 324b, 326a, 326b), recouverte par une deuxième section de jonction (314a, 314b, 316a, 316b) dans la direction transversale (16), de chaque débord (66a, 66b), de largeur (P'), et le débord respectif (66a, 66b) de largeur (H) dans la direction transversale (16) vaut au moins 0,01, en particulier au moins 0,04, de manière plus particulière au moins 0,07, de manière plus particulière au moins 0,10 mais de préférence au plus 0,90, en particulier au plus 0,80, de manière plus particulière au plus 0,70, de manière plus particulière au plus 0,60, de manière plus particulière au plus 0,50 et de manière encore plus particulière au plus 0,40.

**9.** Article pour incontinents selon l'une ou plusieurs des revendications précédentes, **caractérisé en ce que** dans la section de superposition avant et/ou la section de superposition arrière (36, 38), le rapport M'/H entre la section partielle (352a, 352b, 353a, 353b) recouverte par une section (350a, 350b, 351a, 351b) perméable à l'air dans la direction transversale (16) de chaque débord (66a, 56b), de largeur (M'), et le débord (66a, 66b) dans la direction transversale (16), de largeur (H), vaut au moins 0,01, en particulier au moins 0,04, de manière plus particulière au moins 0,07, de manière plus particulière au moins 0,10 mais de préférence au plus 0,90, en particulier au plus 0,80, de manière plus particulière au plus 0,70, de manière plus particulière au plus 0,60, de manière plus particulière au plus 0,50 et de manière encore plus particulière au plus 0,40.

**10.** Article pour incontinents selon l'une ou plusieurs des revendications précédentes, **caractérisé en ce que** le rapport P'/N entre la section partielle (324a, 324b, 326a, 326b), recouverte par une deuxième section de jonction (314a, 314b, 316a, 316b) dans la direction transversale (16), de chaque débord (66a, 66b), de largeur (P'), et la section latérale (360a, 360b, 362a, 362b) de la partie ventrale (4) et/ou de la partie dorsale (6) dans la direction transversale (16), de largeur (N), vaut au moins 0,01, en particulier au moins 0,015, de manière plus particulière au moins 0,020, mais de préférence au plus 0,35, en particulier au plus 0,30, de manière plus particulière au plus 0,25, de manière plus particulière au plus 0,20, de manière plus particulière au plus 0,15 et de manière encore plus particulière au plus 0,10.

**11.** Article pour incontinents selon l'une ou plusieurs des revendications précédentes, **caractérisé en ce que** le rapport M'/N entre la section partielle (352a, 352b, 353a, 353b) recouverte par une section (350a, 350b, 351a, 351b) perméable à l'air dans la direction transversale (16) de chaque débord (66a, 66b), de largeur (M'), et la section latérale (360a, 360b, 362a, 362b) de la partie ventrale (4) et/ou de la partie dorsale (6) dans la direction transversale (16), de largeur (N), vaut au moins 0,01, en particulier au moins 0,015, de manière plus particulière au moins 0,020, mais de préférence au plus 0,35, en particulier au plus 0,30, de manière plus particulière au plus 0,25, de manière plus particulière au plus 0,20, de manière plus particulière au plus 0,15 et de manière encore plus particulière au plus 0,10.

**12.** Article pour incontinents selon l'une ou plusieurs des revendications précédentes, **caractérisé en ce que** les ouvertures (346) sont disposées selon un motif ponctuel, la somme des surfaces couvertes par les ouvertures (346) représentant une proportion d'au moins 1,5 %, en particulier d'au moins 2,0 %, de manière plus particulière d'au moins 2,3 %, de manière encore plus particulière d'au moins 2,5 %, et de préférence d'au plus 20,0 %, en particulier d'au plus 15,0 %, de manière plus particulière d'au plus 10,0 %, de manière encore plus particulière d'au plus 8,0 % de manière encore plus particulière d'au plus 7,0 %, de manière encore plus particulière d'au plus 6,0 % de la surface totale recouverte par une section (350a, 350b, 351a, 351b) perméable à l'air et en particulier d'une deuxième section de jonction (314a, 314b, 316a, 316b).

**13.** Article pour incontinents selon l'une ou plusieurs des revendications précédentes, **caractérisé en ce que** la section (350a, 350b, 351a, 351b) perméable à l'air présente une perméabilité à l'air d'un facteur 1,5, en particulier d'un facteur 2, notamment d'un facteur 2,5, de manière plus particulière d'un facteur 3, de manière encore plus particulière d'au plus 5, de manière encore plus particulière d'au plus 4,5 fois plus grande que la perméabilité à l'air de la première section (65a, 65a', 65b, 65b') du débord (66a, 66b) à l'extérieur de la zone (350a, 350b, 351a, 351b) perméable à l'air.

**14.** Article pour incontinents selon l'une ou plusieurs des revendications précédentes, **caractérisé en ce que** le débord (66a, 66b) du matériau (62) de feuille arrière et/ou du matériau (84) de feuille de recouvrement représente dans la direction transversale (16) en somme, et donc des deux côtés des bords longitudinaux (46) du corps d'absorption (7), de 25 à 50 %, de manière plus particulière de 30 à 45 % et de manière encore plus particulière de 35 à 45 % de la plus grande largeur (E) de la partie d'entrejambes (8).

Fig. 1

Fig. 2

Fig. 3

EP 2 629 732 B1

Fig. 4

314b, 350b

349

4

VI    VI

344

346, 340, 342

66b

8

65b

Fig. 5

62  84    342, 340    48    11

66b

13

346    346    4

Fig. 6

Fig. 7

Fig. 8

Fig. 9

Fig. 10

Fig. 11

Fig. 12

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2004052260 A1
- WO 03039423 A1
- WO 2005067842 A1
- WO 2005016200 A1
- EP 1392212 B1
- WO 2010028751 A1
- DE 19716253 A1
- EP 0446867 A2